# EUROPEAN PATENT SPECIFICATION

(11) **EP 4 146 320 B1**
(45) Date of publication and mention of the grant of the patent: **04.03.2026**
(21) Application number: 21728749.9
(22) Date of filing: 07.05.2021
(51) Int. Cl.: A61M 25/06, A61M 25/00

(54) **EXPANDABLE SHEATH FOR INTRODUCING AN ENDOVASCULAR DELIVERY DEVICE INTO A BODY**
ERWEITERBARE HÜLLE ZUM EINFÜHREN EINER ENDOVASKULÄREN ABGABEVORRICHTUNG IN EINEN KÖRPER
GAINE EXPANSIBLE POUR L'INTRODUCTION D'UN DISPOSITIF D'ADMINISTRATION ENDOVASCULAIRE DANS UN CORPS

(30) Priority: 08.05.2020 US 202063021945 P; 31.07.2020 US 202063059764 P
(43) Date of publication of application: 15.03.2023
(73) Proprietor: Edwards Lifesciences Corporation, Irvine, CA 92614 (US)
(72) Inventor: RUIZ, David, Guadalupe, Corona, CA 92882 (US); GAFFNEY, Leah, Paige, Irvine, CA 92614 (US); LEE, Jeong, Soo, Irvine, CA 92614 (US); TAMIR, Ilan, Irvine, CA 92614 (US); FINE, Maxwell, Harrison, Irvine, CA 92614 (US); TRAN, Sonny, Irvine, CA 92614 (US); SALEH, Nasser, William, Irvine, CA 92614 (US)
(74) Representative: Eisenführ Speiser
(86) International application number: PCT/US2021/031225
(87) International publication number: WO 2021/226420

(56) References cited:
- US-A1- 2002 173 785
- US-A1- 2008 082 120
- US-A1- 2010 094 392
- US-A1- 2018 256 858

## Description

### CROSS-REFERENCE TO RELATED APPLICATIONS

This application claims the benefit of U.S. Provisional Application No.63/059,764, filed July 31, 2020 and U.S. Provisional Application No. 63/021,945, filed May 8, 2020.

### FIELD

The present application concerns aspects of a sheath for use with catheter-based technologies for repairing and/or replacing heart valves and delivering a prosthetic device, such as a prosthetic valve to a heart via the patient's vasculature.

### BACKGROUND

Endovascular delivery catheter assemblies are used to implant prosthetic devices, such as a prosthetic valve, at locations inside the body that are not readily accessible by surgery or where access without invasive surgery is desirable. For example, aortic, mitral, tricuspid, and/or pulmonary prosthetic valves can be delivered to a treatment site using minimally invasive surgical techniques.

An introducer sheath can be used to safely introduce a delivery apparatus into a patient's vasculature (e.g., the femoral artery). An introducer sheath generally has an elongated sleeve that is inserted into the vasculature and a housing that contains one or more sealing valves that allow a delivery apparatus to be placed in fluid communication with the vasculature with minimal blood loss. A conventional introducer sheath typically requires a tubular loader to be inserted through the seals in the housing to provide an unobstructed path through the housing for a valve mounted on a balloon catheter. A conventional loader extends from the proximal end of the introducer sheath and therefore decreases the available working length of the delivery apparatus that can be inserted through the sheath and into the body.

Conventional methods of accessing a vessel, such as a femoral artery, prior to introducing the delivery system include dilating the vessel using multiple dilators or sheaths that progressively increase in diameter. This repeated insertion and vessel dilation can increase the amount of time the procedure takes, as well as the risk of damage to the vessel.

Radially expanding intravascular sheaths have been disclosed. Such sheaths tend to have complex mechanisms, such as ratcheting mechanisms that maintain the shaft or sheath in an expanded configuration once a device with a larger diameter than the sheath's original diameter is introduced.

US 2018/0256858 A1 (US'858) discloses an expandable delivery sheath which includes an elastic outer tubular layer and an inner tubular layer. The inner tubular layer includes a thick wall portion integrally connected to a thin wall portion. The thin wall portion includes longitudinal reinforcing members/rods that facilitate unfolding during the passage of the implant, thus decreasing the push force and increasing the consistency of the push force. The inner tubular layer has a non-expanded or folded condition wherein the thin wall portion folds onto an outer surface of the thick wall portion under urging of the elastic outer tubular layer. When an implant passes therethrough, the outer tubular layer stretches and the inner tubular layer unfolds into an expanded lumen diameter. Once the implant passes, the outer tubular layer again urges the inner tubular layer into the non-expanded condition with the sheath reassuming its smaller profile (cf. Abstract of US'858).

However, delivery and/or removal of prosthetic devices and other material to or from a patient still poses a significant risk to the patient. Furthermore, accessing the vessel remains a challenge due to the relatively large profile of the delivery system that can cause longitudinal and radial tearing of the vessel during insertion. The delivery system can additionally dislodge calcified plaque within the vessels, posing an additional risk of clots caused by the dislodged plaque.

Accordingly, there remains a need in the art for an improved introducer sheath for endovascular systems used for implanting valves and other prosthetic devices.

### SUMMARY

The claimed invention is defined in independent claim 1 and relates to a sheath for delivering a medical device. Preferred configurations of the claimed sheath are defined in dependent claims 2 to 11. The claimed invention also provides a method of manufacturing a sheath for delivering a medical device as defined in independent claim 12. Dependent claims 13 to 15 define preferred configurations of the claimed method. In the following, 1 inch can be converted into 25,4 mm.

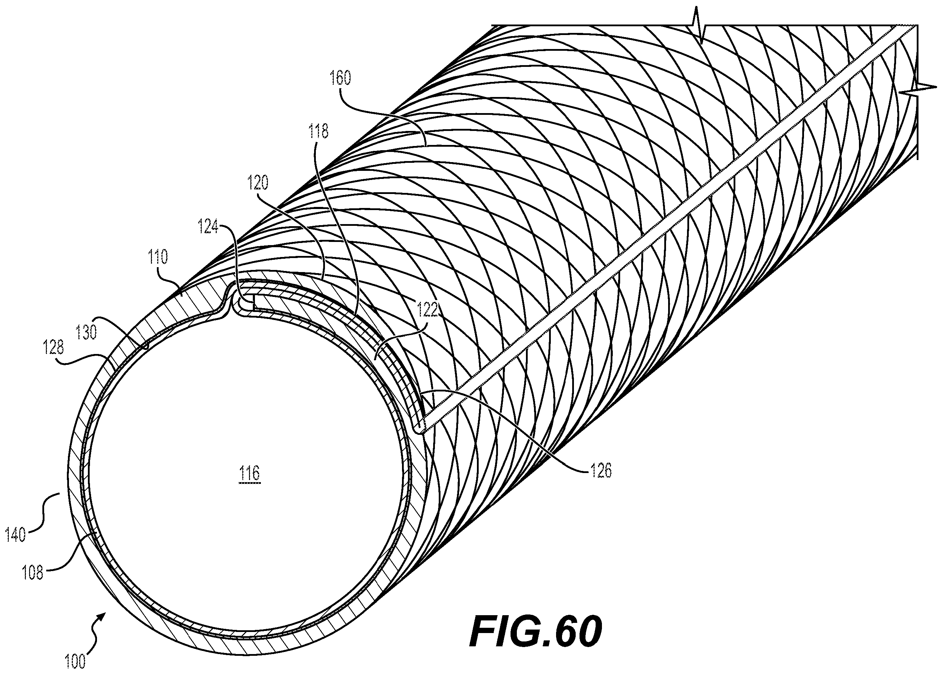

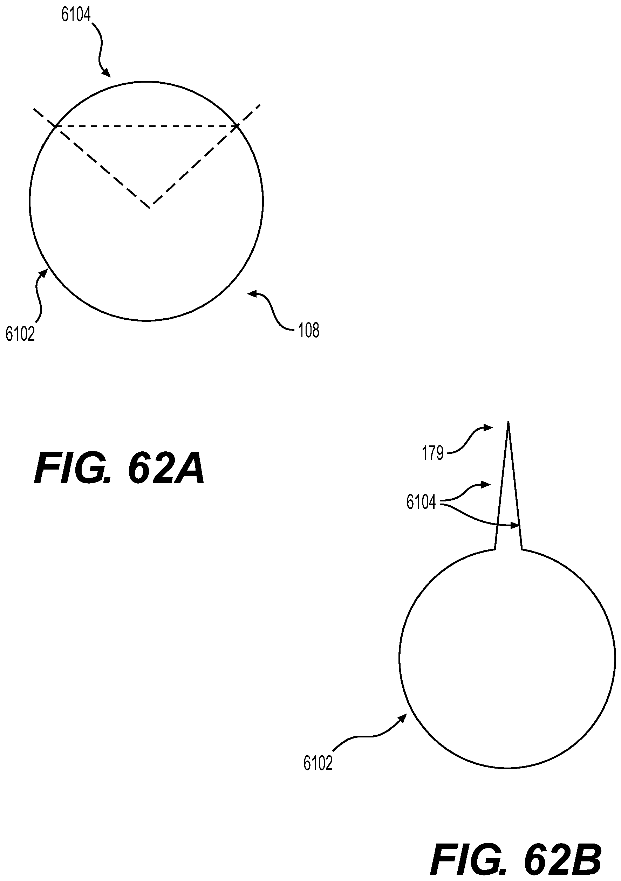

Certain aspects and/or particularly preferred configurations of the claimed invention are discussed hereafter for example in conjunction with Figs. 60 to 63B. Also described herein are related aspects, examples, embodiments and arrangements useful for understanding the claimed invention.

As disclosed herein, in some aspects, the present expandable sheath can minimize trauma to the vessel by allowing for temporary expansion of a portion of the introducer sheath to accommodate a delivery system, followed by a return to the original diameter once the delivery system passes through. In some aspects, disclosed is a sheath with a smaller profile than that of prior art introducer sheaths. Furthermore, the aspects disclosed herein can reduce the length of time a procedure takes, as well as reduce the risk of a longitudinal or radial vessel tear or plaque dislodgement because only one sheath is required, rather than several different sizes of sheaths. The disclosed aspects of the present expandable sheath can require only a single vessel insertion instead of requiring multiple insertions for the dilation of the vessel.

In one aspect, disclosed herein is a sheath for introducing a prosthetic device comprising an inner liner (layer) and an outer layer. At least a portion of the sheath can be designed or configured to locally expand from a first diameter to a second diameter as the prosthetic device is pushed through a lumen of the sheath, and then at least partially return to the first diameter once the prosthetic device has passed through. In some aspects, an elastic outer cover can also be disposed about the outer layer.

In some aspects, disclosed is a sheath comprising a proximal end and a distal end opposite one another. Some aspects disclosed herein can include a hemostasis valve at or near the proximal end of the sheath. In some aspects, the outer diameter of the sheath can decrease along a gradient from the proximal end to the distal end of the sheath. In yet other aspects, the outer diameter of the sheath can be substantially constant along at least a majority of the length of the sheath.

One aspect described herein is directed to a sheath for delivering a medical device, wherein the sheath has a proximal and a distal end and comprises an expandable inner liner having an inner surface and an outer surface, wherein the inner surface of the expandable inner liner defines a lumen and wherein the inner liner comprises at least one folded portion having an inner portion and outer portion; an outer layer having an inner surface and an outer surface and extending at least partially around the inner liner such that a first portion of the outer surface of the outer layer is positioned adjacent to the inner portion of the at least one folded portion of the inner liner, while a first portion of the inner surface of the outer layer is positioned adjacent to the outer portion of the at least one folded portion of the inner liner; and wherein the inner liner comprises at least one first portion and at least one second portion, wherein an outer surface of the at least one first portion comprises a composition and/or morphology that is substantially different from an outer surface of the at least one second portion; and wherein an outwardly directed radial force from a prosthetic device moving through the inner lumen unfolds the at least one folded portion to allow expansion of the sheath. At least a portion of the sheath is configured to expand to accommodate the prosthetic device.

In some aspects, the at least one first portion of the inner liner is etched. While in other aspects, the at least one second portion of the inner liner is unetched.

In certain aspects, the at least one second portion can be etched and subsequently surface-modified such that a composition of the outer surface of the at least one second portion becomes substantially different from a composition of the outer surface of the at least one first portion.

It is understood that in any of the disclosed herein aspects, the outer layer can be configured such that the overlapping portion overlaps the underlying portion, wherein at least a portion of the folded portion of the inner tubular layer is positioned between the overlapping and underlying portions. In some aspects, at least a portion of the sheath is configured such that a plurality of segments of the sheath each locally expands one at a time from a rest configuration having a first diameter to an expanded configuration having a second diameter that is larger than the first diameter to facilitate passage of the prosthetic device through the lumen of the inner liner. Each segment can have a length defined along the longitudinal axis of the sheath, and each segment of the sheath can be configured to at least partially return to the first diameter once the prosthetic device has passed through. In some aspects, when each segment of the sheath is in the expanded configuration, a length of the folded portion corresponding to the length of the segment at least partially unfolds (e.g., by separating and/or straightening). A length of the overlapping portion corresponding to the length of the segment can be configured to move with respect to the underlying portion when each segment of the sheath expands from the rest configuration to the expanded configuration.

In certain aspects, methods of making a sheath are also disclosed. One method can, for example, and without limitations, include providing a mandrel having a first diameter, providing a first tube having a second diameter, the second diameter being larger than the first diameter, mounting the first tube on the mandrel, gathering excess material of the first tube and folding the excess material to one side to form a folded portion of the inner liner. A second tube can then be provided, and the second tube can be cut to form a coiled layer. An adhesive can be applied to at least a portion of the coiled layer, and the coiled layer can be mounted on the first tube such that the adhesive is positioned between the first tube and the coiled layer. The folded portion can be lifted in order to position a portion of the coiled layer under the folded portion.

In some aspects, some methods can also include applying heat to the first tube, coiled layer, and mandrel so as to thermally fuse the first tube and the coiled layer together. In some other aspects, an elastic outer cover can also be secured to the outer surface of the coiled layer. In still further exemplary and unlimiting aspects, a soft tip portion can be coupled to a distal end of the expandable sheath to facilitate passing the expandable sheath through a patient's vasculature.

In another aspect, a method of manufacturing a sheath for delivering a medical device can comprise providing an inner liner having an inner surface and an outer surface, wherein the inner surface defines a lumen therethrough, wherein the inner liner comprises at least one first portion and at least one second portion, wherein an outer surface of the at least one first portion comprises a composition and/or morphology that is substantially different from an outer surface of the at least one second portion; and wherein the inner liner comprises at least one folded portion having an inner portion and outer portion; providing an outer layer having an inner surface and an outer surface such that it extends at least partially around the inner liner such that at least a first portion of the outer surface of the outer layer is positioned adjacent to the inner portion of the at least one folded portion of the inner liner, while a first portion of the inner surface of the outer layer is positioned adjacent to the outer portion of the at least one folded portion of the inner liner; and forming an expandable sheath that is configured to expand when an outwardly directed radial force from a prosthetic device moving through the inner lumen unfolds the at least one folded portion.

In still further aspects, the at least one first portion and at least one second portion of the inner liner can be formed by a selective etching. In such exemplary aspects, the selective etching is performed around the circumference, linearly along a length of the sheath, or a combination thereof.

Disclosed herein is an aspect where the at least one first portion can be formed by masking a portion of the inner liner to form a masked portion and then by etching remaining unmasked portions of the inner liner with an etchant. In still further aspects, the methods as disclosed herein can further comprise a step of forming the at least one second portion by unmasking the masked portion after the etching.

In the aspects, as disclosed herein, the methods comprise selective etching. In such exemplary aspects, the selective etching can be done with an etchant. In such aspects, the etchant can comprise a liquid, a fluid, a gas, a plasma, or a combination thereof.

The foregoing and other features and advantages of the disclosure will become more apparent from the following detailed description, which proceeds with reference to the accompanying figures.

### BRIEF DESCRIPTION OF THE DRAWINGS

**FIG. 1** is an elevation view of a sheath according to the present disclosure along with an endovascular delivery apparatus for implanting a prosthetic valve.
**FIGS. 2A****, B,** and **D** are section views of a sheath in various aspects for introducing a prosthetic device into a patient, and **FIG. 2C** is a perspective view of one component of such a sheath.
**FIG. 3** is an elevation view of the sheath shown in **FIG. 2****.**
**FIGS. 4A-4B** are elevation views of two aspects of a sheath according to the present disclosure, having varying outer diameters.
**FIG. 5** illustrates an elevation view of one aspect of a sheath, expanded at a first location to accommodate a delivery system.
**FIG. 6** shows an elevation view of the sheath in one aspect, expanded at a second location farther down the sheath.
**FIG. 7** shows a section view of another aspect of a sheath that further comprises an outer covering or shell.
**FIG. 8** illustrates an elevation view of one aspect of a sheath with an outer covering or shell.
**FIG. 9** illustrates a partial elevation view of one aspect of an intermediate tubular layer that can be used to construct a sheath according to the present disclosure.
**FIG. 10** illustrates a partial elevation view of another aspect of an intermediate tubular layer having a variable diamond design.
**FIG. 11** illustrates a partial elevation view of another aspect of an intermediate tubular layer having a diamond design with spring struts.
**FIG. 12** illustrates a partial elevation view of another aspect of an intermediate tubular layer having a diamond design with straight struts.
**FIG. 13** illustrates a partial elevation view of another aspect of an intermediate tubular layer having a saw tooth design with spring struts.
**FIG. 14** illustrates a partial elevation view of another aspect of an intermediate tubular layer having a saw tooth design with straight struts.
**FIG. 15** illustrates a partial elevation view of another aspect of an intermediate tubular layer having a diamond design with straight struts.
**FIG. 16** illustrates a partial elevation view of another aspect of an intermediate tubular layer having a helical or spiral design.
**FIG. 17** illustrates a partial elevation view of another aspect of an intermediate tubular layer having a diamond design with non-straight struts.
**FIG. 18** illustrates a partial elevation view of another aspect of an intermediate tubular layer having an alternative diamond design with non-straight struts.
**FIG. 19** illustrates a partial elevation view of another aspect of an intermediate tubular layer having yet another diamond design with non-straight struts.
**FIG. 20** illustrates a partial elevation view of another aspect of an intermediate tubular layer having a diamond design with struts.
**FIG. 21** illustrates a partial elevation view of another aspect of an intermediate tubular layer having a design similar to that shown in **FIG. 20** but with additional struts.
**FIG. 22** illustrates a partial elevation view of another aspect of an intermediate tubular layer having a diamond design with spiral struts.
**FIG. 23** illustrates a partial elevation view of another aspect of an intermediate tubular layer having a diamond design with adjacent struts.
**FIG. 24** illustrates a section view of one aspect of a sheath having a longitudinal notch.
**FIG. 25** shows a section view of one aspect of a sheath having a longitudinal cut in the inner liner.
**FIG. 26** shows a perspective view of one aspect of a sheath having a plurality of notches or cuts in the outer tubular layer.
**FIG. 27A** illustrates a section view of one aspect of a sheath, wherein the outer tubular layer contains a longitudinal cut, and the inner liner extends into the gap created by the cut in the outer tubular layer, in an unexpanded configuration; and **FIGS. 27B-27E** show section views of various aspects of a sheath in the unexpanded configuration.
**FIG. 28** shows a section view of the sheath of **FIG. 27A** in an expanded configuration.
**FIGS. 29A-29D** show section views of various aspects of a sheath having overlapping sections.
**FIG. 30** illustrates a block diagram of one aspect of a method of making a sheath according to the present disclosure.
**FIG. 31** illustrates a block diagram of another aspect of a method of making a sheath according to the present disclosure.
**FIGS. 32A-32H** illustrates section or elevation views of various method steps of the methods shown in **FIGS. 30-31****.**
**FIG. 33** illustrates a plan view of one aspect of a sheath having a partial slit or score line.
**FIG. 34** illustrates a plan view of another aspect of a sheath having a partial slit or score line.
**FIG. 35** is an elevation view of an expandable sheath according to the present disclosure and a representative housing.
**FIG. 36** is an enlarged cutaway view of the distal end of the sheath of **FIG. 35****.**
**FIG. 37** is a section view of the distal end of the sheath of **FIG. 35****,** taken along line **37-37** in **FIG. 36.**
**FIG. 38** is a section view of a proximal section of the sheath of **FIG. 35****,** taken along line **38-38** in **FIG. 35****.**
**FIG. 39** is a section view of the sheath of **FIG. 35** in a rest (unexpanded) configuration, taken along line **39-39** in **FIG. 35****.**
**FIG. 40** is the section view of the sheath of **FIG. 39****,** in an expanded configuration.
**FIG. 41** shows an elevation view of an expandable sheath having an elastic outer cover, according to another aspect.
**FIG. 42** illustrates a section view of the sheath of **FIG. 41****,** taken along line **42-42 in** **FIG. 41****.**
**FIG. 43** illustrates a section view of the sheath shown in **FIG. 42****,** in an expanded configuration.
**FIG. 44** illustrates a section view of another aspect of an expandable sheath.
**FIG. 45** shows an expanded configuration of the sheath of **FIG. 44****.**
**FIG. 46** illustrates a section view of another aspect of an expandable sheath.
**FIG. 47** shows an expanded configuration of the sheath of **FIG. 46****.**
**FIG. 48** illustrates a section view of another aspect of an expandable sheath according to the present disclosure.
**FIG. 49** illustrates a section view of another aspect of an expandable sheath.
**FIG. 50** is a section view of an example sheath in an unexpanded configuration.
**FIG. 51** is a section view of the sheath of **FIG. 50** in an expanded configuration.
**FIG. 52** is a section view of the sheath of **FIG. 50****,** including an outer jacket.
**FIG. 53** is a section view of the sheath of **FIG. 35** in a rest (unexpanded) configuration, including an outer jacket, taken along line **39-39** in **FIG. 35****.**
**FIG. 54** is a section view of the sheath of **FIG. 53** in a rest (unexpanded) configuration, taken along line **39-39** in **FIG. 35****.**
**FIG. 55** is a section view of the sheath of **FIG. 54****,** in an expanded configuration.
**FIG. 56** is a section view of the sheath of **FIG. 54** in a rest (unexpanded) configuration, including a lubricant between the outer layer and the outer jacket.
**FIG. 57** is a section view of the sheath of **FIG. 54** in a rest (unexpanded) configuration, including a lubricant and a bonding strip.
**FIG. 58** is a bottom perspective view of the sheath of **FIG. 57****.**
**FIG. 59** is a bottom perspective view of the sheath of **FIG. 57****.**
**FIG. 60** is a top perspective view of the sheath of **FIG. 54****.**
**FIGS.61A-B** is a schematic view of an inner liner having first and second portions as disclosed in one aspect.
**FIGS. 62A-B** is a schematic view of an inner liner having first and second portions as disclosed in one aspect.
**FIGS. 63A-B** is a schematic view of an inner liner having first and second portions as disclosed in one aspect.

### DETAILED DESCRIPTION

The present disclosure can be understood more readily by reference to the following detailed description, examples, drawings, and claims, and their previous and following description. However, before the present articles, systems, and/or methods are disclosed and described, it is to be understood that this disclosure is not limited to the specific or exemplary aspects of articles, systems, and/or methods disclosed unless otherwise specified, as such can, of course, vary. It is also to be understood that the terminology used herein is for the purpose of describing particular aspects only and is not intended to be limiting.

The following description of the disclosure is provided as an enabling teaching of the disclosure in its best, currently known aspect. To this end, those skilled in the relevant art will recognize and appreciate that many changes can be made to the various aspects of the disclosure described herein while still obtaining the beneficial results of the present disclosure. It will also be apparent that some of the desired benefits of the present disclosure can be obtained by selecting some of the features of the present disclosure without utilizing other features. Accordingly, those of ordinary skill in the pertinent art will recognize that many modifications and adaptations to the present disclosure are possible and may even be desirable in certain circumstances and are a part of the present disclosure. Thus, the following description is again provided as illustrative of the principles of the present disclosure and not in limitation thereof.

### DEFINITIONS

As used in this application and the claims, the singular forms "a," "an," and "the" include the plural forms unless the context clearly dictates otherwise. Thus, for example, reference to a "polymer" includes aspects having two or more such polymers unless the context clearly indicates otherwise.

It is appreciated that certain features of the disclosure, which are, for clarity, described in the context of separate aspects, can also be provided in combination or as a single aspect. Conversely, various features of the disclosure, which are, for brevity, described in the context of a single aspect, can also be provided separately or in any suitable combination.

As used herein, the terms "optional" or "optionally" mean that the subsequently described event or circumstance may or may not occur and that the description includes instances where said event or circumstance occurs and instances where it does not.

It is also to be understood that the terminology used herein is for the purpose of describing particular aspects only and is not intended to be limiting. As used in the specification and the claims, the term "comprising" can include the aspects "consisting of" and "consisting essentially of." Additionally, the term "includes" means "comprises."

For the terms "for example" and "such as," and grammatical equivalences thereof, the phrase "and without limitation" is understood to follow unless explicitly stated otherwise.

References in the specification and concluding claims to parts by weight of a particular element or component in a composition or article denotes the weight relationship between the element or component and any other elements or components in the composition or article for which a part by weight is expressed. Thus, in a composition or a selected portion of a composition containing 2 parts by weight of component X and 5 parts by weight component Y, X and Y are present at a weight ratio of 2:5 and are present in such ratio regardless of whether additional components are contained in the composition.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements. Furthermore, when numerical ranges of varying scope are set forth herein, it is contemplated that any combination of these values inclusive of the recited values may be used. Further, ranges can be expressed herein as from "about" one particular value and/or to "about" another particular value. When such a range is expressed, another aspect includes from the one particular value and/or to the other particular value.

Similarly, when values are expressed as approximations, by use of the antecedent "about," it will be understood that the particular value forms another aspect. It will be further understood that the endpoints of each of the ranges are significant both in relation to the other endpoint and independently of the other endpoint. Unless stated otherwise, the term "about" means within 5% (e.g., within 2% or 1%) of the particular value modified by the term "about."

Throughout this disclosure, various aspects of the disclosure can be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6, etc., as well as individual numbers within that range, for example, 1, 2, 2.7, 3, 4, 5, 5.3, 6 and any whole and partial increments therebetween. This applies regardless of the breadth of the range.

As used herein, the term "composition" is intended to encompass a product comprising the specified ingredients in the specified amounts, as well as any product which results, directly or indirectly, from a combination of the specified ingredients in the specified amounts.

A weight percent of a component, unless specifically stated to the contrary, is based on the total weight of the formulation or composition in which the component is included.

As used herein, the term "substantially" means that the subsequently described event or circumstance completely occurs or that the subsequently described event or circumstance generally, typically, or approximately occurs.

As used herein, the term "substantially," when used in reference to a composition, refers to at least about 80%, at least about 85%, at least about 90%, at least about 91%, at least about 92%, at least about 93%, at least about 94%, at least about 95%, at least about 96%, at least about 97%, at least about 98%, at least about 99%, or about 100% by weight, based on the total weight of the composition, of a specified feature or component.

As used herein, the term "substantially," in, for example, the context "substantially free" refers to a composition having less than about 1 % by weight, e.g., less than about 0.5 % by weight, less than about 0.1 % by weight, less than about 0.05 % by weight, or less than about 0.01 % by weight of the stated material, based on the total weight of the composition.

As used herein, the terms "substantially identical reference composition" or "substantially identical reference article" refer to a reference composition or article comprising substantially identical components in the absence of an inventive component. In another exemplary aspect, the term "substantially," in, for example, the context "substantially identical reference composition," refers to a reference composition comprising substantially identical components and wherein an inventive component is substituted with a common in the art component.

Further, the terms "coupled" and "associated" generally mean electrically, electromagnetically, and/or physically (e.g., mechanically or chemically) coupled or linked and does not exclude the presence of intermediate elements between the coupled or associated items.

It will be understood that, although the terms "first," "second," etc., may be used herein to describe various elements, components, regions, layers and/or sections. These elements, components, regions, layers, and/or sections should not be limited by these terms. These terms are only used to distinguish one element, component, region, layer, or section from another element, component, region, layer, or a section. Thus, a first element, component, region, layer, or section discussed below could be termed a second element, component, region, layer, or section without departing from the teachings of exemplary aspects.

It is understood that the terms "layer" and "liner" can be used interchangeably. For example, the aspects describing an "inner liner" also include aspects describing an "inner layer." Similarly, the aspects describing an "outer layer" also include aspects describing an "outer liner.".

Spatially relative terms, such as "beneath," "below," "lower," "above," "upper," and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the term "below" can encompass both an orientation of above and below. The device may be otherwise oriented (rotated 90 degrees or at other orientations), and the spatially relative descriptors used herein are interpreted accordingly.

As used herein, the term "atraumatic" is commonly known in the art and refers to a device or a procedure that minimized tissue injury.

Although the operations of exemplary aspects of the disclosed method can be described in a particular, sequential order for convenient presentation, it should be understood that disclosed aspects can encompass an order of operations other than the particular, sequential order disclosed. For example, operations described sequentially may, in some cases, be rearranged or performed concurrently. Further, descriptions and disclosures provided in association with one particular aspect are not limited to that aspect and may be applied to any aspect disclosed.

While aspects of the present disclosure can be described and claimed in a particular statutory class, such as the system statutory class, this is for convenience only, and one of ordinary skill in the art will understand that each aspect of the present disclosure can be described and claimed in any statutory class. Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is in no way intended that an order be inferred in any respect. This holds for any possible non-express basis for interpretation, including matters of logic with respect to arrangement of steps or operational flow, plain meaning derived from grammatical organization or punctuation, or the number or type of aspects described in the specification.

Moreover, for the sake of simplicity, the attached figures may not show the various ways (readily discernable, based on this disclosure, by one of ordinary skill in the art) in which the disclosed system, method, and apparatus can be used in combination with other systems, methods, and apparatuses. Additionally, the description sometimes uses terms such as "produce" and "provide" to describe the disclosed method. These terms are high-level abstractions of the actual operations that can be performed. The actual operations that correspond to these terms can vary depending on the particular implementation and are, based on this disclosure, readily discernible by one of ordinary skill in the art.

The present disclosure may be understood more readily by reference to the following detailed description of various aspects of the disclosure and the examples included therein and to the Figures and their previous and following description.

The present disclosure may be understood more readily by reference to the following detailed description of various aspects of the disclosure and the examples included therein and to the Figures and their previous and following description.

Disclosed aspects of an expandable sheath can minimize trauma to the vessel by allowing for temporary expansion of a portion of the introducer sheath to accommodate the delivery system, followed by a return to the original diameter once the device passes through. In some aspects, disclosed herein is a sheath with a smaller profile (e.g., a smaller diameter in the rest configuration) than that of prior art introducer sheaths. Furthermore, the disclosed herein aspects can reduce the length of time a procedure takes, as well as reduce the risk of a longitudinal or radial vessel tear or plaque dislodgement because only one sheath is required, rather than several different sizes of sheaths. Aspects of the present expandable sheath can avoid the need for multiple insertions for the dilation of the vessel. Such expandable sheaths can be useful for many types of minimally invasive surgery, such as any surgery requiring introduction of an apparatus into a subject's vessel. For example, the sheath can be used to introduce other types of delivery apparatus for placing various types of intraluminal devices (e.g., stents, prosthetic heart valves, stented grafts, etc.) into many types of vascular and non-vascular body lumens (*e.g.*, veins, arteries, esophagus, ducts of the biliary tree, intestine, urethra, fallopian tube, other endocrine or exocrine ducts, etc.).

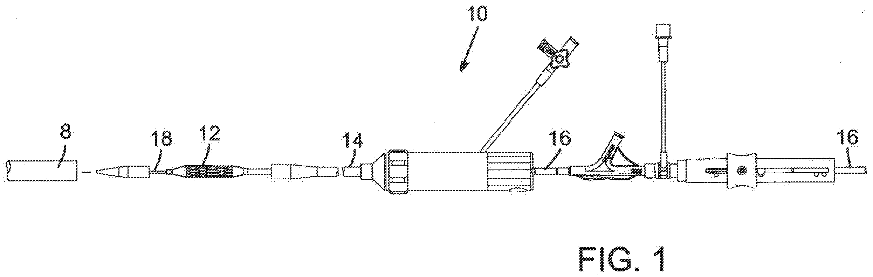

**FIG. 1** illustrates a sheath **8** according to the present disclosure, in use with a representative delivery apparatus **10,** for delivering a prosthetic device **12,** such as a tissue heart valve to a patient. The apparatus **10** can include a steerable guide catheter **14** (also referred to as a flex catheter), a balloon catheter **16** extending through the guide catheter **14,** and a nose catheter **18** extending through the balloon catheter **16.** The guide catheter **14,** the balloon catheter **16,** and the nose catheter **18** in the illustrated aspect are adapted to slide longitudinally relative to each other to facilitate delivery and positioning of the valve **12** at an implantation site in a patient's body as described in detail below. Generally, sheath **8** is inserted into a vessel, such as the transfemoral vessel, passing through the skin of the patient, such that the distal end of the sheath **8** is inserted into the vessel. Sheath **8** can include a hemostasis valve at the opposite, proximal end of the sheath. The delivery apparatus **10** can be inserted into the sheath **8,** and the prosthetic device **12** can then be delivered and implanted within the patient.

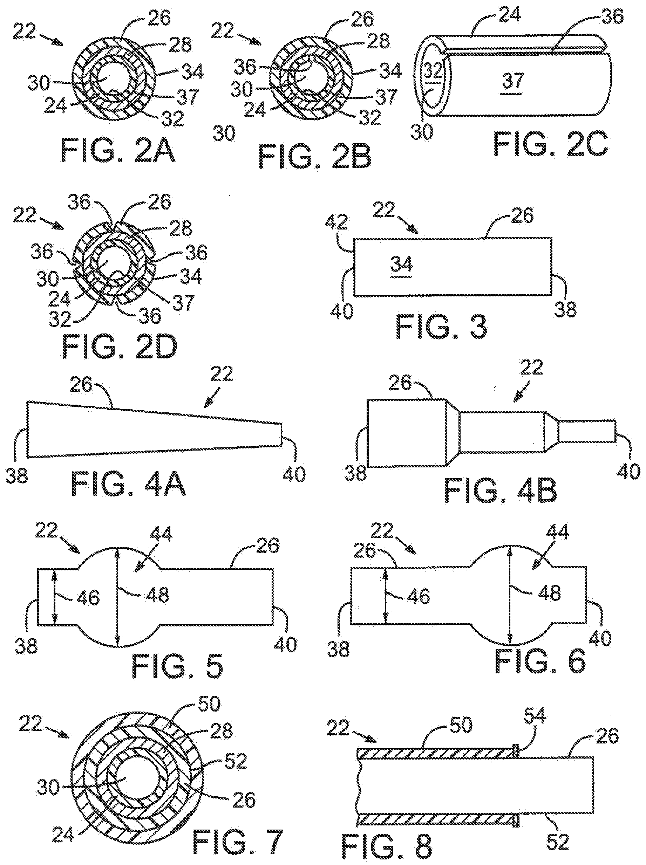

**FIGS. 2A, 2B,** and **2D** show section views of exemplary aspects of a sheath **22** for use with a delivery apparatus such as that shown in **FIG. 1****.** Example expandable sheaths are also disclosed in U.S. Patent Application No. 12/249,867, filed October 10, 2008 (now U.S. Patent No. 8,690,936), U.S. Patent Application No. 13/312,739, filed December 6, 2011 (now U.S. Patent No. 8,790,387), U.S. Patent Application No. 14/248,120 filed on April 8, 2014 (now U.S. Patent No. 9,301,840), U.S. Patent Application No. 14/324,894, filed July 7, 2014 (now U.S. Patent No. 9,301,841), U.S. Patent Application No. 15/057,953, filed March 1, 2016 (now U.S. Patent No. 9,987,134), U.S. Patent Application No. 15/997,587, filed June 4, 2018, U.S. Patent Application No. 16/149,953, filed on October 2, 2018 (now U.S. Patent No. 10,524,905), U.S. Patent Application No. 16/149,956, filed on October 2, 2018 (now U.S. Patent No. 10,517,720), U.S. Patent Application No. 16/149,960, filed on October 2, 2018 (now U.S. Patent No. 10,524,906, and U.S. Patent Application No. 16/149,969, filed on October 2, 2018 (now U.S. Patent No. 10,524,907).

**FIG. 2C** shows a perspective view of one aspect of an inner liner **24** for use with the sheath **22.** Sheath **22** includes an inner liner, such as inner polymeric tubular layer **24,** an outer layer, such as outer polymeric tubular layer **26,** and an intermediate tubular layer **28** disposed between the inner and outer polymeric tubular layers **24, 26.** The sheath **22** defines a lumen **30** through which a delivery apparatus can travel into a patient's vessel in order to deliver, remove, repair, and/or replace a prosthetic device. Such introducer sheaths **22** can also be useful for other types of minimally invasive surgery, such as any surgery requiring introduction of an apparatus into a subject's vessel. For example, the sheath **22** also can be used to introduce other types of delivery apparatus for placing various types of intraluminal devices (*e.g.,* stents, stented grafts, etc.) into many types of vascular and non-vascular body lumens (*e.g.,* veins, arteries, esophagus, ducts of the biliary tree, intestine, urethra, fallopian tube, other endocrine or exocrine ducts, etc.).

The outer polymeric tubular layer **26** and the inner polymeric tubular layer **24** can comprise, for example, PTFE (e.g., Teflon^{®}), polyimide, PEEK, polyurethane, nylon, polyethylene, polyamide, polyether block amides (e.g., PEBAX^{®}), polyether block ester copolymer, polyesters, fluoropolymers, polyvinyl chloride, thermoset silicone, latex, poly-isoprene rubbers, polyolefin, other medical grade polymers, or combinations thereof. The intermediate tubular layer **28** can comprise a shape memory alloy such as Nitinol and/or stainless steel, cobalt chromium, spectra fiber, polyethylene fiber, aramid fiber, or combinations thereof.

The inner polymeric tubular layer **24** can advantageously be provided with a low coefficient of friction on its inner surface. For example, the inner polymeric tubular layer **24** can have a coefficient of friction of less than about 0.1. Some aspects of a sheath **22** can include a lubricious liner on the inner surface **32** of the inner polymeric tubular layer **24.** Such a liner can facilitate passage of a delivery apparatus through the lumen **30** of the sheath **22.** Examples of suitable lubricious liners include materials that can reduce the coefficient of friction of the inner polymeric tubular layer **24,** such as PTFE, polyethylene, polyvinylidene fluoride, and combinations thereof. Suitable materials for a lubricious liner also include other materials desirably having a coefficient of friction of about 0.1 or less.

The inner diameter of the intermediate tubular layer **28** varies depending on the application and size of the delivery apparatus and prosthetic device. In some aspects, the inner diameter ranges from about 0.005 inches to about 0.400 inches. The thickness of the intermediate tubular layer **28** can be varied depending on the desired amount of radial expansion, as well as the strength required. For example, the thickness of the intermediate tubular layer **28** can be from about 0.002 inches to about 0.025 inches. The thicknesses of the inner polymeric tubular layer 24 and the outer polymeric tubular layer **26** can also be varied depending on the particular application of the sheath **22.** In some aspects, the thickness of the inner polymeric tubular layer **24** ranges from about 0.0005 inches to about 0.010 inches, and in one particular aspect, the thickness is about 0.002 inches. Outer polymeric tubular layer **26** can have a thickness of from about 0.002 inches to about 0.015 inches, and in one particular aspect, the outer polymeric tubular layer **26** has a thickness of about 0.010 inches.

The hardness of each layer of the sheath **22** can also be varied depending on the particular application and desired properties of the sheath **22.** In some aspects, the outer polymeric tubular layer **26** has a Shore hardness of from about 25 Durometer to about 75 Durometer.

Additionally, some aspects of a sheath **22** can include an exterior hydrophilic coating on the outer surface **34** of the outer polymeric tubular layer **26.** Such a hydrophilic coating can facilitate insertion of the sheath **22** into a patient's vessel. Examples of suitable hydrophilic coatings include the Harmony^{™} Advanced Lubricity Coatings and other Advanced Hydrophilic Coatings available from SurModics, Inc., Eden Prairie, MN. DSM medical coatings (available from Koninklijke DSM N.V, Heerlen, the Netherlands), as well as other hydrophilic coatings, are also suitable for use with the sheath **22.**

In some aspects, the outer surface **34** of the outer polymeric tubular layer **26** can be modified. For example, surface modifications such as plasma etching can be performed on the outer surface **34.** Similarly, other surfaces, both outer and inner, can be surface modified according to certain aspects and desired application. In some aspects, surface modification can improve adhesion between the layers in the areas of the modification.

**The** sheath **22** also can have at least one radiopaque filler or marker. The radiopaque filler or marker can be associated with the outer surface **34** of the outer polymeric tubular layer **26.** Alternatively, the radiopaque filler or marker can be embedded or blended within the outer polymeric tubular layer **24.** Similarly, the radiopaque filler or marker can be associated with a surface of the inner polymeric tubular layer **24** or the intermediate tubular layer **28** or embedded within either or both of those layers.

Suitable materials for use as a radiopaque filler or marker include, for example, barium sulfite, bismuth trioxide, titanium dioxide, bismuth subcarbonate, or combinations thereof. The radiopaque filler can be mixed with or embedded in the material used to form the outer polymeric tubular layer **26** and can comprise from about 5% to about 45% by weight of the outer polymeric tubular layer. More or less radiopaque material can be used in some aspects, depending on the particular application.

In some aspects, the inner polymeric tubular layer **24** can comprise a substantially uniform cylindrical tube. In alternative aspects, the inner polymeric tubular layer **24** can have at least one section of discontinuity along its longitudinal axis to facilitate radial expansion of the inner polymeric tubular layer **24.** For example, the inner polymeric tubular layer **24** can be provided with one or more longitudinal notches and/or cuts **36** extending along at least a portion of the length of the sheath **22.** Such notches or cuts **36** can facilitate radial expansion of the inner polymeric tubular layer **24,** thus accommodating passage of a delivery apparatus or other device. Such notches and/or cuts **36** can be provided near the inner surface **32,** near the outer surface **37,** and/or substantially through the entire thickness of the inner polymeric layer **24.** In aspects with a plurality of notches and/or cuts **36,** such notches and/or cuts **36** can be positioned such that they are substantially equally spaced from one another circumferentially around the inner polymeric layer **24.** Alternatively, notches and cuts **36** can be spaced randomly in relation to one another or in any other desired pattern. Some or all of any provided notches and/or cuts **36** can extend longitudinally along substantially the entire length of the sheath **22.** Alternatively, some or all of any provided notches and/or cuts **36** can extend longitudinally only along a portion of the length of the sheath **22.**

**As** shown in **FIGS. 2B** and **2C** (which illustrates only the inner polymeric tubular layer **24),** in some aspects, the inner polymeric tubular layer **24** contains at least one notch or cut **36** that extends longitudinally and parallel to an axis defined by the lumen **30,** extending substantially the entire length of the sheath **22.** Thus, upon introduction of a delivery apparatus, the inner polymeric tubular layer **24** can split open along the notch and/or cut **36** and expand, thus accommodating the delivery apparatus.

Additionally or alternatively, as shown in **FIG. 2D****,** the outer polymeric tubular layer **26** can comprise one or more notches and/or cuts **36.** Notches and/or cuts **36,** in some aspects, do not extend through the entire thickness of the outer tubular layer **26.** The notches and/or cuts **36** can be separable upon radial expansion of the sheath **22.** The outer polymeric tubular layer **26** can be retractable longitudinally or able to be pulled back away from the intermediate tubular layer **28** and the inner polymeric tubular layer **24.** In aspects with a retractable outer polymeric tubular layer **26,** the outer polymeric tubular layer **26** can be retracted to accommodate or facilitate passage of a delivery apparatus through the lumen **30** and then can be replaced to its original position on the sheath **22.**

**FIG. 3** illustrates an elevation view of the sheath **22** shown in **FIG. 2A**. In this view, only the outer polymeric tubular layer **26** is visible. The sheath **22** comprises a proximal end **38** and a distal end 40 opposite the proximal end **38.** The sheath **22** can include a hemostasis valve inside the lumen of the sheath **22,** at or near the proximal end **38** of the sheath **22.**

Additionally, the sheath **22** can comprise a soft tip **42** at the distal end **40** of the sheath **22.** Such a soft tip **42** can be provided with a lower hardness than the other portions of the sheath **22.** In some aspects, the soft tip **42** can have a Shore hardness from about 25 D to about 40 D.

As shown in **FIG. 3**, the unexpanded original outer diameter of the sheath **22** can be substantially constant across the length of the sheath **22,** substantially from the proximal end **38** to the distal end **40.** In alternative aspects, such as the ones illustrated in **FIGS. 4A-4B****,** the original unexpanded outer diameter of the sheath **22** can decrease from the proximal end **38** to the distal end **40.** As shown in the aspect in **FIG. 4A****,** the original unexpanded outer diameter can decrease along a gradient, from the proximal end **38** to the distal end **40.** In alternative aspects, such as the one shown in **FIG. 4B****,** the original unexpanded outer diameter of sheath **22,** can incrementally step down along the length of the sheath **22,** wherein the largest original unexpanded outer diameter is near the proximal end **38,** and the smallest original unexpanded outer diameter is near the distal end **40** of the sheath **22.**

As shown in **FIGS. 5-6****,** the sheath **22** can be designed to locally expand as the prosthetic device is passed through the lumen of the sheath **22** and then substantially return to its original shape once the prosthetic device has passed through that portion of the sheath **22**. For example, **FIG. 5** illustrates a sheath **22** having a localized bulge **44,** representative of a device being passed through the internal lumen of the sheath **22.** **FIG. 5** shows the device close to the proximal end **38** of the sheath **22,** close to the area where the device is introduced into the sheath **22**. **FIG. 6** shows the sheath **22** of **FIG. 5**, with the device having progressed further along the sheath **22.** The localized bulge **44** is now closer to the distal end **40** of the sheath **22,** and thus, is about to be introduced to a patient's vessel. As evident from **FIGS. 5** and **6****,** once the localized bulge associated with the device has passed through a portion of the lumen of the sheath **22,** that portion of the sheath **22** can automatically return to its original shape and size, at least in part due to the materials and structure of the sheath **22.**

The sheath **22** has an unexpanded inner diameter equal to the inner diameter of the inner polymeric tubular layer (not visible in **FIGS. 5-6**) and an unexpanded outer diameter **46** equal to the outer diameter of the outer polymeric tubular layer **26.** The sheath **22** is designed to be expanded to an expanded inner diameter and an expanded outer diameter **48,** which are larger than the unexpanded inner diameter and the unexpanded outer diameter **46,** respectively. In one representative aspect, the unexpanded inner diameter is about 16 Fr, and the unexpanded outer diameter **46** is about 19 Fr, while the expanded inner diameter is about 26 Fr, and the expanded outer diameter **48** is about 29 Fr. Different sheaths 22 can be provided with different expanded and unexpanded inner and outer diameters, depending on the size requirements of the delivery apparatus for various applications. Additionally, some aspects can provide more or less expansion depending on the particular design parameters, the materials, and/or configurations used.

In some aspects of a sheath according to the present disclosure, and as shown in section in **FIG. 7** and in elevation in **FIG. 8**, the sheath **22** can additionally comprise an outer covering, such as outer polymeric covering **50,** disposed on the outer surface **52** of the outer polymeric tubular layer **26.** The outer polymeric covering **50** can provide a protective covering for the underlying sheath **22.** In some aspects, the outer polymeric covering **50** can contain a self-expandable sheath in a crimped or constrained state and then release the self-expandable sheath upon removal of the outer polymeric covering **50.** For example, in some aspects of a self-expandable sheath, the intermediate layer **28** can comprise Nitinol and/or other shape memory alloys, and the intermediate layer **28** can be crimped or radially compressed to a reduced diameter within the outer polymeric tubular layer **26** and the outer polymeric covering **50.** Once the self-expandable sheath is at least partially inserted into a patient's vessel, the outer polymeric covering **50** can be slid back, peeled away, or otherwise at least partially removed from the sheath. To facilitate removal of the outer polymeric covering **50,** a portion of the outer polymeric covering **50** can remain outside the patient's vessel, and that portion can be pulled back or removed from the sheath to allow the sheath to expand. In some aspects, substantially the entire outer polymeric covering **50** can be inserted, along with the sheath, into a patient's vessel. In these aspects, an external mechanism attached to the outer polymeric covering **50** can be provided, such that the outer polymeric covering can be at least partially removed from the sheath once the sheath is inserted into a patient's vessel.

Once no longer constrained by the outer polymeric covering **50,** the radially compressed intermediate layer **28** can self-expand, causing expansion of the sheath along the length of the intermediate layer **28.** In some aspects, portions of the sheath can radially collapse, at least partially returning to the original crimped state, as the sheath is being withdrawn from the vessel after completion of the surgical procedure. In some aspects, such collapse can be facilitated and/or encouraged by an additional device or layer that, in some aspects, can be mounted onto a portion of the sheath prior to the sheath's insertion into the vessel.

**The** outer polymeric covering **50,** in some aspects, is not adhered to the other layers of the sheath **22.** For example, the outer polymeric covering **50** may be slidable with respect to the underlying sheath, such that it can be easily removed or retracted from its initial position on the sheath **22.**

**As** seen in **FIG. 8****,** the outer polymeric covering 50 can include one or more peel tabs **54** to facilitate manual removal of the outer polymeric covering **50.** The outer polymeric covering **50** can be automatically or manually retractable and/or splittable to facilitate radial expansion of the sheath **22.** Peel tabs **54** can be located approximately 90 degrees from any cut or notch present in the outer polymeric covering **50,** and approximately 180 degrees offset from one another. In alternative aspects, the peel tabs **54** can extend substantially around the circumference of the outer polymeric covering **50,** thus resulting in a single circular peel tab **54.**

Suitable materials for the outer polymeric covering **50** are similar to those materials suitable for the inner polymeric tubular layer and the outer polymeric tubular layer and can include PTFE and/or high density polyethylene.

Turning now to the intermediate tubular layer **28,** several different configurations are possible. The intermediate tubular layer **28** is generally a thin, hollow, substantially cylindrical tube comprising an arrangement, pattern, structure, or configuration of wires or struts, however, other geometries can also be used. The intermediate tubular layer **28** can extend along substantially the entire length of the sheath **22,** or alternatively, can extend only along a portion of the length of sheath **22.** Suitable wires can be round, ranging from about 0.0005 inches thick to about 0.10 inches thick, or flat, ranging from about 0.0005 inches x 0.003 inches to about 0.003 inches x 0.007 inches. However, other geometries and sizes are also suitable for certain aspects. If a braided wire is used, the braid density can be varied. Some aspects have a braid density of about thirty picks per inch to about eighty picks per inch and can include up to thirty-two wires in various braid patterns.

One representative aspect of an intermediate tubular layer comprises a braided Nitinol composite, which is at least partially encapsulated by an inner polymeric tubular member and an outer polymeric tubular member disposed on inner and outer surfaces of the intermediate tubular layer, respectively. Such encapsulation by polymeric layers can be accomplished by, for example, fusing the polymeric layers to the intermediate tubular layer or dip coating the intermediate tubular layer. In some aspects, an inner polymeric tubular member, an intermediate tubular layer, and an outer polymeric tubular layer can be arranged on a mandrel, and the layers can then be thermally fused or melted into one another by placing the assembly in an oven or otherwise heating it. The mandrel can then be removed from the resulting sheath. In other aspects, dip coating can be used to apply an inner polymeric tubular member to the surface of a mandrel. The intermediate tubular layer can then be applied, and the inner polymeric tubular member allowed to cure. The assembly can then be dip coated again, such as to apply a thin coating of, for example, polyurethane, which will become the outer polymeric tubular member of the sheath. The sheath can then be removed from the mandrel.

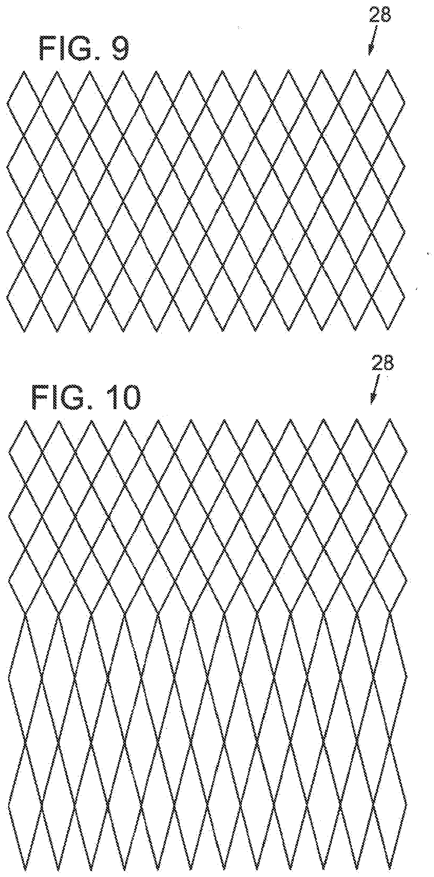

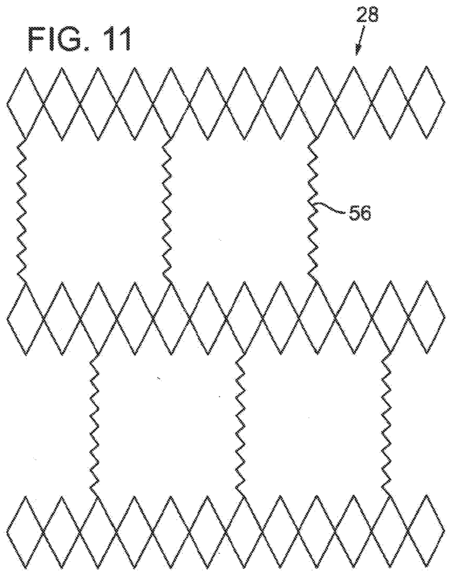

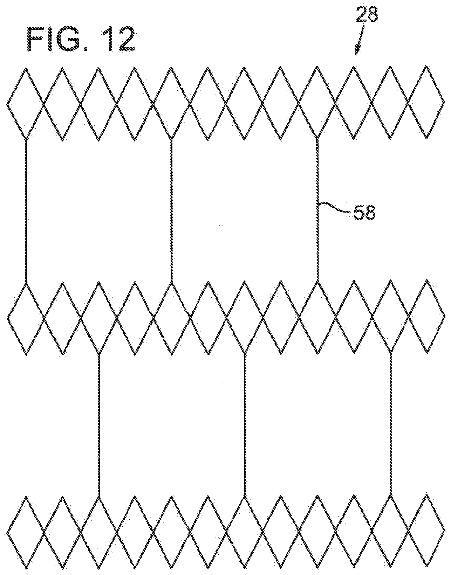

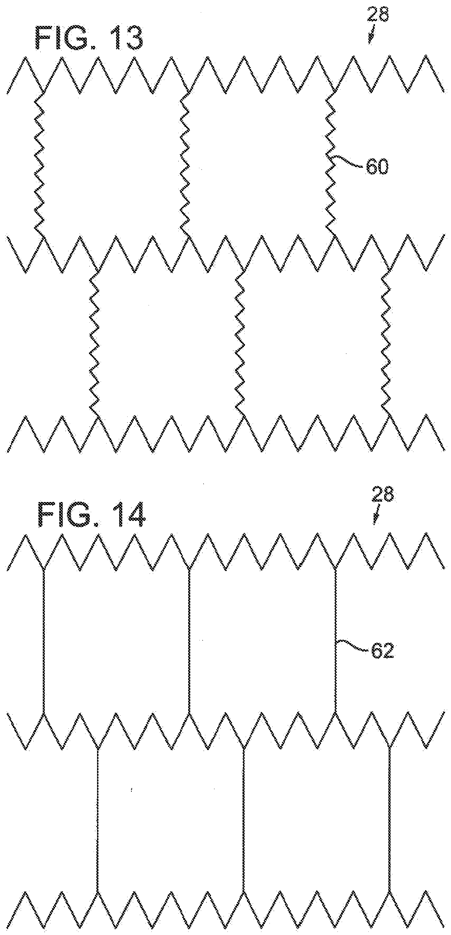

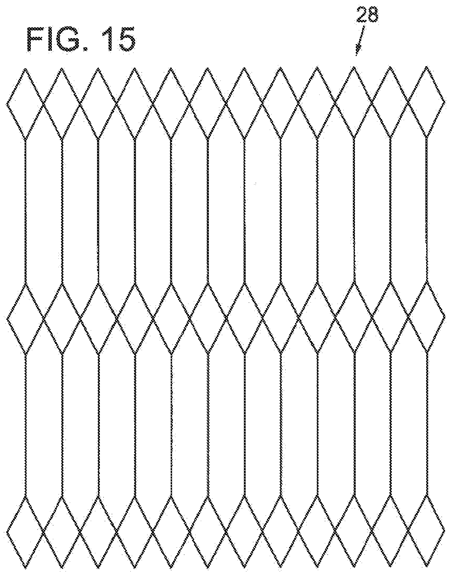

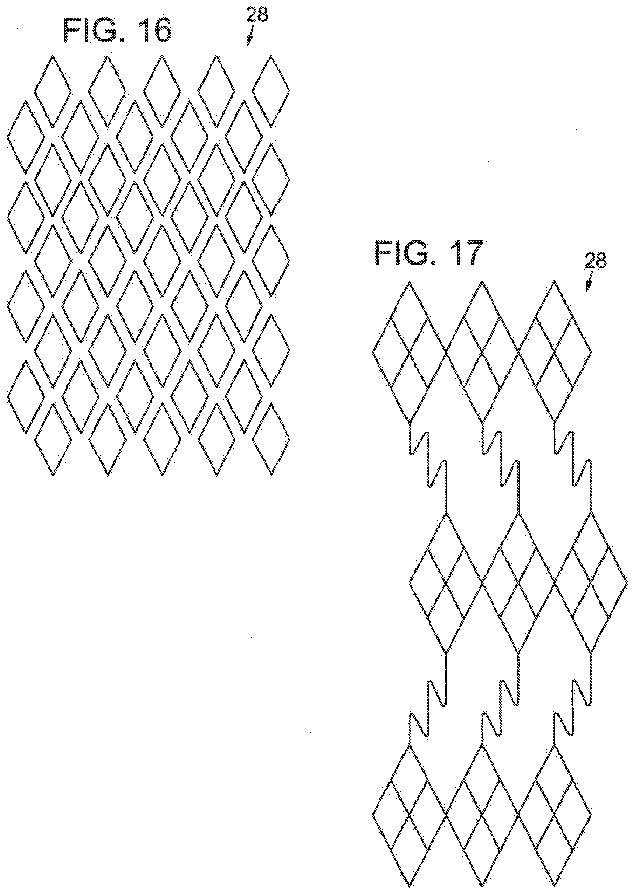

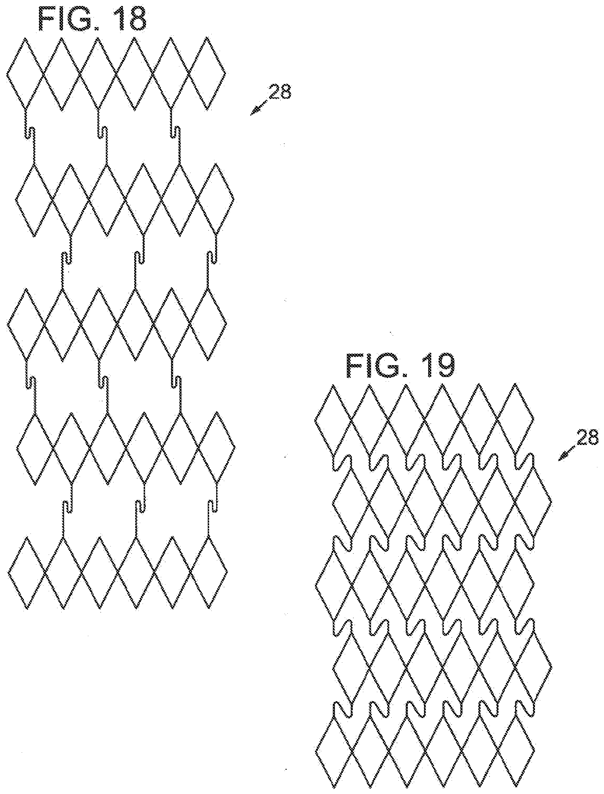

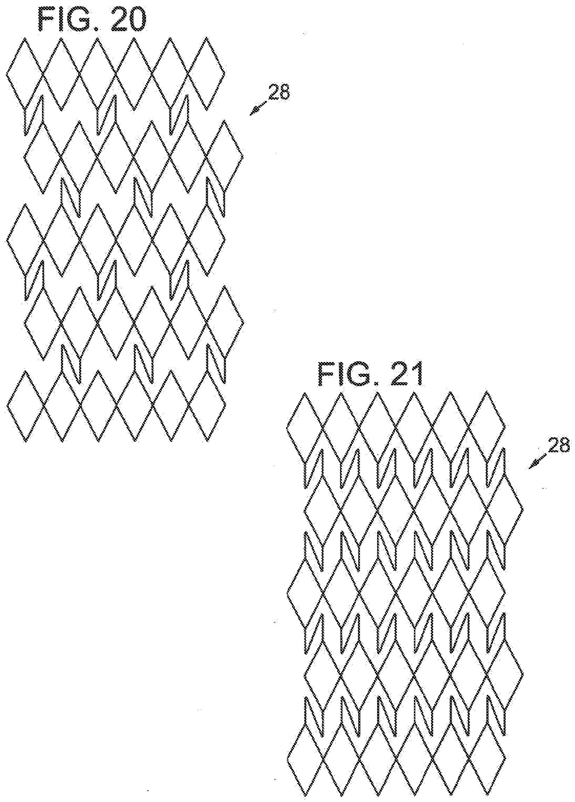

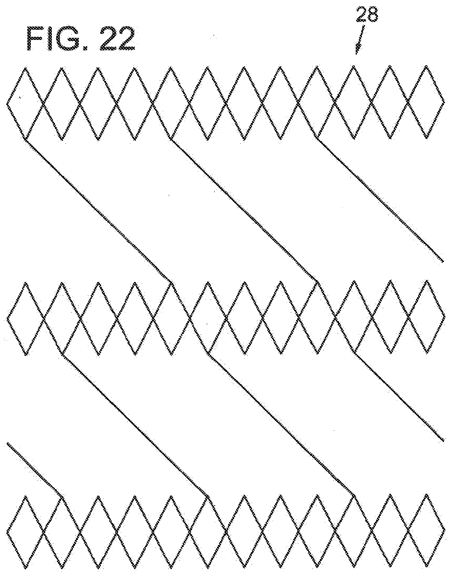

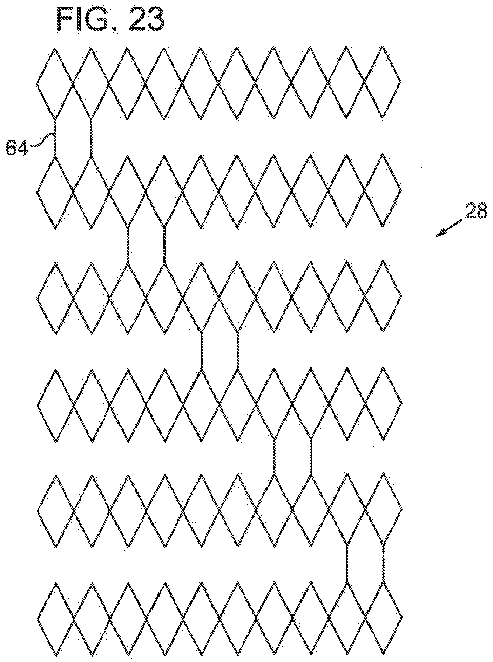

Additionally, the intermediate tubular layer **28** can be, for example, braided or laser cut to form a pattern or structure, such that the intermediate tubular layer 28 is amenable to radial expansion. **FIGS. 9-23** illustrate partial elevation views of various structures for the intermediate tubular layer. Some illustrated structures, such as those shown in **FIGS. 11-14** and **23,** include at least one discontinuity. For example, the struts **56, 58, 60, 62, 64,** shown in **FIGS. 11****,** **12****,** **13, 14****,** and **23****,** respectively, resulting in a discontinuous intermediate tubular layer **28** in that the struts **56, 58, 60, 62, 64** separate adjacent sections of the intermediate tubular layer **28** from each other, where the sections are spaced apart from each other along a longitudinal axis parallel to the lumen of the sheath. Thus, the structure of the intermediate tubular layer **28** can vary from section to section, changing along the length of the sheath.

The structures shown in **FIGS. 9-23** are not necessarily drawn to scale. Components and elements of the structures can be used alone or in combination within a single intermediate tubular layer **28.** The scope of the intermediate tubular layer **28** is not meant to be limited to these particular structures; they are merely exemplary aspects.

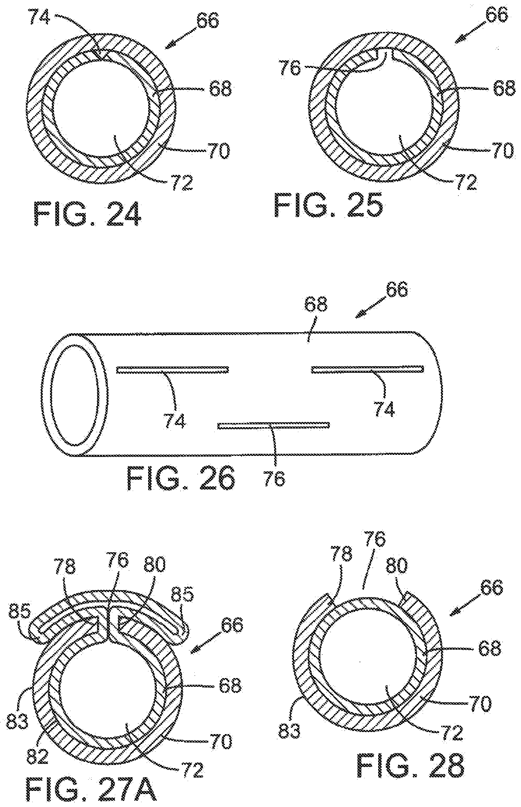

Alternative aspects of a sheath for introducing a prosthetic device are also described. For example, **FIGS. 24-26** illustrate a section view and a perspective view, respectively, of a sheath **66** for introducing a prosthetic device into a body. The sheath **66** comprises an inner liner, such as inner polymeric layer **68,** an outer layer, such as polymeric tubular layer **70,** and a hemostasis valve (not shown). The inner polymeric layer **68** and the outer polymeric tubular layer **70** at least partially enclose a lumen **72,** through which a delivery apparatus and prosthetic device can pass from outside the patient's body into the patient's vessel. Either or both of the inner polymeric layer **68** and the outer polymeric layer **70** can be provided with at least one longitudinal notch and/or cut to facilitate radial expansion of the sheath.

For example, **FIG. 24** illustrates a longitudinal notch **74** in the inner polymeric layer **68** that can facilitate radial expansion of the sheath **66.** The longitudinal notch **74** can separate or split open completely upon application of a radial force due to insertion of a delivery apparatus or prosthetic device. Similarly, **FIG. 25** illustrates a longitudinal cut **76** in the inner polymeric layer **68** that can also facilitate radial expansion of the sheath **66.** The outer polymeric layer **70** can, additionally or alternatively, comprise one or more longitudinal cuts **76** or notches **74.** Such cuts and/or notches, whether in the inner polymeric layer **68** or the outer polymeric layer **70,** can extend substantially through the entire thickness of the layer or can extend only partially through the thickness of the layer. The cuts and/or notches can be positioned at or near the inner or outer surface, or both surfaces, of the inner and/or outer polymeric layers **68, 70.**

**FIG. 26** illustrates a perspective view of one aspect of an inner polymeric layer **68** with longitudinal notches **74** and a longitudinal cut **76.** More or fewer notches **74** and/or cuts **76** can be provided. For clarity, the outer polymeric layer **70** is not shown in **FIG. 26****.** As shown in **FIG. 26**, longitudinal notches **74** and/or cuts **76** can extend only along a portion of the length of sheath **66.** In alternative aspects, one or more notches **74** and/or cuts **76** can extend substantially along the entire length of the sheath **66.** Additionally, notches **74** and/or cuts **76** can be positioned randomly or patterned.

One particular aspect of a sheath **66** comprises a sheath having a notch or cut in the outer polymeric layer **70** or the inner polymeric layer 68 that extends longitudinally along approximately 75% of the length of the sheath 66. If such a notch or cut extends only partially through the associated layer, it can have a relatively low tear force, such as a tear force of about 0.5 lbs., so that the notch splits open relatively easily during use.

The inner polymeric layer **68** and the outer polymeric layer **70** can optionally be adhered together or otherwise physically associated with one another. The amount of adhesion between the inner polymeric layer **68** and the outer polymeric layer **70** can be variable over the surfaces of the layers. For example, little to no adhesion can be present at areas around or near any notches and/or cuts present in the layers so as not to hinder the radial expansion of the sheath **66.** Adhesion between the layers can be created by, for example, thermal bonding and/or coatings. In some aspects, a sheath **66** can be formed from an extruded tube, which can serve as the inner polymeric layer **68.** The inner polymeric layer **68** can be surface-treated, such as by plasma etching, chemical etching, or other suitable methods of surface treatment. By treating the surface of the inner polymeric layer **68,** the outer surface of the inner polymeric layer **68** can have areas with altered surface angles that can provide better adhesion between the inner polymeric layer **68** and the outer polymeric layer **70.** The treated inner polymeric layer can be dip coated in, for example, a polyurethane solution to form the outer polymeric layer **70.** In some configurations, the polyurethane may not adhere well to untreated surface areas of the inner polymeric layer **68.** Thus, by surface treating only surface areas of the inner polymeric layer **68** that are spaced away from the areas of expansion (*e.g.*, the portion of the inner polymeric layer **68** near notches **74** and/or cuts **76),** the outer polymeric layer **70** can be adhered to some areas of the inner polymeric layer **68,** while other areas of the inner polymeric layer **68** remain free to slide relative to the outer polymeric layer **70,** thus allowing for expansion of the diameter of the sheath **66.** Thus, areas around or near any notches **74** and/or cuts **76** can experience little to no adhesion between the layers, while other areas of the inner and outer polymeric layers **68, 70** can be adhesively secured or otherwise physically associated with each other.

As with previously disclosed aspects, the aspects illustrated in **FIGS. 24-26** can be applied to sheaths having a wide variety of inner and outer diameters. Applications can utilize a sheath of the present disclosure with an inner diameter of the inner polymeric layer **68** that is expandable to an expanded diameter of from about 3 Fr to about 26 Fr. The expanded diameter can vary slightly along the length of the sheath **66.** For example, the expanded outer diameter at the proximal end of the sheath **66** can range from about 3 Fr to about 28 Fr, while the expanded outer diameter at the distal end of the sheath **66** can range from about 3 Fr to about 25 Fr. In some aspects, a sheath **66** can expand to an expanded outer diameter that is from about 10% greater than the original unexpanded outer diameter to about 100% greater than the original unexpanded outer diameter.

In some aspects, the outer diameter of the sheath **66** gradually decreases from the proximal end of the sheath **66** to the distal end of the sheath **66.** For example, in one aspect, the outer diameter can gradually decrease from about 26 Fr at the proximal end to about 18 Fr at the distal end. The diameter of the sheath **66** can transition gradually across substantially the entire length of the sheath **66.** In other aspects, the transition or reduction of the diameter of the sheath **66** can occur only along a portion of the length of the sheath **66.** For example, the transition can occur along a length from the proximal end to the distal end, where the length can range from about 0.5 inches to about the entire length of sheath **66.**

Suitable materials for the inner polymeric layer **68** can have a high elastic strength and include materials discussed in connection with other aspects, especially Teflon (PTFE), polyethylene (*e.g*., high density polyethylene), fluoropolymers, or combinations thereof. In some aspects, the inner polymeric layer **68** preferably has a low coefficient of friction, such as a coefficient of friction from about 0.01 to about 0.5. Some exemplary aspects of a sheath **66** comprise an inner polymeric layer **68** having a coefficient of friction of about 0.1 or less.

Likewise, suitable materials for the outer polymeric layer **70** include materials discussed in connection with other aspects and other thermoplastic elastomers and/or highly elastic materials.

**The** Shore hardness of the outer polymeric layer **70** can be varied for different applications and aspects. Some aspects comprise an outer polymeric layer with a Shore hardness of from about 25A to about 80A, or from about 20D to about 40D. One particular aspect comprises a readily available polyurethane with a Shore hardness of 72A. Another particular aspect comprises a polyethylene inner polymeric layer dipped in polyurethane or silicone to create the outer polymeric layer.

**The** sheath **66** can also include a radiopaque filler or marker, as described above. In some aspects, a distinct radiopaque marker or band can be applied to some portion of the sheath **66.** For example, a radiopaque marker can be coupled to the inner polymeric layer **68,** the outer polymeric layer **70,** and/or can be positioned in between the inner and outer polymeric layers **68, 70.**

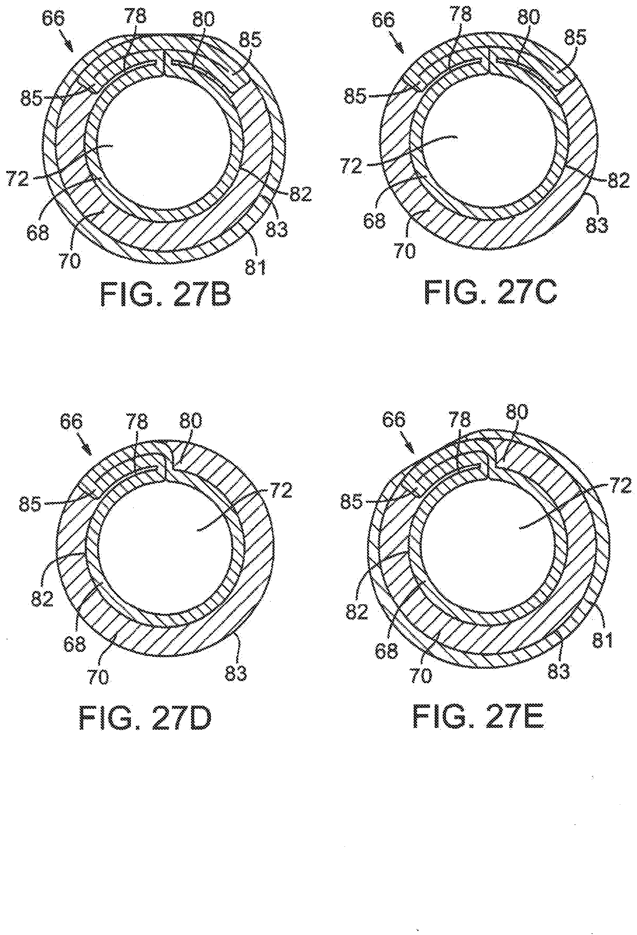

**FIGS. 27A-27E** and **28** illustrate section views of various aspects of unexpanded **(****FIGS. 27A-27E****)** and expanded **(****FIG. 28****)** sheaths **66** according to the present disclosure. The sheath **66** includes a split outer polymeric tubular layer **70** having a longitudinal cut **76** through the thickness of the outer polymeric tubular layer **70** such that the outer polymeric tubular layer **70** comprises a first portion **78** and a second portion **80** separable from one another along the cut **76.** An expandable inner polymeric layer **68** is associated with an inner surface **82** of the outer polymeric tubular layer **70,** and, in the unexpanded configuration shown in **FIG. 27A****,** a portion of the inner polymeric layer **68** extends through a gap created by the cut **76** and can be compressed between the first and second portions **78, 80** of the outer polymeric tubular layer **70.** Upon expansion of the sheath **66,** as shown in **FIG. 28****,** first and second portions **78, 80** of the outer polymeric tubular layer **70** have separated from one another, and the inner polymeric layer **68** is expanded to a substantially cylindrical tube. In some aspects, two or more longitudinal cuts **76** may be provided through the thickness of the outer polymeric tubular layer **70.** In such aspects, a portion of the inner polymeric layer **68** may extend through each of the longitudinal cuts **76** provided in the outer polymeric tubular layer **70.**

Preferably, the inner polymeric layer **68** comprises one or more materials that are elastic and amenable to folding and/or pleating. For example, **FIG. 27A** illustrates an inner polymeric layer **68** with folded regions **85.** As seen in **FIGS. 27A-****27E,** the sheath **66** can be provided with one or more folded regions **85.** Such folded regions **85** can be provided along a radial direction and substantially conform to the circumference of the outer polymeric tubular layer **70.** At least a portion of the folded regions **85** can be positioned adjacent the outer surface **83** of the outer polymeric tubular layer **70.**

Additionally, as shown in **FIGS. 27B** and **27E****,** at least a portion of the folded region or regions **85** can be overlapped by an outer covering, such as outer polymeric covering **81.** The outer polymeric covering **81** can be adjacent at least a portion of the outer surface **83** of the outer polymeric tubular layer **70.** The outer polymeric covering **81** serves to at least partially contain the folded regions **85** of the inner polymeric layer **68** and can also prevent the folded regions **85** from separating from the outer polymeric tubular layer **70** when, for example, the sheath **66** undergoes bending. In some aspects, the outer polymeric covering **81** can be at least partially adhered to the outer surface **83** of the outer polymeric tubular layer **70.** The outer polymeric covering **81** can also increase the stiffness and/or durability of the sheath **66.**

Additionally, as shown in **FIGS. 27B** and **27E****,** the outer polymeric covering **81** may not entirely overlap the circumference of the sheath **66.** For example, the outer polymeric covering **81** may be provided with first and second ends, where the ends do not contact one another. In these aspects, only a portion of the folded region **85** of the inner polymeric layer 68 is overlapped by the outer polymeric covering **81.**

In aspects wherein a plurality of folded regions **85** are present, the regions can be equally displaced from each other around the circumference of the outer polymeric tubular layer **70.** Alternatively, the folded regions can be off-center, different sizes, and/or randomly spaced apart from each other. While portions of the inner polymeric layer **68** and the outer tubular layer **70** can be adhered or otherwise coupled to one another, the folded regions **85** preferably are not adhered or coupled to the outer tubular layer **70.** For example, adhesion between the inner polymeric layer **68** and the outer tubular layer **70** can be highest in areas of minimal expansion.

One particular aspect of the sheath illustrated in **FIGS. 27A-28** comprises a polyethylene (*e.g*., high density polyethylene) outer polymeric tubular layer **70** and a PTFE inner polymeric layer **68.** However, other materials are suitable for each layer, as described above. Generally, suitable materials for use with the outer polymeric tubular layer **70** include materials having a high stiffness or modulus of strength that can support expansion and contraction of the inner polymeric layer **68.**

In some aspects, the outer polymeric tubular layer **70** comprises the same material or combination of materials along the entire length of the outer polymeric tubular layer **70.** In alternative aspects, the material composition can change along the length of the outer polymeric tubular layer **70.** For example, the outer polymeric tubular layer can be provided with one or more segments, where the composition changes from segment to segment. In one particular aspect, the Durometer rating of the composition changes along the length of the outer polymeric tubular layer **70** such that segments near the proximal end comprise a stiffer material or combination of materials, while segments near the distal end comprise a softer material or combination of materials. This can allow for a sheath **66** having a relatively stiff proximal end at the point of introducing a delivery apparatus while still having a relatively soft distal tip at the point of entry into the patient's vessel.

As with other disclosed aspects, the aspects of sheath **66** shown in **FIGS. 27A-28** can be provided in a wide range of sizes and dimensions. For example, the sheath **66** can be provided with an unexpanded inner diameter of from about 3 Fr to about 26 Fr. In some aspects, the sheath **66** has an unexpanded inner diameter of from about 15 Fr to about 16 Fr. In some aspects, the unexpanded inner diameter of the sheath **66** can range from about 3 Fr to about 26 Fr at or near the distal end of sheath **66,** while the unexpanded inner diameter of the sheath **66** can range from about 3 Fr to about 28 Fr at or near the proximal end of sheath **66.** For example, in one aspect, the unexpended sheath **66** can transition from an unexpanded inner diameter of about 16 Fr at or near the distal end of the sheath **66** to an unexpanded inner diameter of about 26 Fr at or near the proximal end of the sheath **66.**

The sheath **66** can be provided with an unexpanded outer diameter of from about 3 Fr to about 30 Fr, and, in some aspects, has an unexpanded outer diameter of from about 18 Fr to about 19 Fr. In some aspects, the unexpanded outer diameter of the sheath **66** can range from about 3 Fr to about 28 Fr at or near the distal end of sheath **66,** while the unexpanded outer diameter of the sheath **66** can range from about 3 Fr to about 30 Fr at or near the proximal end of sheath **66.** For example, in one unexpanded aspect, the sheath **66** can transition from an unexpanded outer diameter of about 18 Fr at or near the distal end of the sheath **66** to an unexpanded outer diameter of about 28 Fr at or near the proximal end of the sheath **66.**

The thickness of the inner polymeric layer **68** can vary, but in some preferred aspects is from about 0.002 inches to about 0.015 inches. In some aspects, expansion of the sheath **66** can result in expansion of the unexpanded outer diameter of from about 10% or less to about 430% or more.

As with other illustrated and described aspects, the aspects shown in **FIGS. 27A-28** can be provided with a radiopaque filler and/or a radiopaque tip marker, as described above. The sheath **66** can be provided with a radiopaque tip marker provided at or near the distal tip of the sheath **66.** Such a radiopaque tip marker can comprise materials such as those suitable for the radiopaque filler, platinum, iridium, platinum/iridium alloys, stainless steel, other biocompatible metals, or combinations thereof.

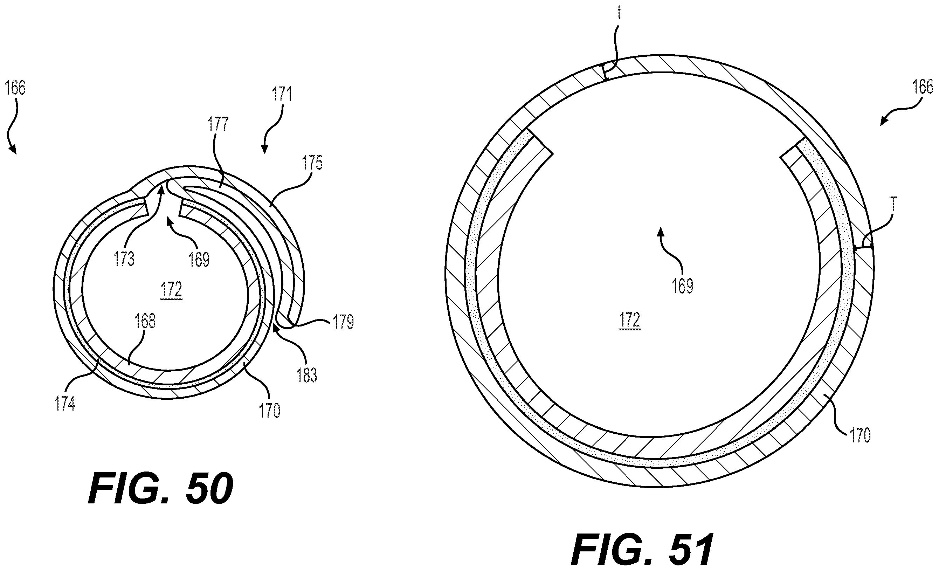

**FIGS. 50** and **51** show cross-sectional views of an expandable sheath **166** having an inner tubular layer **168** with a longitudinal slit **169.** In some aspects, the longitudinal slit **169** extends the entire length of the inner tubular layer **168.** An outer tubular layer **170** envelops the inner tubular layer 168 and includes a longitudinally extending, folded flap **171.** In some aspects, the folded flap **171** extends the entire length of the outer tubular layer **170.** The folded flap **171** overlies a portion of the outer surface **183** of the outer tubular layer **170** when the sheath is in an unexpanded state **(****FIG. 50****).** When a prosthetic device is moved through an inner lumen **172** of the sheath **166,** it applies an outwardly directed radial force on the inner tubular layer **168** that widens the longitudinal slit **169** and unfolds the folded flap **171.** **FIG. 51** shows the sheath **166** in the unexpanded state, with the longitudinal slit **169** widened and the outer tubular layer **170** unfolded.

The folded flap **171** of the outer tubular layer **170** has a base **173.** The base **173** can be positioned radially outwardly from the longitudinal slit **169.** In some aspects, the base **173** is centered over the longitudinal slit **169.** The folded flap **171** further includes a longitudinally extending overlying portion **175** extending from the base **173** to a longitudinally extending crease **179** at the edge of the flap **171.** The longitudinally extending overlying portion **175** overlies a longitudinally extending underlying portion **177** and is separated from the underlying portion **177** by the crease **179.** Underlying portion **177** contacts the outer surface **183** of the outer tubular layer **170** when the sheath is in an unexpanded state, as shown in **FIG. 50****.**

Some aspects of sheath **166** can include multiple longitudinally extending folded flaps that overlie portions of the outer surface **183** of the outer tubular layer **170,** positioned at various locations around the circumference of sheath **166.** For example, 2, 3, 4, 5, 6, 7, 8, 9, or 10 longitudinally extending folded flaps can be positioned around the circumference. In some aspects, these multiple folded flaps are equally spaced around the circumference of the sheath **166.**

The folded flap **171** extends circumferentially around a portion of the sheath **166.** In some aspects, the longitudinally extending flap **171** extends around about 20% to about 40% of the outer circumference of the outer tubular layer **170** when the sheath **166** is unexpanded (including about 20%, about 21%, about 22%, about 23%, about 24%, about 25%, about 26%, about 2272%, about 28%, about 29%, about 30%, about 31%, about 32%, about 33%, about 34%, about 35%, about 36%, about 37%, about 38%, about 39%, and about 40% of the outer circumference of the outer tubular layer **170).** In one example, for a sheath having a 14F (4.7 millimeters) unexpanded outer diameter, the folded flap extends around 85 to 120 degrees, or about 23%-35%, of the outer circumference (resulting in an inner lumen having an expanded diameter of about 7.6 millimeters to about 8.4 millimeters, which can be used with a valve having a 6.4 millimeter crimped outer diameter and a 26 millimeter expanded outer diameter).

In **FIG. 51****,** the wall thickness of flap **171** is labeled **t,** and the wall thickness of other parts of the outer tubular layer **170** are labeled ***T*.** In some aspects, a portion of the flap **171** (such as, for example, the underlying portion **177** or the overlying portion 175) can have a wall thickness ***t*** that is thinner than a wall thickness **(*T*)** of other portions of the outer tubular layer. This variation in wall thickness promotes even column strength around the circumference of the sheath **166,** which reduces kinking and minimizing the total outer diameter of the sheath. The wall thickness variation can also facilitate the folding process. In some aspects, the entire flap **171** has a wall thickness ***t*** that is thinner than a wall thickness ***T*** of the remainder of the outer tubular layer. In one example, the wall thickness of ***t*** can be from about 0.003 inches to about 0.007 inches, while the wall thickness of ***T*** can be from about 0.008 inches to about 0.012 inches. In other aspects, such as the one shown in **FIG. 51****,** the wall thickness ***t*** of flap **171** is about equal to the wall thickness ***T*** of the remainder of the outer tubular layer **170.**

The outer tubular layer **170** is formed of a material having a low coefficient of friction, a high tensile modulus, and a high ultimate tensile strength in order to improve the push force transmission through the length of sheath 166 while reducing kinking. Good push force transmission means that the force applied by the practitioner to advance the sheath is predictable, responsive, and consistent along the length of the sheath. However, an excessively high tensile modulus may limit the ability of the longitudinally extending flap **171** to open, which could hamper push force transmission. A desirable range for the tensile modulus of the outer tubular layer 170 is from about 300 MPa to about 2,000 MPa (including about 300 MPa, about 400 MPa, about 500 MPa, about 600 MPa, about 700 MPa, about 800 MPa, about 900 MPa, about 1000 MPa, about 1100 MPa, about 1200 MPa, about 1300 MPa, about 1400 MPa, about 1500 MPa, about 1600 MPa, about 1700 MPa, about 1800 MPa, about 1900 MPa, and about 2000 MPa). In some aspects, the tensile modulus may preferably be at least 700 MPa. High axial and radial stiffnesses enable the sheath to be easily inserted and to resist collapse within the body.

The ultimate tensile strength of the outer tubular layer **170** can be at least 50 MPa. A high ultimate tensile strength helps to prevent the material from tearing while the prosthetic device is advancing through the sheath.

Exemplary materials for formation of the outer tubular layer **170** of the aspects shown in **FIGS. 50** and **51** include high density polyethylene (HDPE), polyamide, copolyamide, polyether block amide (PEBAX^{®}), or a blend of polyamide. Materials having shape memory properties are advantageous because the outer tubular layer **170** can be given a bias toward the folded state (for example, by heat setting). This facilitates refolding of the outer tubular layer **170** after passage of a prosthetic device. PEBAX^{®} is an exemplary shape memory material that may be heat set toward the folded state.

In some aspects, the outer surface **183** of the outer tubular layer **170** can include a hydrophilic coating. In some aspects, a bond can be created between the underlying portion **177** of the folded flap **171** and the outer surface **183** of the outer tubular layer **170.** The bond can be a thermal bond (with portions of the contacting layers melted together), or it can be a separate layer of adhesive.

**As** discussed above, the inner tubular layer **168** of the aspects shown in **FIGS. 50** and **51** include a longitudinal slit **169.** The inner tubular layer **168** includes a first longitudinally extending end **178** and a second longitudinally extending end **180,** the first and second longitudinally extending ends **178, 180** defining the longitudinal slit **171.**

The inner tubular layer **168** forms a low friction barrier between a passing prosthetic device and the higher friction outer tubular layer **170.** The inner tubular layer 168 extends around at least 80% (or at least 85%, or at least 90%, or at least 95%) of the circumference of the inner lumen **172** when the sheath **166** is in an unexpanded state. This high degree of coverage limits contact between the passing prosthetic device and the higher friction outer tubular layer **170.** In some aspects, the coefficient of friction per ASTM D1894 of the inner tubular layer **168** (static or dynamic) is 0.30 or less. In other aspects, the coefficient of friction is 0.25 or less.

In some aspects, the low-friction inner tubular layer **168** includes, or is formed of, a material having a tensile modulus of at least about 300 MPa (and up to about 1400 MPa) to provide good push force transmission while providing kink resistance. This material could be, for example, high density polyethylene or a fluoropolymer. Exemplary fluoropolymers include polytetrafluoroethylene, ethylene fluorinated ethylene propylene, or perfluoro alkoxy.

A tie layer **174** can be positioned between the two layers, thereby adhering the inner tubular layer **168** to the outer tubular layer **170.** In some aspects, the tie layer **174** can be formed of polyurethane or functionalized polyolefin. In some aspects, the contacting surface of the inner tubular layer **168** can be etched to improve bonding to the tie layer. For example, an inner tubular layer **168** that includes, or is formed of, a fluoropolymer might be etched on its outer surface to improve thermal bonding to the tie layer **174.**

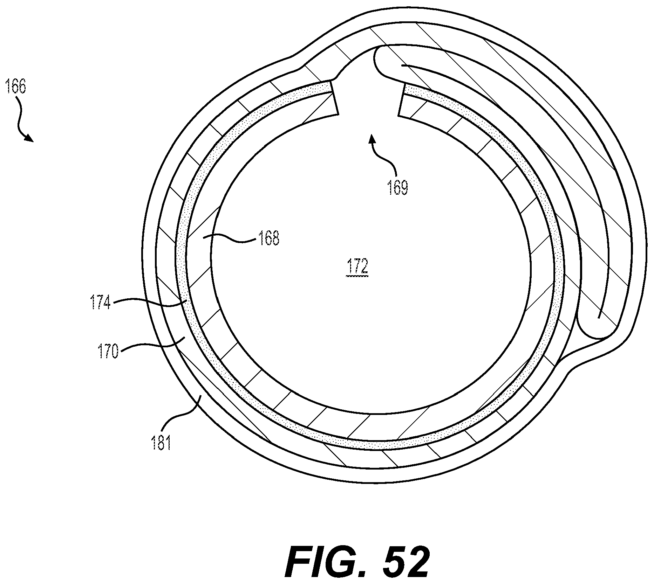

Some aspects of the sheath **166** shown in **FIGS. 50** and **51** can also include an outer jacket **181,** as shown in **FIG. 52****.** The outer jacket **181** is elastomeric (having a relatively low tensile modulus compared to the inner and outer tubular layers **168, 170).** As mentioned above, in some aspects, the outer tubular layer **170** can be included or can be formed of a shape memory material (for example, a heat-set polymer such as PEBAX^{®}) that facilitates refolding of the outer tubular layer **170** after expansion. In some aspects, the sheath **166** can include an elastomeric outer jacket **181** that extends over and envelops the outer tubular layer **170.** The outer jacket **181** and the shape memory feature can be used alone or in conjunction with each other to facilitate refolding (i.e., the elastomeric outer jacket **181** can extend over an outer tubular layer **170** that comprises a shape memory material). The refolding of the outer tubular layer **170** can preferably return the sheath **166** back to its original outer diameter or to a value close to the original outer diameter (for example, to within about 10%, about 20%, about 30%, about 40%, or about 50% of the original outer diameter). The elastomeric outer jacket **181** can include or can be formed completely of materials such as low durometer polyurethane (for example, less than Shore 85A), styrene elastomer (for example, less than Shore 85A), latex, or low durometer PEBAX^{®} (for example, less than Shore 35D).

Methods of using the sheath of **FIGS. 50-52** include first inserting an expandable sheath **166** into the vasculature of a subject and advancing the prosthetic device through an inner lumen **172** of the expandable sheath **166.** The prosthetic device applies an outwardly directed radial force to the inner tubular layer **168** of the expandable sheath **166.** In some aspects, the outwardly directed radial force is transmitted through the inner tubular layer **168,** the tie layer **174,** and the outer tubular layer **170.** The outwardly directed radial force widens a longitudinal slit **169** in the inner tubular layer **168.** In certain aspects, the widening of the longitudinal slit **169** travels the full length of the expandable sheath.

The outwardly directed radial force further unfolds the longitudinally extending flap **171** of the outer tubular layer **170** to expand the expandable sheath. The unfolding of the flap **171** can include sliding a longitudinally extending overlying portion **175** circumferentially against a longitudinally extending underlying portion **177** of flap **171.** The underlying portion **177** can slide circumferentially against an outer surface **183** of the outer tubular layer **170.** In some aspects, the unfolding of the longitudinally extending flap **171** occurs at a position radially outward from the longitudinal slit 169 of the inner tubular layer **168.** In still further aspects, the unfolding of the flap **171** can extend the full length of the expandable sheath **166.**

The longitudinal slit **169** of the inner tubular layer **168** narrows once the outwardly directed radial force has ceased (i.e., once the prosthetic device has passed by). The slit **169** may narrow back to its original width or to a value close to the original width (for example, to a value within about 10% of the original width). The narrowing can occur along the entire length of the sheath **166.** The prosthetic device is then delivered to the procedure site.

The longitudinally extending flap **171** at least partially refolds once the prosthetic device ceases to apply the outwardly directed radial force (i.e., once it has passed by). In some aspects, the longitudinally extending flap **171** refolds itself due to a shape memory bias toward the folded state. In some aspects, an inwardly directed radial force is applied to the outer surface **183** of the outer tubular layer **170** to refold the longitudinally extending flap **171** (for example, by elastomeric outer jacket **181).**

An example method of making the sheath is as follows. These steps are not meant to be limiting. The steps given can be reordered as needed. Other steps can be added, or in other examples, some steps may not be necessary. Sizes are approximate. 1) Start with a PTFE inner liner of about 0.200 inch inner diameter (ID), wall thickness about 0.004 inches, 2) load PTFE inner liner on to a tapering mandrel from about 0.200 inches to about 0.187 inches, 3) stretch on to an 0.187 inch outer diameter (OD) mandrel section under heat, 4) flare proximal end of 0.200 inch ID PTFE to 0.340 inches ID under heat, 5) by expanding with air pressure, load tie layer such as Tecoflex 80A having about 0.200 inch ID and 0.004 inch wall thickness over the PTFE inner liner along the body section (optionally the tie layer can be applied after expanding the inner layer with air pressure),, 6) adhere tie layer to inner liner, for example, by covering it with FEP (fluorinated ethylene propylene) heat shrink tubing and applying heat, 7) remove FEP thermal shrink tubing if it was used, 8) create a longitudinal slit along the body section of the assembly, 9) load the subassembly having a slit on to a 0.187 inch OD mandrel, 10) load outer layer over the body, 11) fold outer layer, 12) heat set the fold, for example, by inserting the subassembly with a fold inside a heat shrink tubing and placing the assembly into the oven, 13) remove the shrink tubing if it was used, 14) remove sheath from the mandrel. In some aspects, an outer elastomeric jacket is added over the heat set outer layer prior to removal from the mandrel.

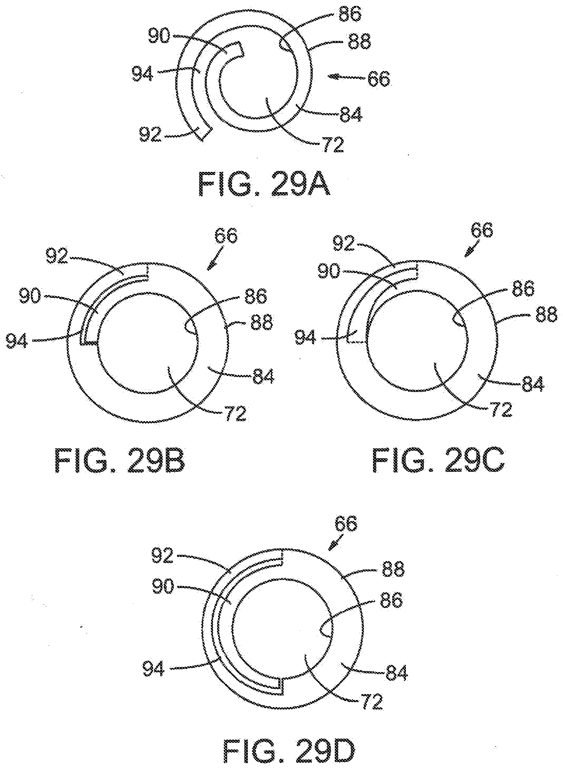

**FIGS. 29A-29D** show section views of other possible configurations of a sheath **66** for introducing a prosthetic device into a patient's vasculature. The sheath **66** comprises a polymeric tubular layer **84** having an inner surface **86** and an outer surface **88.** The thickness of the polymeric tubular layer **84** extends from the inner surface **86** to the outer surface **88.** As shown in **FIGS. 29B-29D****,** the polymeric tubular layer **84** can be formed with at least a first angular portion **90** of reduced thickness adjacent the inner surface **86** and a second angular portion **92** of reduced thickness adjacent the outer surface **88,** with the second portion **92** at least partially overlapping the first portion **90.** **FIG. 29A** illustrates a similar configuration, where a second portion **92** at least partially overlaps a first portion **90** in a partial coil configuration. In the aspect of **FIG. 29A****,** the second portion **92** and the first portion **90** can have the same thickness.

In preferred aspects, the first and second portions **90, 92** are not adhered to one another. In some aspects, and as best seen in **FIG. 29A****,** there can be a small gap **94** between the first and second portions **90, 92** that can give the sheath **66** the appearance of having two interior lumens **72, 94.** **FIGS. 29A-29D** illustrate the sheath **66** in unexpanded configurations. Preferably, upon expansion of the sheath **66,** the ends of the first and second portions **90, 92** abut or are in close proximity to each other to reduce or eliminate any gap between them.

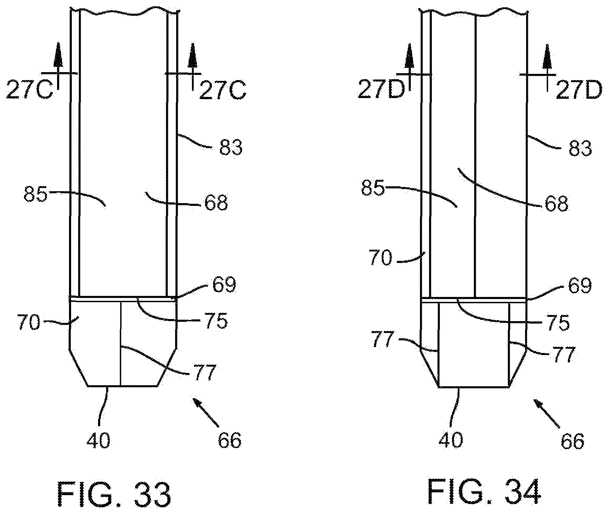

In some aspects, a sheath **66** can comprise a partial slit or score line along at least a portion of its length. For example, as shown in **FIG. 33****,** a sheath **66** can comprise an outer polymeric tubular layer **70** over an inner polymeric layer **68.** The inner polymeric layer can extend through a cut in the outer polymeric tubular layer **70** to form a folded region **85** on the outer surface of the outer polymeric tubular layer **70,** such as also shown in **FIG. 27C****.** The folded region **85** of the inner liner, in some aspects, terminates before the outer polymeric tubular layer **70** (*i.e*., the outer polymeric tubular layer **70** is longer than the inner liner). As shown in **FIG. 33****,** in these aspects, the sheath **66** can comprise a partial slit or score line **77** that can extend from the termination (distal end) **75** of the folded region **85** to the distal end **40** of the sheath **66.** In some aspects, score line **77** can facilitate expansion of the sheath **66.**

Score line **77** can be substantially centrally located with respect to the folded region **85.** In alternative aspects, score line **77** can be positioned in other locations relative to the folded region **85.** Also, sheath **66** can comprise one or more score lines **77.** For example, as shown in **FIG. 34****,** one or more score lines **77** can be peripherally located with respect to the folded region **85.** The one or more score lines **77** can be positioned anywhere around the circumference of the outer polymeric tubular layer **70.** In aspects comprising a radiopaque marker **69** as seen in **FIG. 33**, a score line **77** can extend from, for example, the distal end of the radiopaque marker **69** substantially to the distal end **40** of the sheath **66.**

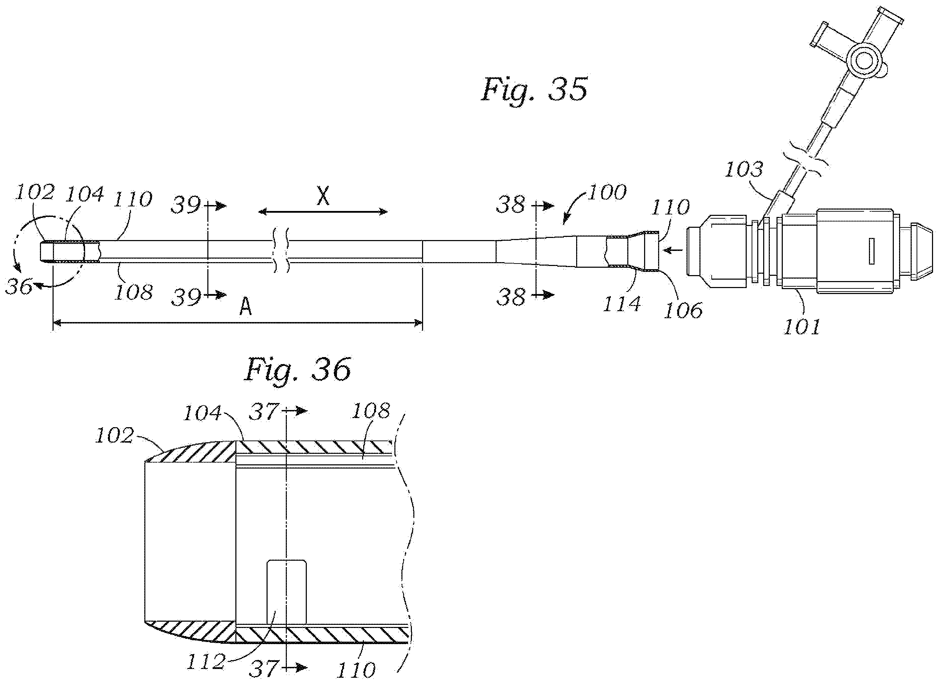

**FIGS. 35** and **36** illustrate an expandable sheath **100** according to the present disclosure, which can be used with a delivery apparatus for delivering a prosthetic device, such as a tissue heart valve, into a patient. In general, the delivery apparatus can include a steerable guide catheter (also referred to as a flex catheter) a balloon catheter extending through the guide catheter, and a nose catheter extending through the balloon catheter (*e.g*., as depicted in **FIG. 1**). The guide catheter, the balloon catheter, and the nose catheter can be adapted to slide longitudinally relative to each other to facilitate delivery and positioning of the valve at an implantation site in a patient's body. However, it should be noted that the sheath **100** can be used with any type of elongated delivery apparatus used for implanting balloon-expandable prosthetic valves, self-expanding prosthetic valves, and other prosthetic devices. Generally, sheath **100** can be inserted into a vessel (*e.g*., the femoral or iliac arteries) by passing through the skin of the patient, such that a soft tip portion **102** at the distal end **104** of the sheath **100** is inserted into the vessel. The sheath **100** can also include a proximal flared end portion **114** to facilitate mating with an introducer housing **101** and catheters mentioned above (*e.g*., the proximal flared end portion **114** can provide a compression fit over the housing tip and/or the proximal flared end portion **114** can be secured to the housing 101 via a nut or other fastening device or by bonding the proximal end of the sheath to the housing). The introducer housing **101** can house one or more valves that form a seal around the outer surface of the delivery apparatus once inserted through the housing, as known in the art. The delivery apparatus can be inserted into and through the sheath **100,** allowing the prosthetic device to be advanced through the patient's vasculature and implanted within the patient.

Sheath **100** can include a plurality of layers. For example, sheath **100** can include an inner liner **108** and an outer layer **110** disposed around the inner liner **108.** The inner liner **108** can define a lumen through which a delivery apparatus can travel into a patient's vessel in order to deliver, remove, repair, and/or replace a prosthetic device, moving in a direction along the longitudinal axis X. As the prosthetic device passes through the sheath **100,** the sheath locally expands from a first, resting diameter to a second, expanded diameter to accommodate the prosthetic device. After the prosthetic device passes through a particular location of the sheath **100,** each successive expanded portion or segment of the sheath **100** at least partially returns to the smaller, resting diameter. In this manner, the sheath **100** can be considered self-expanding in that it does not require the use of a balloon, dilator, and/or obturator to expand.

**The** inner and outer layers **108, 110** can comprise any suitable materials. Suitable materials for the inner liner **108** include polytetrafluoroethylene (PTFE), ethylene tetrafluoroethylene (ETFE), nylon, polyethylene, polyether block amide (*e.g*., PEBAX^{®}), and/or combinations thereof. In some exemplary aspects, the inner liner **108** can comprise a lubricious, low-friction, or hydrophilic material, such as PTFE. Such a low coefficient of friction materials can facilitate passage of the prosthetic device through the lumen defined by the inner liner **108.** In other aspects, the inner liner **108** can have a coefficient of friction of less than about 0.1. Some aspects of a sheath **100** can include a lubricious liner on the inner surface of the inner liner **108.** Examples of suitable lubricious liners include materials that can further reduce the coefficient of friction of the inner liner **108,** such as PTFE, polyethylene, polyvinylidene fluoride, and combinations thereof. Suitable materials for a lubricious liner also include other materials desirably having a coefficient of friction of about 0.1 or less.

Suitable materials for the outer layer **110** include nylon, polyethylene, PEBAX^{®}, HDPE, polyurethanes (*e.g*., Tecoflex^{™}), and other medical grade materials. In one aspect, the outer layer 110 can comprise high density polyethylene (HDPE) and Tecoflex^{™} (or other polyurethane material) extruded as a composite. In some aspects, the Tecoflex^{™} can act as an adhesive between the inner liner **108** and the outer layer **110** and may only be present along a portion of the inner surface of the outer layer **110.** Other suitable materials for the inner and outer layers are also disclosed in U.S. Patent Application Publication No. 2010/0094392.

Additionally, some aspects of a sheath **100** can include an exterior hydrophilic coating on the outer surface of the outer layer **110.** Such a hydrophilic coating can facilitate insertion of the sheath **100** into a patient's vessel. Examples of suitable hydrophilic coatings include the Harmony^{™} Advanced Lubricity Coatings and other Advanced Hydrophilic Coatings available from SurModics, Inc., Eden Prairie, MN. DSM medical coatings (available from Koninklijke DSM N.V, Heerlen, the Netherlands), as well as other hydrophilic coatings (*e.g*., PTFE, polyethylene, polyvinylidene fluoride), are also suitable for use with the sheath **100.**

Best seen in **FIG. 36**, the soft tip portion **102** can comprise, in some aspects, low-density polyethylene (LDPE) and can be configured to minimize trauma or damage to the patient's vessels as the sheath is navigated through the vasculature. For example, in some aspects, the soft tip portion **102** can be slightly tapered to facilitate passage through the vessels. The soft tip portion **102** can be secured to the distal end **104** of the sheath **100,** such as by thermally bonding the soft tip portion **102** to the inner and outer layers of the sheath **100.** Such a soft tip portion **102** can be provided with a lower hardness than the other portions of the sheath **100.** In some aspects, the soft tip **102** can have a Shore hardness from about 25 D to about 40 D. The tip portion **102** is configured to be radially expandable to allow a prosthetic device to pass through the distal opening of the sheath **100.** For example, the tip portion **102** can be formed with a weakened portion, such as an axially extending score line or perforated line that is configured to split and allow the tip portion to expand radially when the prosthetic device passes through the tip portion (such as shown in the aspects of **FIGS. 33** and **34**).

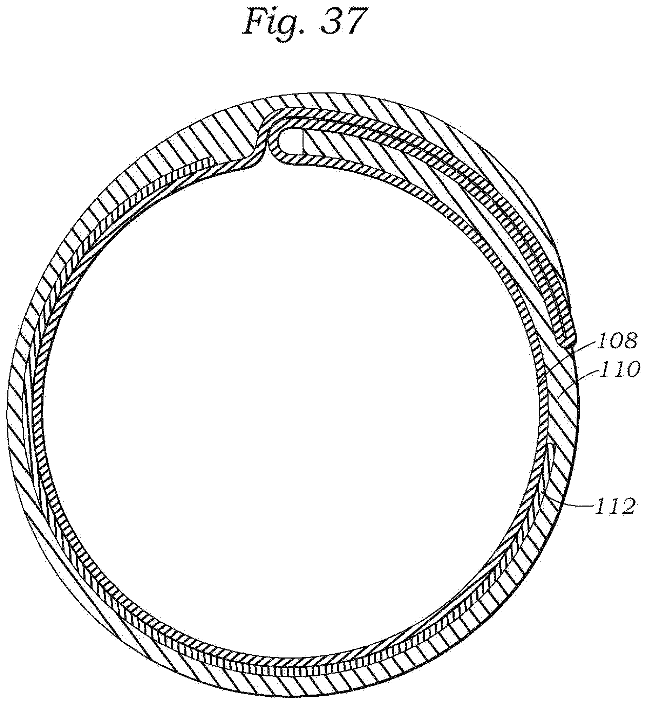

**FIG. 37** shows a cross-section view of the sheath **100** taken near the distal end **104** of the sheath **100.** As shown in **FIGS. 36** and **37****,** the sheath **100** can include at least one radiopaque filler or marker, such as a discontinuous, or C-shaped, band **112** positioned near the distal end **104** of the sheath **100.** The marker **112** can be associated with the inner and/or outer layers **108, 110** of the sheath **100.** For example, as shown in **FIG. 37**, the marker **112** can be positioned between the inner liner **108** and the outer layer **110.** In alternative aspects, the marker **112** can be associated with the outer surface of the outer layer **110.** In some aspects, the marker **112** can be embedded or blended within the inner or outer layers **108, 110.**

**The** C-shaped band **112** can serve as a radiopaque marker or filler to enable visibility of the sheath **100** under fluoroscopy during use within a patient. The C-shaped band **112** can comprise any suitable radiopaque material, such as barium sulfite, bismuth trioxide, titanium dioxide, bismuth subcarbonate, platinum, iridium, and combinations thereof. In one specific aspect, the C-shaped band can comprise about 90% platinum and about 10% iridium. In other aspects, the marker **112** needs not to be a C-shaped band. Other shapes, designs, and configurations are possible. For example, in some aspects, the marker **112** can extend around the entire circumference of the sheath **100.** In other aspects, the marker **112** can comprise a plurality of small markers spaced around the sheath **100.**

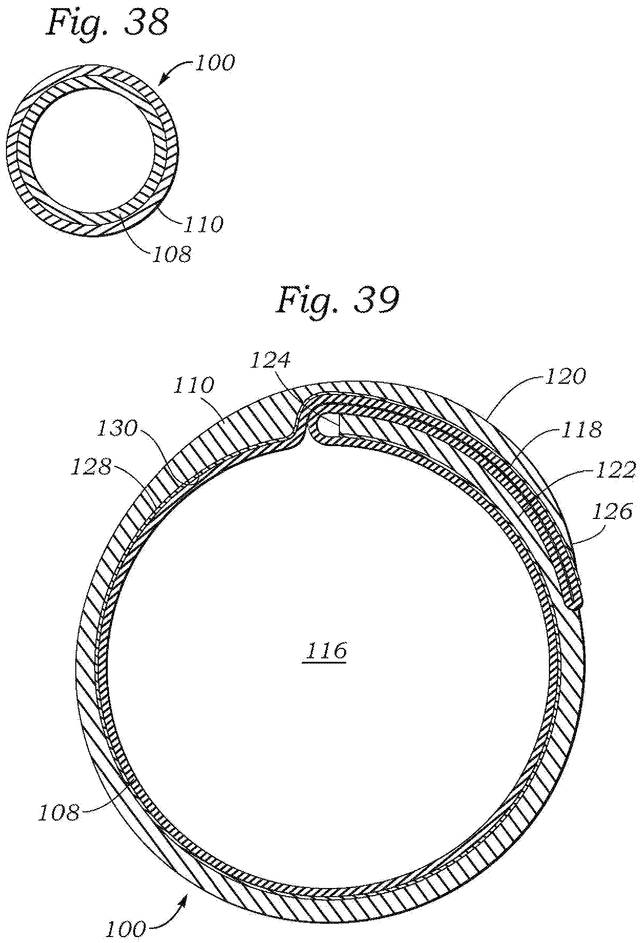

**FIGS. 38** and **39** show additional cross sections taken at different points along the sheath **100.** **FIG. 38** shows a cross-section of a segment of the sheath near the proximal end **106** of the sheath **100,** as indicated by line **38-38** in **FIG. 35****.** The sheath **100** at this location can include inner liner **108** and outer layer **110.** At this location, near the proximal end of the sheath, the layers **108, 110** can be substantially tubular, without any slits or folded portions in the layers. By contrast, the layers **108, 110** at different locations along the sheath **100** (*e.g*., at the point indicated by line **39-39** in **FIG. 35****)** can have a different configuration.

As shown in **FIG. 39**, the inner liner **108** can be arranged to form a substantially cylindrical lumen **116** therethrough. Inner liner **108** can include one or more folded portions **118.** In the aspect shown in **FIG. 39**, inner liner **108** is arranged to have one folded portion **118** that can be positioned on either side of the inner liner **108.** The folded portion **118** includes a first fold (e.g., a longitudinally extending fold line) and a second fold and an overlapping portion extending circumferentially therebetween (when the sheath is in an unexpanded configuration). As illustrated in **FIG. 39**, the folded portion **118** comprises an overlap in a radial direction of at least two thicknesses of the inner liner **108.** Inner liner **108** can be continuous in that there are no breaks, slits, or perforations in inner liner **108.** Outer layer **110** can be arranged in an overlapping fashion such that an overlapping portion **120** overlaps at least a part of the folded portion **118** of the inner liner **108.** As shown in **FIG**. **39**, the overlapping portion **120** also overlaps an underlying portion **122** of the outer layer **110.** The underlying portion **122** can be positioned to underlie both the overlapping portion **120** of the outer layer **110,** as well as the folded portion **118** of the inner liner **108.** Thus, the outer layer **110** can be discontinuous in that it includes a slit or a cut in order to form the overlapping and underlying portions **120, 122.** In other words, a first edge **124** of the outer layer **110** is spaced apart from a second edge **126** of the outer layer **110** so as not to form a continuous layer.

As shown in **FIG. 39****,** the sheath **100** can also include a thin layer of bonding or adhesive material **128,** also referred to as a tie layer, positioned between the inner and outer layers **108, 110.** In one aspect, the adhesive material **128** can comprise a polyurethane material such as Tecoflex^{™} or etched PTFE tubing. The adhesive material **128** can be positioned on an inner surface **130** of at least a portion of the outer layer **110** so as to provide adhesion between selected portions of the inner and outer layers **108, 110.** For example, the outer layer **110** may only include an adhesive layer **128** around the portion of the inner surface **130** that faces the lumen-forming portion of the inner liner **108.** In other words, the adhesive layer **128** can be positioned so that it does not contact the folded portion **118** of the inner liner **108** in some aspects. In other aspects, the adhesive layer **128** can be positioned in different configurations as desired for the particular application. For example, as shown in **FIG. 39**, the adhesive layer **128** can be positioned along the entire inner surface **130** of the outer layer **110.** In an alternative aspect, the adhesive layer can be applied to the outer surface of the inner liner **108** instead of the inner surface of the outer layer. The adhesive layer **128** can be applied to all or selected portions on the inner liner **108;** for example, the adhesive layer **128** can be formed only on the portion of the inner liner that faces the lumen-forming portion of the outer layer and not on the folded portion. The configuration of **FIG. 39** allows for radial expansion of the sheath 100 as an outwardly directed radial force is applied from within (*e.g*., by passing a medical device such as a prosthetic heart valve through the lumen **116).** As radial force is applied, the folded portion **118** can at least be partially separated, straighten, and/or unfold, and/or the overlapping portion **120** and the underlying portion **122** of the outer layer **110** can slide circumferentially with respect to one another, thereby allowing the diameter of lumen **116** to enlarge.

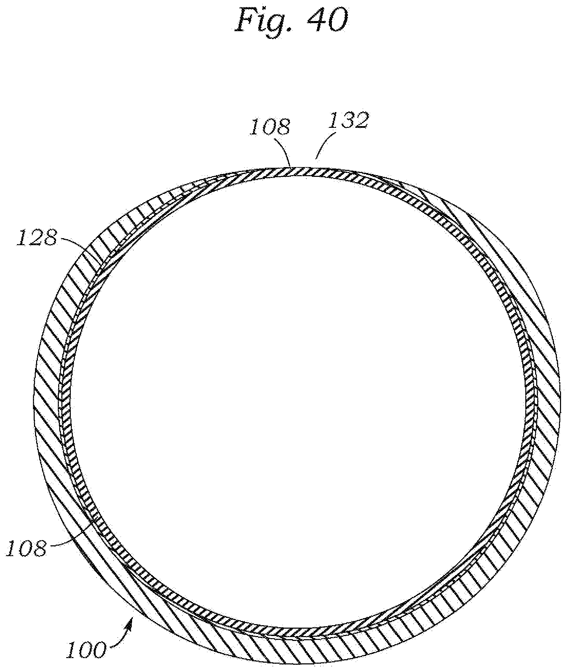

In this manner, the sheath **100** is configured to expand from a resting configuration **(****FIG. 39****)** to an expanded configuration shown in **FIG. 40****.** In the expanded configuration, as shown in **FIG. 40****,** an annular gap **132** can form between the longitudinal edges of the overlapping portion **120** and the underlying portion **122** of the outer layer **110.** As the sheath **100** expands at a particular location (i.e., locally expands at the location of the passing prosthetic device), the overlapping portion **120** of the outer layer **110** can move circumferentially with respect to the underlying portion **122** as the folded portion **118** of the inner liner **108** unfolds. This movement can be facilitated by the use of a low-friction material for inner liner **108,** such as PTFE. Further, the folded portion **118** can at least partially separate and/or unfold to accommodate a medical device having a diameter larger than that of lumen **116** in the resting configuration. As shown in **FIG. 40****,** in some aspects, the folded portion of the inner liner **108** can completely unfold so that the inner liner **108** forms a cylindrical tube at the location of the expanded configuration.

The sheath **100** can be configured such that it locally expands at a particular location corresponding to the location of the medical device along the length of the lumen **116,** and then locally contracts once the medical device has passed that particular location. Thus, a bulge may be visible, traveling longitudinally along the length of the sheath as a medical device is introduced through the sheath, representing continuous local expansion and contraction as the device travels the length of the sheath **100.** In some aspects, each segment of the sheath **100** can locally contract after removal of any radial outward force such that it regains the original resting diameter of lumen **116.** In some aspects, each segment of the sheath 100 can locally contract after removal of any radial outward force such that it at least partially returns to the original resting diameter of lumen **116.**

The layers **108, 110** of sheath **100** can be configured, as shown in **FIG. 39** along at least a portion of the length of the sheath **100.** In some aspects, the layers **108, 110** can be configured as shown in **FIG. 39** along the length A **(****FIG. 35****)** extending from a location adjacent the soft tip portion **102** to a location closer to the proximal end **106** of the sheath **100.** In this matter, the sheath is expandable and contractable only along a portion of the length of the sheath corresponding to length A (which typically corresponds to the section of the sheath inserted into the narrowest section of the patient's vasculature).

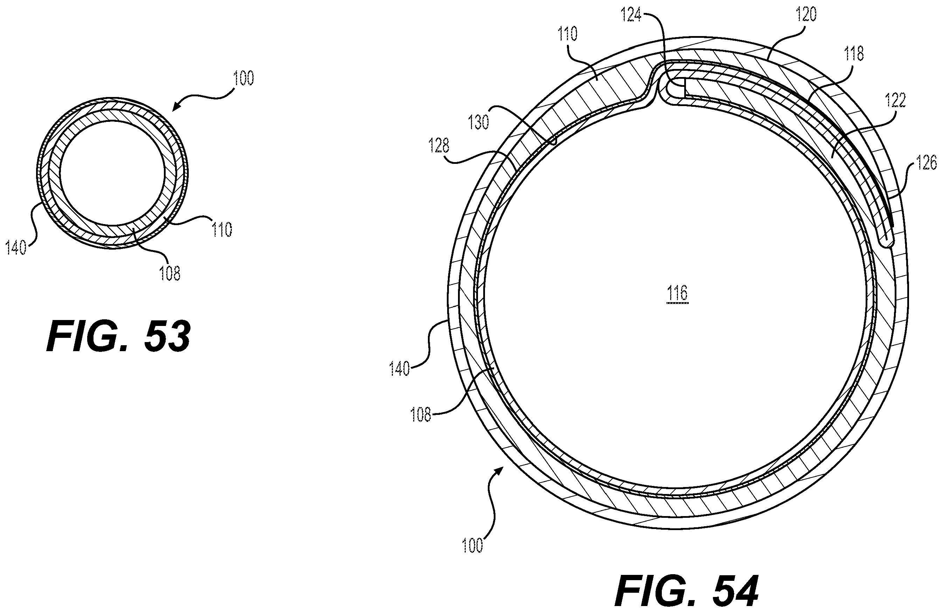

**The** sheath **100** of **FIG. 35** can include an outer jacket **140** extending over the outer layer **110.** **FIGS. 53** and **54** show additional cross sections taken at different points along the sheath **100.** Similar to **FIG. 38****,** **FIG. 53** shows a cross-section of a segment of the sheath near the proximal end **106** of the sheath **100,** as indicated by line **38-38** in **FIG. 35****.** The sheath **100** at this location can include inner liner **108,** outer layer **110,** adhesive layer **128,** and an outer jacket **140.** At this location, near the proximal end of the sheath, the layers **108, 110,** and outer jacket **140** can be substantially tubular, without any slits or folded portions in the layers. By contrast, the layers **108, 110** at different locations along the sheath **100** (*e.g*., at the point indicated by line **39-39** in **FIG. 35**) can have a different configuration, while the outer jacket **140** maintains a substantially tubular shape, without slits or folds.

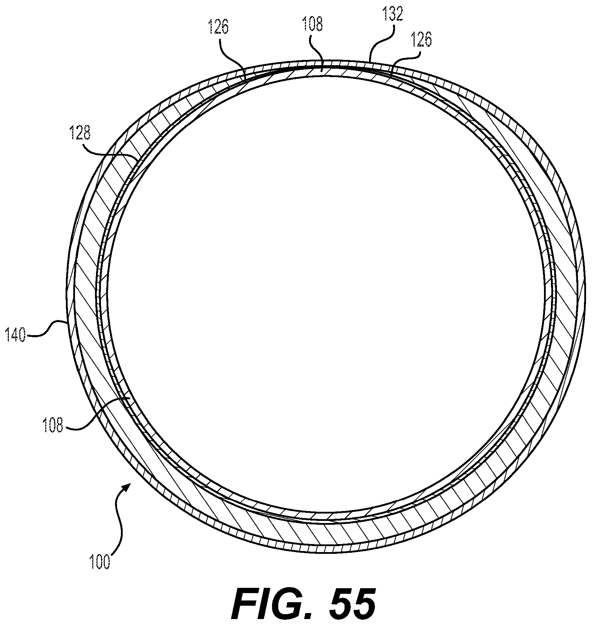

As shown in **FIG. 54**, and described above with respect to **FIG. 39**, the inner liner **108** can be arranged to form a substantially cylindrical lumen **116** extending therethrough. The inner liner **108** can include one or more folded portions **118.** The outer layer **110** can be arranged in an overlapping fashion such that an overlapping portion **120** overlaps at least a part of the folded portion **118** of the inner liner **108** as well as the underlying portion **122** (positioned to underlie the folded portion **118** of the inner liner **108)** when the sheath is unexpanded. The sheath **100** is configured to locally expand from an unexpanded configuration in which the lumen **116** has a first diameter to an expanded configuration in which the lumen **116** has a second diameter larger than the first diameter. The sheath **100** expands in response to an outwardly directed radial force exerted by a medical device against the inner liner **108** as it passes through the lumen **116.** During expansion, the first fold/folded edge moves closer to the second fold/folded edge to shorten the folded portion **118.** As shown in **FIG. 55**, in some aspects, the folded portion **118** of the inner liner **108** can completely unfold so that the inner liner **108** forms a cylindrical tube at the location of the expanded configuration. When the sheath is expanded, a portion of the inner liner **108** extends through the opening/gap provided in the outer layer **110,** where the opening is formed by the longitudinally extending edge of the overlapping portion **120** and a longitudinally extending edge of the underlying portion **122.** As the prosthetic device passes, the sheath **100** then locally contracts at least partially back to the unexpanded configuration.

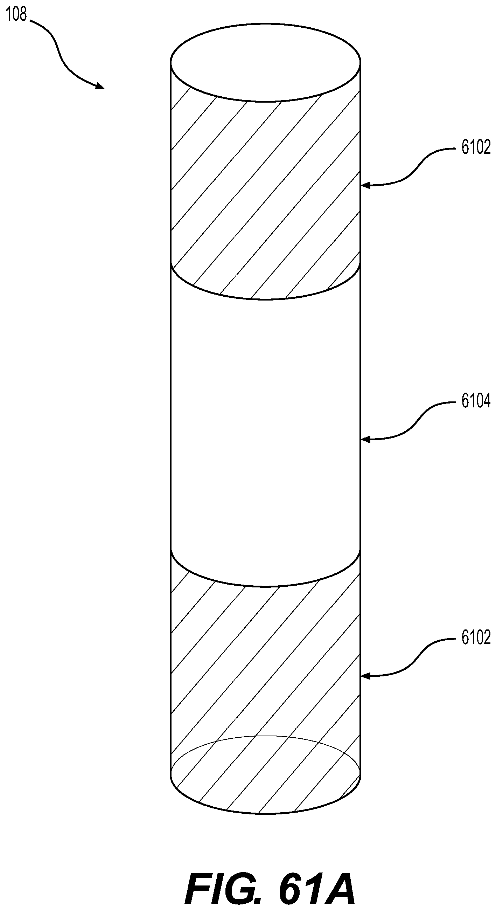

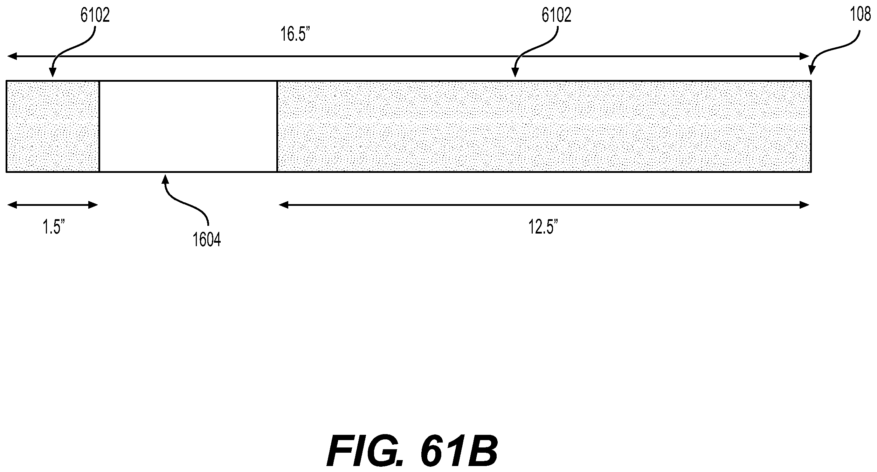

**As** described above, the sheath **100** includes an inner liner **108.** Various exemplary aspects of the inner liner **108** (before the fold has been created) are shown in **FIGS. 61-63****.** The liner **108** can comprise at least one first portion **6102,** and at least one second portion **6104.** In such aspects, the outer surface of the at least one first portion can have a composition that is substantially different from an outer surface of the at least one second portion. In yet further aspects, the outer surface of the at least one first portion can have a morphology that is substantially different from an outer surface of the at least one second portion. In such exemplary aspects, the at least one first portion **6102,** can be etched, while the at least one second portion **6104** of the inner liner is unetched.

It is understood that the etching can be done by any known in the art methods. For example, and without limitation, the etching can be done by exposing at least a portion of the inner liner to an etchant. The choice of the specific etchant can be dependent on the composition of the inner liner itself. In certain aspects and as disclosed herein, the etchant can be a liquid, fluid, or gas, a plasma. In yet other exemplary aspects, the etchant can comprise sodium naphthalene, or it can comprise a plasma.

**As** described above, the inner liner **108** can comprise polytetrafluoroethylene (PTFE), fluorinated ethylene propylene (FEP), polyimide, polyetheretherketone (PEEK), polyurethane, nylon, polyethylene, polyamide, or combinations thereof. In yet further aspects, the inner liner comprises a fluorocarbon-based polymer, for example, PTFE and/or FEP. In aspects where the inner liner is a fluorocarbon, the etchant can be present as a solution, fluid, or gas. It can also be accelerated by applying elevated temperature, electricity, or voltage, or any combination thereof. Yet, in other aspects, the etchant can be a plasma. In such an exemplary aspect, the plasma etchant can be an oxygen plasma, hydrogen plasma, nitrogen plasma, argon plasma, or any combination thereof. It is understood that the degree of etching can be easily controlled by the desired masking, temperature of the solution, or power of the plasms, and the like.

In certain aspects, to form (or preserve) unetched portions of the inner liner, these portions can be masked first. It is understood that any masking methods can be utilized. In certain aspects, the masking can be performed by covering the portions that should not be etched with the materials that can withstand etching conditions without compromising the masked inner liner. In yet other aspects, the etching can be performed such that only the portions that are desired to be etched are exposed to the etchant.

In still further exemplary aspects, where the inner liner **108** comprises PTFE, at least a portion of this inner liner can be exposed to an exemplary etchant such as a sodium naphthalene solution. The exposure to the sodium naphthalene solution can result in a reaction between the sodium naphthalene and fluorine atoms present on the outer surface of the inner liner. In such exemplary and unlimiting aspects, the outer surface of the at least one first portion can comprise a fluorine-to-carbon ratio that is lower than a fluorine-to-carbon ratio of the unetched portions.

In yet further aspects, the etching can also affect a roughness of the outer surface of the inner liner in various portions. For example, the outer surface of the at least one first portion of the inner liner can exhibit an RMS value that is lower than an RMS value of the outer surface of the at least one second portion of the inner liner.

In yet further aspects, the etching can also affect the surface energy of the outer surface of the inner liner in various portions.

In yet further aspects, the etching can also affect the polarity of the outer surface of the inner liner in various portions.

In yet further aspects, the etching can also affect contact angle with a polar solvent like water on the outer surface of the inner liner.

In still further aspects, the inner liner **108** can be fully etched, and then some of the etched portions can be further surface-modified to form at least one second portion **6104** that is different from other etched (first) portions **6102.** In such aspects, the at least one second portion can have the outer surface having a composition that is substantially different from a composition of the etched (first) portions. Similarly, there are also disclosed aspects, where the at least one second portion can have the outer surface having a morphology that is substantially different from a morphology of the etched (first) portions.

Various methods of surface modification can be utilized. In certain aspects, the surface modification can be done by exposing etched portions to the desired solution and chemically modifying the specific portions while masking the etched portions. The chemical surface modification can also be achieved by exposing the desired portions of the etched surface to a plasma treatment, for example. Yet, in other aspects, the surface modification can be a physical surface modification. It is understood that any known in the art physical surface modification can be utilized. In some aspects, the surface can be modified by the use of laser, for example, by laser ablation. While in other aspects, it can also be achieved by any method that results in the removal of the outermost layer of the outer surface of the inner liner.

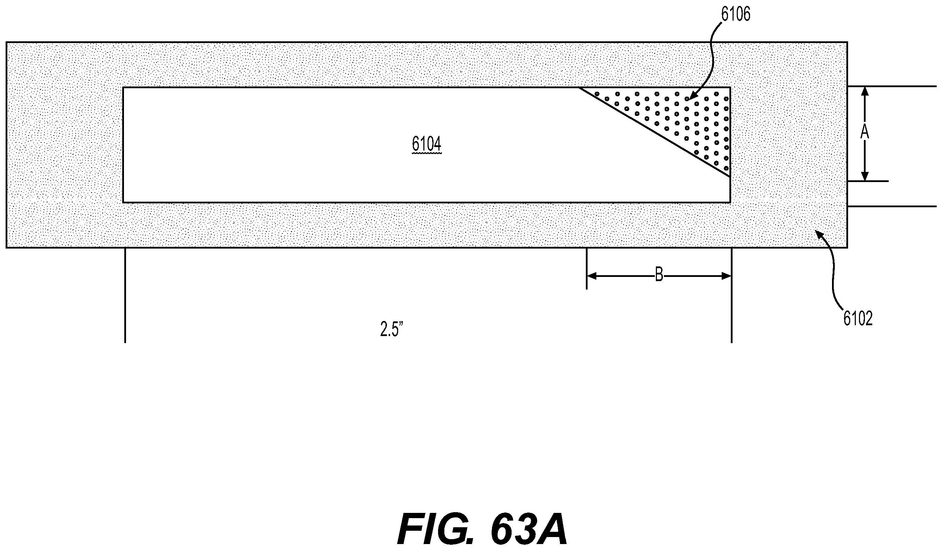

In still further aspects, when for example, and without limitations, the laser ablation is utilized to surface-modify the etched first portions to form the second portions, such second portions can comprise a gradient morphology. However, it is also understood that the gradient morphology of the second portions is not limiting, and any shape of the structure of the ablating can be implemented. It is understood that a kerf of the laser can control the actual shape, for example. In yet other aspects, the shape of the ablation can be determined by the stability of the first portions (etched portions) of the inner liner. Some exemplary schematics of the gradient morphology **6106** are shown in **FIGS. 63A-B.** It is understood that the gradient morphology can have any predetermined pattern. In some exemplary and unlimiting aspects, the gradient can have a checkered, stripped, dotted, wavy, crisscross pattern, or any combination of the patterns depending on the desired utilization. It is understood that the gradient pattern can be controlled through a masking process allowing exposure to the laser ablation only in the desired locations.

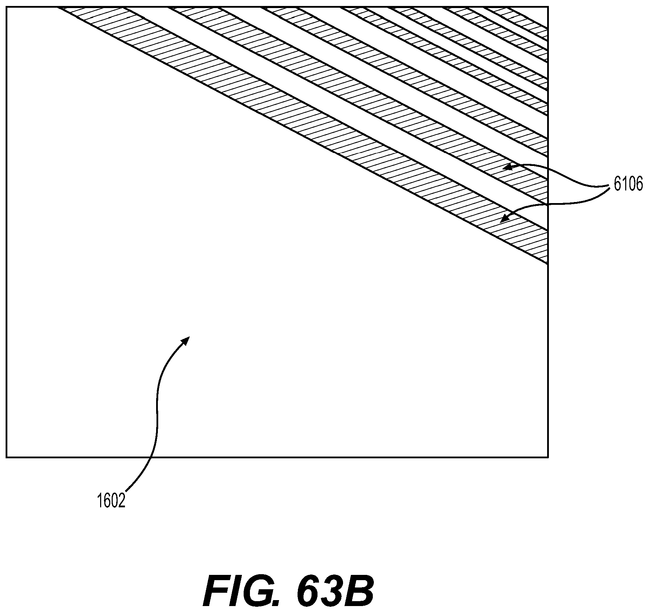

In still further aspects, the gradient morphology can be controlled by laser power, frequency, and/or speed to arrive at the pattern having a desired shape, width, spacing between the liner (specific pattern), angle, directions, and/or depth of the lines. In certain aspects, the longitudinal lines can be formed with the laser ablation. In yet other aspects, and as shown in **FIG. 63B****,** the lines formed by the laser ablation can have a different width or different angles.

In certain aspects, and as disclosed above, the at least one second portion, whether it is a completely unetched portion or it is the portion that was first etched and then surface-modified by any known in the art means, can extend longitudinally along a length of the inner liner. While in still further aspects, and as shown for example, in **FIGS. 61A-B** the at least one second portion, whether it is a completely unetched portion or the portion that was first etched and then surface-modified by any known in the art means, can extend circumferentially around a circumference of the inner liner.

In some exemplary aspects, the first and/or the second portions of the inner liner can have any shape. It is understood that in some exemplary and unlimiting aspects, the shape of the etched, unetched, or etched, and then surface-modified portions can be square, rectangular, rhombic, polygonal, trapezoid, pyramid, circular, oval, triangular, star, and the like. It is understood that in certain aspects, the second portion can have a combination of the unetched surface of the inner liner and etched and then the surface-modified surface of the inner liner. For example, the second portion can have an edge having any shape that has a gradient morphology, while the rest of the second section is unetched.

In still further aspects, the at least one second portion of the inner liner can extend along the inner portion and/or the outer portion of the at least one folded portion of the inner liner. In such aspects, the portion of the inner liner that is prepared to form a fold in the final sheath configuration can stay unetched, for example, to form second portions along the length of the inner liner forming a fold. The examples of forming such unetched portions that can form a future fold are shown in **FIGS 61A-B.** In such exemplary aspects, the portion of the inner liner that forms the fold and crease **179** can be masked to stay unetched.

**Yet** in still further aspects and as described herein, the outer surface of the inner liner can be fully etched and then some of the portions of the outer surface are surface-modified by the laser ablation, for example, to form second portions that are substantially different from the etched (first) portions of the inner liner.

In still further aspects and as disclosed herein, the inner liner can have a wall thickness. In such exemplary aspects, the wall thickness of the inner liner can be from about 0.002 inches to about 0.006 inches, including exemplary values of about 0.0025 inches, about 0.003 inches, about 0.0035 inches, about 0.004 inches, about 0.0045 inches, about 0.005 inches, and about 0.0055 inches.

In yet further aspects, the at least one first portion has a wall thickness that is different from a wall thickness of the at least one second portion. In such exemplary aspects, where, for example, the at least one second portion is unetched portion of the inner liner, the wall thickness of this portion is up to about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, or up to about 2% larger than the wall thickens of the at least one first portion.

While in still further exemplary aspects, where, for example, the inner liner is fully etched and the at least one second portion is then formed by a surface-modification of the desired etched portions of the inner liner, the wall thickness of this portion is up to about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, or up to about 2% smaller than the wall thickness of the at least one first portion.

**As** discussed in detail herein, the inner liner can comprise two or more of the first portions and/or second portions. It is understood that in the aspects where the two or more of the first portions and/or second portions are present, each of the first portions can have the same or different wall thickness, and each of the second portions can have the same or different wall thickness.

It is even further understood that if, for example, two or more second portions are present, each of these portions can comprise either unetched material or etched and then surface-modified material.

In certain aspects and as shown in **FIGS. 61A-B,** for example, the first portion can be followed by the second portion that can be then followed by an additional first portion. It is understood that each of the two or more first portions, for example, can have the same or different length. Similarly, each of the two or more second portions can have the same or different lengths. For example, and without limitation, and as shown in **FIG. 61B****,** the first portion **6102,** can have a length of about 1.5 inches starting at the proximal end of the sheath. This first portion **6102** can then be followed with the second portion **6104** having an exemplary and unlimiting length of about 2.5 inches and followed again by the additional first portion having a much larger length. Again, it is understood that this depiction is only exemplary, and any other combination of the first and second portions having any desired lengths can be present. For example, and without limitation, the second portion can be located on the proximal end of the sheath, followed by the first portion and so on.

In still further aspects, the at least one second portion, so the inner liner extends along portions of the sheath that contact the outer surface of the outer layer. It is understood that the at least one second portion of the inner liner is in contact with the outer layer around the fold of the sheath. It is further understood that having these second portions improves sheath performance by reducing the stickiness to the outer layer, for example, making an unfolding process during the passage of the medical device easier. As disclosed in detail above, the outer layer can comprise any known in the art polymers. In certain aspects, the outer layer can comprise a high-density polyethylene (HDPE), nylon, and/or polypropylene polymer.

In still further aspects, the at least one second portion of the inner liner can exhibit substantially less stickiness to the outer layer as compared to the stickiness of the at least one first portion of the inner liner to the outer layer.

In still further aspects, the outer surface of the at least one second portion exhibits a contact angle from greater than about 65° to about 140°, including exemplary values of about 70°, about 75°, about 80°, about 85°, about 90°, about 95°, about 100°, about 105°, about 110°, about 115°, about 120°, about 125°, about 130°, and about 135°. While in yet other aspects, the outer surface of the at least one first portion exhibits a contact angle from about 30° to about 90°, including exemplary values of about 35°, about 40°, about 45°, about 50°, about 55°, about 60°, about 65°, about 70°, about 75°, about 80°, and about 85°.

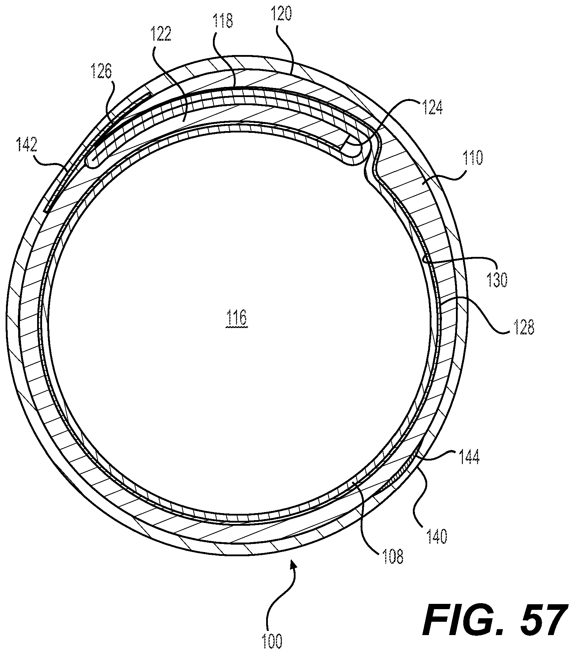

In still further aspects, and as shown in **FIG. 57****,** the sheath can also comprise an outer jacket **140.** In such aspects, the outer jacket **140** can comprise an inner surface and outer surface and a proximal end and a distal end and extend at least partially around the outer layer such that the inner surface of the outer jacket overlies the outer surface of the outer layer. In some aspects, the outer jacket can have a proximal end and a distal end. In certain aspects, the proximal end of the outer jacket is abutted to the proximal end of the sheath. Yet, in other aspects, the distal end of the outer jacket can be anywhere along the length of the sheath. While in some exemplary aspects, the distal end of the outer jacket can abut the distal end of the sheath.

In certain aspects, where the outer jacket is present the at least one second portion of the inner liner is positioned at least around the distal end of the outer jacket. In such an exemplary aspect, as shown in **FIG. 63A****,** for example, the second portion can have both unetched surface **6104** and gradient surface **6106.** In this specific and unlimiting example, the gradient portion of the second portion is in a protruding area, or in other words, in the area where the outer jacket overlaps with the outer layer of the sheath.

Also disclosed herein are sheaths where the inner liner comprises two or more folded portions. In such aspects, it is understood that the at least one second portion of the inner liner can also extend along the inner portion and/or the outer portion of each of the two or more folded portions.

Also disclosed herein are aspects referring to the sheaths having a reduced insertion force as compared to any other available commercial sheaths. As one of ordinary skill in the art would readily appreciate, the reduction in the insertion force of the sheath and any medical device passing through results in reduced or substantially eliminated danger to the patients during the medical procedure. It is understood that the insertion force of the inventive sheaths, reference sheaths, and any other commercially available sheaths is measured using the same standardized technique for a proper comparison. In such aspects, a tensile tester, Instron 3366, can be used to measure a simulated insertion force that is required for a prosthetic valve to enter the described sheath. An additional constriction tube can be used to simulate vascular elasticity that contributes to push force. In certain exemplary aspects, the test can be performed in a water bath in a temperature range from room temperature to a normal body temperature (for example and without limitation from about 20 °C to less than about 40 °C). The specimen can be kept in a straight configuration. The test can be performed with a tapered mandrel to simulate a valve entering the sheath.

The sheaths as described herein that comprise one or more first and second portions exhibit a push force upon passing of the prosthetic device of at least about 10% lower, at least about 20% lower, at least about 30% lower, at least about 50% lower than a substantially identical reference sheath that does not comprise the second portion. Still, in further aspects, the sheaths as described herein and that comprise one or more first and second portions exhibit a push force upon passing of the prosthetic device of less than about 50 N, less than about 45 N, or less than about 40 N, less than about 35 N.

In still further examples, and as discussed above, sheath **100,** unetched portions or etched and then surface-modified portions (second portions) can be provided along those surfaces of the inner liner **108** that come into contact with the outer surface of the outer layer **110.** In the exemplary aspects where the tie layer is also present, like **128,** those portions of the inner liner **108,** excluding the tie layer **128,** would not include etching. For example, it is contemplated that etching is not included between the inner surface of the folded portion **118** of the inner liner **108** and the underlying portion **122** of the outer layer **110.** By excluding etching on the portions where the inner liner **108** and the outer surface of the outer layer **110** are in direct contact helps to facilitate the release of the inner surface of the folded portion **118** and the outer layer **110** during the expansion of the sheath **100.** In yet further exemplary aspects, it is contemplated that portions that were etched and then surface-modified to form the second portions, as discussed above, are included between the inner surface of the folded portion **118** of the inner liner **108** and the underlying portion **122** of the outer layer **110.** By including these disclosed second portions where the inner liner **108** and the outer surface of the outer layer **110** are in direct contact helps to facilitate the release of the inner surface of the folded portion **118** and the outer layer **110** during the expansion of the sheath **100.**

Again as disclosed above, the wall thickness of the inner liner can vary, but in some examples, the wall thickness of the inner liner **108** ranges between about 0.002 inches and about 0.006 inches (including about 0.002 inches, about 0.003 inches, about 0.004 inches, about 0.005 inches, about 0.006 inches). In other examples, the wall thickness of the inner liner ranges between about 0.003 includes and about 0.005 inches. In a further example, the wall thickness of the inner liner **108** ranges between about 0.0035 inches and about 0.0045 inches (including about 0.0035 inches, about 0.0040 inches, about 0.0045 inches).

**As** described above, the sheath **100** includes an outer layer **110** exerting a radially inward force on the inner liner **108.** In general, the outer layer **110** can comprise a polymeric material. As described above, the outer layer **110** can be comprised of PTFE, polyimide, PEEK, polyurethane, nylon, polyethylene, polypropylene, polyamide, polyether block amides, polyether block ester copolymer, thermoset silicone, latex, poly-isoprene rubbers, high density polyethylene (HDPE), Tecoflex^{™}, or combinations thereof. In an exemplary aspect, the inner liner **108** can comprise PTFE, and the outer layer **110** can comprise a combination of HDPE and Tecoflex^{™}. The outer layer **110** can have a wall thickness ranging between about 0.007 inches and about 0.013 inches (including 0.007 inches, about 0.008 inches, about 0.009 inches, about 0.010 inches, about 0.011 inches, about 0.012 inches, about 0.013 inches).In another example, the outer layer **110** can have a wall thickness ranging between about 0.008 inches and about 0.012 inches. In another example, the outer layer **110** can have a wall thickness ranging between about 0.009 inches and about 0.011 inches.

As described above, the sheath **100** includes an outer jacket **140** that extends over and envelopes the outer layer **110.** While the outer layer **110** can be discontinuous, in that it includes a slit or a cut in order to form the overlapping and underlying portions **120, 122** as described above, the outer jacket **140** can include a continuous outer layer covering the inner and outer layers **108, 110.**

The outer jacket **140** can be constructed from an elastomeric material such as a soft polymer material having good elasticity while also being abrasion- and tear-resistant. Generally, the outer jacket **140** has relatively low tensile modules compared to the inner and outer layers **108, 110.** Example materials for the outer jacket **140** include, for example, a polyether block amide such as PEBAX^{®}, or a thermoplastic polyurethane such as Neusoft^{®} and Tecoflex^{™} 80A B20. In some examples, the outer jacket **140** comprises an elastomer (e.g., an elastic polymer) with shore hardness (durometer) ranging between about 10 and about 95 Shore A. The outer jacket **140** can comprise an elastomer with an elongation at break of ranging between about 40% and about 800% (including about 40%, about 50%, about 60%, about 70%, about 80%, about 90%, about 100%, about 150%, about 200%, about 250%, about 300%, about 350%, about 400%, about 450%, about 500%, about 550%, about 600%, about 650%, about 700%, about 800%). The outer jacket **140** can comprise an elastomer with a wall thickness ranging between about 0.003 inches and about 0.015 inches (including about 0.003 inches, about 0.004 inches, about 0.005 inches, about 0.006 inches, about 0.007 inches, about 0.008 inches, about 0.009 inches, about 0.010 inches, about 0.011 inches, about 0.012 inches, about 0.013 inches, about 0.014 inches, about 0.015 inches). The wall thickness is measured radially between the inner surface of the outer jacket **140** and the outer surface of the outer jacket **140.**

In some examples, the outer jacket **140** comprises a single material or combination of materials having a constant thickness along the entire length of the outer jacket **140.** In alternative examples, the material composition and/or wall thickness can change along the length of the outer jacket **140.** For example, the outer jacket **140** can be provided with one or more segments, where the composition and/or thickness changes from segment to segment. In an example aspect, the Durometer rating of the composition changes along the length of the outer jacket **140** such that segments near the proximal end comprise a stiffer material or combination of materials, while segments near the distal end comprise a softer material or combination of materials. Similarly, the wall thickness of the outer jacket **140** the wall thickness of the outer jacket **140** in segments near the proximal end can be thicker/greater than the wall thickness of the outer jacket **140** near the distal end.

As illustrated in **FIG. 35****,** the sheath **100** can include a tapered segment adjacent the flared end portion **114** at the proximal end of the sheath **100.** Referred to as a strain relief section, the tapered segment and the flared end portion **114** help ease the transition between the smaller diameter portion of the sheath **100** and the housing **101.** The thickness and/or composition of the outer jacket **140** can be adjusted to increase the Durometer and/or stiffness along the strain relief section. Because this portion of the sheath **100** is usually outside of the patient's body during the procedure, providing the outer jacket **140** with an increased Durometer and/or stiffness along the strain relief section helps to withstand the blood pressure that would otherwise cause the outer jacket **140** to "balloon up" with body fluid/blood. As a result, it allows for a sheath **100** having a relatively stiff proximal end at the point of introducing a delivery apparatus while still having a relatively soft distal tip at the point of entry into the patient's vessel.

The outer jacket **140** can be bonded to the outer layer **110** to prevent the outer jacket **140** from sliding over the outer layer **110** and "bunching up" in response to the friction forces applied by the surrounding tissue during insertion of the sheath 100 into the patient's vasculature. For example, the outer jacket **140** can be bonded at the proximal end and/or distal end of the outer layer **110.** At the proximal and distal ends, the outer jacket **140** can be bonded to the outer layer 110 around the full circumference of the outer layer. At the distal end of the sheath **100,** the outer jacket **140** can alternatively be bonded to the inner liner **108.** For example, the outer jacket 140 can be bonded to the distal end surface of the inner liner **108.**

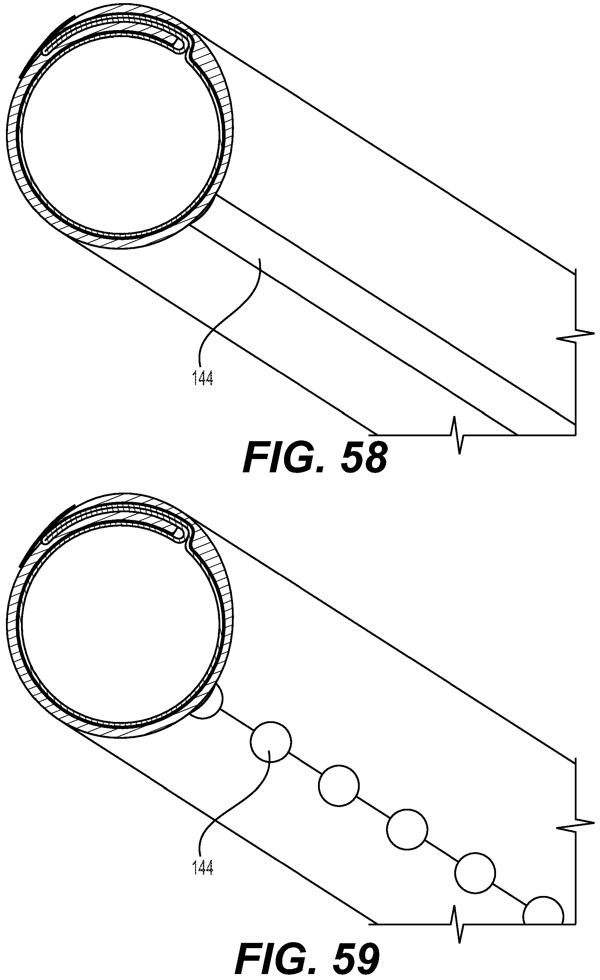

As illustrated in **FIG. 57****,** the outer jacket **140** can be bonded 144 to the outer layer **110** at a circumferential location opposite the folded portion of the inner liner **108.** As provided in **FIGS. 58-59****,** the bond **144** can be spot bonds or linear bond lines extending along all or a portion of the outer layer 110. As provided in **FIG. 57****,** the bond **144** line/spot will also have a width, extending circumferentially around the outer layer **110.** For example, the bond line can cover about 5° to about 90° of the circumference of the outer layer **110** (including about 5°, about 10°, about 15°, about 20°, about 25°, about 30°, about 35°, about 40°, about 45°, about 50°, about 55°, about 60°, about 65°, about 70°, about 75°, about 80°, about 85°, about 90°).

The outer jacket **140** can be bonded to the outer layer **110** and/or inner liner **108** using any mechanical and/or chemical (e.g., adhesive) fastener known in the art. In one example, sheath **100,** the outer jacket **140** and the outer layer **110** and/or inner liner **108** may have similar melting temperatures. Accordingly, an example bonding method includes a thermally bonded coupling between the outer jacket **140,** the outer layer **110,** and/or inner liner **108.** For example, the bond between the outer jacket **140** and the outer layer **110** can be achieved by laser welding and/or a heat compression (e.g., using a heat compression jaw), allowing the location of the bond line to be closely controlled.

As shown in **FIG. 54****,** and described above with respect to **FIG. 39****,** an adhesive layer **128** (e.g., a tie layer) is provided between the inner liner **108** and the outer layer **110** to at least partially adhere the inner liner **108** to the outer layer **110.** In some aspects, the adhesive layer **128** can be selectively provided/located between the inner liner **108** and the outer layer **110** to bond the inner and outer layers **108, 110** at the selected locations of the adhesive layer **128.**

As illustrated in **FIG. 54** (and **FIG. 39**) the adhesive layer **128** is provided on the outer surface of the inner liner **108** and/or the inner surface **130** of the outer layer **110.** For example, the adhesive layer **128** can be provided partially or entirely around the outer surface of the inner liner **108.** Additionally, or alternatively, the adhesive layer **128** can be provided partially and/or entirely around the inner surface **130** of the outer layer **110.** As illustrated in **FIG. 54****,** the adhesive layer **128** extends between the outer layer **110** and the overlapping folded portion **118** of the inner liner **108.** In other aspects, the adhesive layer **128** can extend between the outer surface of the folded portion **118** of the inner liner **108** and the corresponding inner surface of the overlapping portion **120** of the outer layer **110.** As illustrated in **FIG. 54****,** the adhesive layer **128** does not extend between an inner surface of the overlapping folded portion **118** of the inner liner **108** and a corresponding surface of the underlying portion **122** of the outer surface of the outer layer **110.** Excluding the adhesive layer **128** on the portion of the sheath between the inner surface of the folded portion **118** and the underlying portion **122** facilitates expansion of the sheath and prevents undesirable bonding/sticking between the inner and outer layers **108, 110** at this location.

The adhesive material **128** can comprise a material having a Shore A hardness (durometer) less than about 90A. For example, the adhesive material **128** can comprise a thermoplastic polyurethane such as an aliphatic polyether-based thermoplastic polyurethane (TPU). An example of TPU includes Tecoflex^{™} 80A. The adhesive layer **128** can also be composed of an aromatic polyether or polyesters based thermoplastic polyurethane such for example, Pellethane^{™} 80A. The adhesive layer can also be composed of a polyolefin or polyamide, including, for example, a polyolefin (PE, PP, or EVA) modified with maleic anhydride such as an Orevac^{™} resin.

The thickness (wall thickness) of the adhesive layer **128** can vary, but in some examples, the wall thickness of the adhesive layer ranges between about 0.002 inches and about 0.005 inches (including about 0.002 inches, about 0.003 inches, about 0.004 inches, about 0.005 inches). In other examples, the wall thickness of the adhesive layer **128** ranges between about 0.0025 and about 0.0040 (including about 0.0025 inches, about 0.0030 inches, about 0.0035 inches, about 0.0040 inches). In a further example, the wall thickness of the adhesive layer **128** ranges between about 0.0025 inches and about 0.0035 inches (including about 0.0025 inches, about 0.0030 inches, about 0.0035 inches).

In some examples, the sheath **100** can include a lubricant to reduce friction and facilitate expansion/contraction between the outer layer **110** and the outer jacket **140.** The lubricant **142** allows the outer layer **110** and inner liner **108** to unroll easily under the outer jacket **140,** ensuring that the hemostasis and atraumatic benefits achieved by the addition of an outer jacket 140 does not comprise the push force performance of the sheath **100.** I some aspects, the lubricant **142** can reduce the push force necessary to move the prosthetic device through the central lumen **116** of the inner liner **108** during delivery of the prosthetic device and the corresponding local expansion of the sheath **100.**

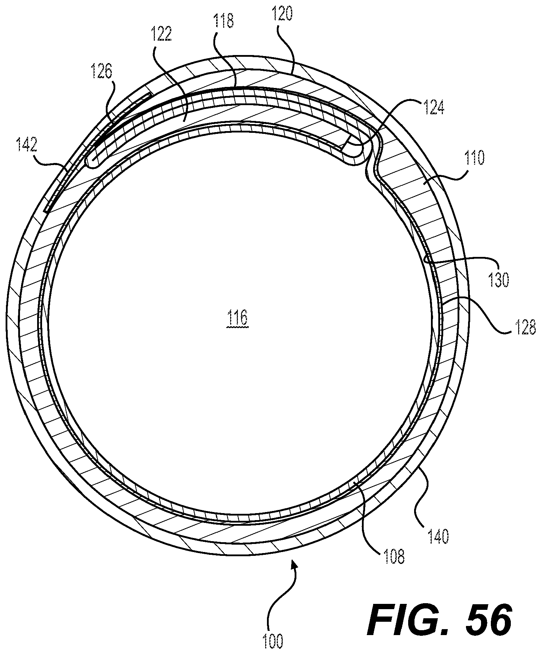

As illustrated in **FIG. 56****,** the lubricant **142** can be selectively applied along an outer surface of the outer layer **110** proximate the longitudinally extending edge **126** of the overlapping portion **120.** In some examples, a portion of the folded portion **118** of the inner liner **108** extends beyond the longitudinally extending edge **126** of the overlapping portion **120** and along an outer surface of the outer layer **110.** In this example, the lubricant **142** is also provided along the protruding portion of the folded portion **118** of the inner liner **108** extending along the outer surface of the outer layer **110** (beyond the edge **126**). In this location, the lubricant **142** also reduces friction between the outer jacket 140 and the inner liner **108** during expansion of the sheath **100.** As illustrated in **FIG. 56****,** the lubricant **142** extends around the circumference of the outer layer **110** beyond the protruding portion of the folded portion **118.**

The lubricant is applied as a band (or spot) that extends both circumferentially and longitudinally along the outer layer **110** (and the protruding portion of the inner liner **108**). In an example sheath **100,** the lubricant **142** is applied as a band that extends both circumferentially around the outer layer **110** and longitudinally along a length of the outer layer **110.** To prevent migration, the lubricant **142** can be composed of a heat-curable material, e.g., a material curable at room temperature. As a result, the material can be applied to a desired location along the outer layer **110** and does not migrate during assembly and/or use of the sheath **100.** The lubricant **142** can be composed of a medical-grade lubricant, such as silicone. Example lubricants include medical-grade curable silicone lubricants, including a platinum-catalyzed thermal curing silicone lubricant such as NuSil^{™} MED10-6670 (heat-curable), a PTFE lubricant such as Duraglide^{™} (curable at room temperature) and/or CHRISTO-LUBE^{™}.

**FIG. 60** illustrates an additional aspect of the sheath **100** of **FIG. 35****.** In this example, the sheath **100** can include a coiled wire **160,** or coiled wire mesh, along a length of the sheath **100.** The coiled wire **160** provides uniform bending of the sheath and prevents kinking. The coiled wire **160** can be embedded in the outer layer b. For example, the coiled wire **160** can be co-extruded with the outer layer **110.** Alternatively, the coiled wire **160** can be provided between the outer layer and the adhesive layer **128.** In another example, the coiled wire **160** is embedded, at least partially, within both the outer layer **110** and the adhesive layer **128.** For example, the coiled wire **160** can be provided on an outer surface of the adhesive layer **128,** and the outer layer **110** is reflowed over.

As illustrated in **FIG. 60****,** the coiled wire **160** defines a helical-shaped path around the longitudinal axis of the sheath **100.** The example of coiled wire **160** includes an overlapping helical-shaped path around the longitudinal axis of the sheath **100,** resulting in a continuous diamond-shaped pattern along the length of the sheath.

The coiled wire **160** can be comprised of a metal or a polymer wire. For example, the coiled wire **160** can be composed of PET, PEEK, stainless steel, and/or nitinol. The coiled wire **160** can be comprised of a flat wire, a round wire, or a combination thereof. The individual wires of the coiled wire **160** can have a diameter/thickness ranging between about 0.002 inches and about 0.008 inches (including about 0.002 inches, about 0.003 inches, about 0.004 inches, about 0.005 inches, about 0.006 inches, about 0.007 inches, about 0.008 inches). In another example, the individual wires of the coiled wire **160** can have a diameter/thickness ranging between about 0.004 inches and about 0.007 inches. In a further example, the individual wires of the coiled wire **160** can have a diameter/thickness of about 0.006 inches. The pitch/distance between adjacent coils of the coiled wire **160** can correspond to the diameter/thickness of the coiled wire. For example, where the diameter/thickness of a single coil of the coiled wire is about 0.006 inches, the spacing/pitch between the wire and the next adjacent coiled wire is about 0.006 inches.

Methods of using the sheath of **FIGS. 53-60** include first inserting the expandable sheath **100** into the vasculature of a subject and advancing a prosthetic device through the inner lumen **116** of the inner liner **108**/sheath **100.** The prosthetic device applies an outwardly directed radial force on the inner liner **108** of the expandable sheath **100.** In some aspects, the outwardly directed radial force is transmitted through the inner liner **108,** the adhesive layer **128,** and the outer layer **110.** The lumen **116** of the sheath **100** expands at the axial location of the prosthetic device due to the outwardly directed radial force exerted by a prosthetic device against an inner surface of the lumen during advancement. During expansion of the lumen **116,** the first fold (folded edge) of the folded portion **118** is moved circumferentially closer to the second fold (folded edge), shortening the overlapping portion of the folded portion **118** that extends circumferentially between the first and second folds, thereby increasing the circumference of the lumen **116.**

**As** the sheath **100** expands at a particular location (i.e., locally expands at the location of the passing prosthetic device), the overlapping portion **120** of the outer layer **110** can move circumferentially with respect to the underlying portion **122** as the folded portion **118** of the inner liner **108** least partially separate and/or unfold, causing the elongate gap(s) **132** provided in the outer layer **110** to widen/expand. The sheath thereby expands to accommodate a medical device having a diameter larger than that of lumen **116** in the resting (unexpanded) configuration. As shown in **FIG. 55****,** in some aspects, the folded portion of the inner liner **108** can completely unfold so that the inner liner **108** forms a cylindrical tube at the location of the expanded configuration. As illustrated in **FIGS. 54** and **55****,** the elongate gap **132** is generally aligned with the longitudinal axis of the lumen **116** such that during expansion, the unfolded portion of the inner liner **108** expands into the gap **132.**

In an unexpanded configuration, the sheath **100** can have an outer diameter less than about 0.030 inches (including less than about 0.029 inches, less than about 0.028 inches, less than about 0.027 inches, less than about 0.026 inches, less than about 0.025 inches, less than about 0.024 inches). Preferably, the unexpanded sheath has an outer diameter ranging between about 0.024 inches and about 0.026 inches. In a fully expanded configuration, the sheath **100** can have an inner diameter greater than 0.040 inches. Preferably, the expanded sheath **100** has an inner diameter ranging between 0.046 inches and 0.054 inches (including about 0.046 inches, about 0.047 inches, about 0.048 inches, about 0.049 inches, about 0.050 inches, about 0.051 inches, about 0.052 inches, about 0.053 inches, about 0.054 inches)

As described above, the inner and outer layers **108, 110** can be bonded together using an adhesive layer **128.** The adhesive layer **128** prevents movement, both longitudinal and radial, between the inner and outer layers **108, 110.** As a result, expansion of the sheath **100** can be limited to only those regions excluding the adhesive layer **128.** For example, as illustrated in **FIG. 54****,** because the adhesive layer **128** is not provided between the inner surface of the folded portion **118** and the underlying portion **122** of the outer layer, expansion of the sheath results in the inner surface of the folded portion extending into the gap **132** created between the first and second edges **124, 126** of the expanded outer layer **110.**

Once the prosthetic device is passed through the lumen **116** (or a particular location along the lumen), the lumen **116** of the sheath can at least partially contracts back to an unexpanded configuration. The outer layer **110** can exert an inwardly directed radial force on the inner liner **108** urging it back to its original folded configuration. Similarly, if a coiled wire **160** was included, the coiled wire can exert an inwardly directed radial force on the outer layer **110** and the inner liner **108** urging them back towards an unexpanded configuration. Likewise, if an outer jacket **140** is included, the outer jacket **140** can exert an inwardly directed radial force on the outer layer **110** and the inner liner **108** urging them back to an unexpanded configuration.

**The** prosthetic device can be delivered through the distal end of the sheath **100** to the delivery site within the patient. The prosthetic device can include a self-expanding heart valve or a stent-mounted heart valve. The heart valve can be extended through the distal end of the elongate lumen **116** at the delivery site. Once outside the lumen **116,** the heart valve can be expanded, and the sheath **100** removed from the treatment site.

An example method of making the sheath is as follows. These steps are not meant to be limiting. The steps given can be reordered as needed. Other steps can be added, or in other examples, some steps may not be necessary. A continuous inner liner **108** defining a lumen therethrough is provided. The inner liner **108** is formed to include a first fold and a second fold and an overlapping folded portion **118** extending circumferentially between the first and second folds. The overlapping folded portion **118** is formed to include overlap in a radial direction of at least two thicknesses of the inner liner **108.** The inner liner **108** can be extruded, including the folded portion **118.** Alternatively, the folded portion **118** can be formed after the inner liner **108** is extruded (e.g., formed on a cylindrical tubular structure).

A discontinuous outer layer **110** is provided (at least partially) around the inner liner **108.** The outer layer **110** is formed to include an overlapping portion **120** and an underlying portion **122** such that at least a portion of the folded portion **118** of the inner liner **108** is positioned between the overlapping portion **120** and the underlying portion **122.** In some aspects, the inner liner **108** and the outer layer **110** are coextruded. In alternative aspects, the inner liner **108** and the outer layer **110** are separately formed and joined together.

**An** adhesive layer **128** is provided between the inner liner **108** and the outer layer **110** for bonding (at least partially) the inner liner **108** to the outer layer **110.** The adhesive layer 128 can be applied to and bond the inner and outer layers **108, 110** axially along a length of the sheath, e.g., along a portion of the entire length of the sheath or along the entire length of the sheath. In an example, the adhesive layer **128** is coextruded with the outer layer **110.** In a further example, the adhesive layer **128** is coextruded with the inner liner **108.** In an alternate example method, the adhesive layer **142** can be applied to the outer surface of the inner liner **108** and/or an inner surface of the outer layer **110.**

After the adhesive layer **128** is applied to the desired locations along the inner and/or outer layer **110,** the outer layer **110** is applied over the inner liner **108.** The outer layer **110** can then be bonded to the inner liner **108** by heat curing the adhesive layer **128.** The adhesive layer **128** can comprise a material curable at a temperature above room temperature, in which case a heat treatment may be applied to the assembled inner liner **108**/outer layer **110.** The adhesive layer **128** may also be composed of a material that cures at room temperature. Accordingly, after application of the adhesive layer **128** and assembly of the outer layer **110** over the inner liner **108** (at a temperature below room temperature), the temperature of the combined layers may be increased to room temperature.

As illustrated in **FIG. 56****,** the lubricant **142** can be selectively applied to the desired location along a length of outer layer **110** and/or outer jacket **140.** As described above, the lubricant **142** can be provided on an outer surface of the outer layer **110** proximate a longitudinally extending edge **126** of the overlapping portion **120,** on any portion of the folded portion **118** extending/protruding beyond edge **126,** and/or on any portion of the outer surface of the outer layer **110** adjacent the protruding portion of the folded portion **118.** The lubricant **142** can be applied as a band extending around a portion of the circumference of the outer layer, the band of lubricant **142** also extending longitudinally along a length of the outer layer **110.** After the lubricant **142** is selectively applied to the outer surface of the outer layer **110,** the outer jacket **140** can be applied over the outer layer **110.** As outlined above, the lubricant **142** can comprise a heat-curable material, in which case a heat treatment can be applied to the outer layer **110**/outer jacket **140.** The lubricant **142** may also be composed of a material that cures at room temperature. Accordingly, after application of the lubricant **142** (at a temperature below room temperature), the temperature of the outer layer **110** (separately or in combination with the outer jacket **140**) can be increased to room temperature.

The outer jacket **140** can then be applied over/around the outer layer **110** and bonded to the outer layer **110** at at least one of the proximal and distal ends of the outer layer **110.** The outer jacket **140** can also be bonded to the outer layer **110** along a length of the outer layer **110.** The outer jacket **140** can be bonded to the outer layer **110** via a heat treatment process, e.g., a reflow process where the outer layer **110** and the outer jacket are headed to a temperature high enough such that the outer layer **110** and the outer jacket **140** are at least partially melted and are then fused together are the heat is removed, and the assembly cools. The entire sheath **100** assembly may be reflowed to reduce the overall outer diameter and regain/ensure a circular shape in cross-section.

As described above, select portions of the outer surface of the inner liner **108** can include a surface treatment such as surface etching. In an example method, surface treatment of the inner liner **108** would occur before application of the outer layer **110.** It is contemplated that it may be desirable to exclude etching from those surfaces of the inner liner **108** that come into contact with the outer surface of the outer layer **110.** For example, etching may not be included between the inner surface of the folded portion **118** of the inner liner **108** and the underlying portion **122** of the outer layer **110.** By excluding etching on the portions where the inner liner **108** and the outer surface of the outer layer **110** are in direct contact helps to facilitate release of the inner surface of the folded portion **118** and the outer layer **110** during expansion of the sheath **100.**

In some instances, it may be necessary to release ("break") any undesirable bonding that occurs between the outer layer **110** and the inner liner **108.** This bonding can occur due to the etching on the outer surface of the inner liner **108** that allows it to stick directly to the outer layer **110** (with/without the adhesive layer **128**). Undesirable bonding can also occur if the outer layer **110** can flows on top of the folded inner liner **108** and "grabs" it during the reflow process.

However, when the inner liner **108** is unetched along those locations excluding the adhesive layer **128,** e.g., those portions adjacent the underlying portion of the outer layer **110,** undesirable bonding between the outer layer **110** and the inner liner **108** at this location is limited. Therefore, it may not be necessary to precondition the sheath to release undesirable bonding between the inner liner **108** and the outer layer **110** because the bonding has not occurred (or is less likely to occur).

Nonetheless, undesirable bonding between the inner liner **108** and the underlying portion **122** of the outer layer **110** can be released while maintaining desirable bonding at the proximal and distal ends of the sheath **100.** For example, a mandrel can be passed at least partially through the lumen **116** of the inner liner **108,** expanding the inner liner **108** and the outer layer **110,** and breaking/releasing any undesirable bonding between the inner liner **108** and the underlying portion **122** of the outer layer **110.**

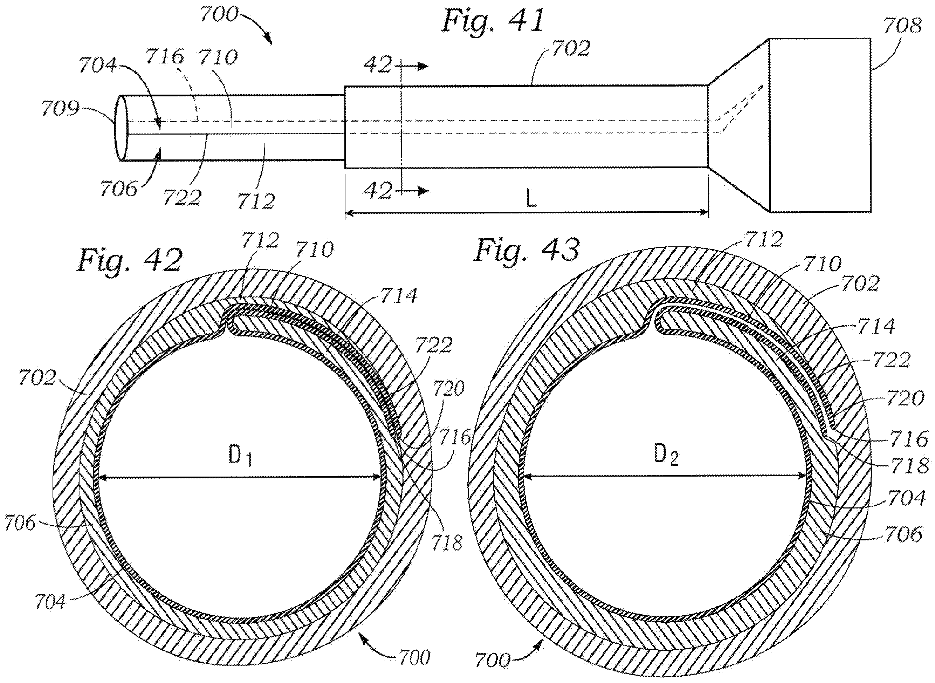

**FIGS. 41-49** illustrate additional aspects and variations on the general sheath **100** described above. It is to be understood that the variations (*e.g*., materials and alternate configurations) described above with reference to **FIGS. 35-40** can also apply to the aspects shown in **FIGS. 41-49****.** Furthermore, the variations described below with reference to **FIGS. 41-49** can also be applied to the sheath described in **FIGS. 35-40****.**

**FIGS. 41-43** illustrate a sheath **700** that additionally includes a strain relief cover, also referred to as an elastic outer cover, or an elastic cover **702** positioned around at least a part of an inner liner **704** and outer layer **706.** As shown in **FIG. 41****,** the elastic cover **702** can extend for a length L along at least a portion of the main body of the sheath **700.** In some aspects, the elastic cover **702** can extend from the proximal end **708** of the sheath **700** and towards the distal end **709** of the sheath. In some aspects, the elastic cover **702** extends only partway down the length of the sheath **700.** In alternate aspects, the elastic cover **702** can extend to a point adjacent the distal end **709** or can extend all the way to the distal end **709** of sheath **700.** Furthermore, the elastic outer cover **702** need not extend all the way to the proximal end **708** of the sheath **700.** In some aspects, the elastic outer cover **702** may extend only partway towards the proximal end **708.** In some aspects, the longitudinal length L of the elastic cover **702** can range from about 10 cm to the entire length of the sheath **700.**

As shown in **FIGS. 42** and **43,** the elastic cover **702** can be a continuous tubular layer, without slits or other discontinuities. The elastic cover **702** can be positioned to surround the entire circumference of outer layer **706** and can extend longitudinally along any portion of the length of the sheath **700.** The elastic outer cover **702** can comprise any pliable, elastic material(s) that expand and contract, preferably with a high expansion ratio. Preferably, the materials used can include low durometer polymers with high elasticity, such as PEBAX^{®}, polyurethane, silicone, and/or polyisoprene. Materials for the elastic outer cover **702** can be selected such that it does not impede expansion of the sheath **700.** In fact, the elastic outer cover 702 can stretch and expand as the sheath **700** expands, such as by movement of the folded or scored inner liner with respect to itself.

The elastic outer cover **702** can, in some aspects, provide hemostasis (*e.g*., prevent blood loss during implantation of the prosthetic device). For example, the elastic outer cover **702** can be sized or configured to form a seal with the patient's artery when inserted, such that blood is substantially prevented from flowing between the elastic outer cover **702** and the vessel wall. The elastic outer cover **702** can be inserted such that it passes the arteriotomy. For example, in aspects where the elastic outer cover **702** does not extend all the way to the distal end **709** of the sheath **700,** the elastic cover **702** can extend distally far enough such that when the sheath **700** is fully inserted into the patient, at least part of the elastic outer cover extends through the ateriotomoy site.

The elastic outer cover can have a thickness ranging from, for example, about 0.001" to about 0.010." In some aspects, the outer cover can have a thickness of from about 0.003" to about 0.006." The elastic outer cover can be configured to expand as the sheath expands, as shown in the expanded configuration in **FIG. 43.**

**FIG. 42** shows a cross-section of the sheath **700** in a resting configuration having an inner diameter D₁. **FIG. 3** shows a cross-section of the sheath **700** in an expanded configuration, having an inner diameter D₂, where D₂ is greater than D₁. Similar to the aspect of **FIGS. 35-40****,** the sheath **700** can include an inner liner **704** having a folded portion **710,** and an outer layer **706** having an overlapping portion **712** and an underlying portion **714.** The overlapping portion **712** overlaps at least a portion of the folded portion **710** of the inner liner, and the underlying portion **714** underlies at least a portion of the folded portion **710.** As shown in **FIGS. 42-43,** in some aspects, the overlapping portion **712** does not overlap the entire folded portion **710** of the inner liner **704,** and thus, a portion of the folded portion **710** can be directly adjacent to the elastic outer cover **702** in locations where the elastic cover **702** is present. In locations where the elastic cover **702** is not present, part of the folded portion **710** may be visible from the outside of the sheath **700,** as seen in **FIG. 41****.** In these aspects, the sheath **700** can include a longitudinal seam **722** where the overlapping portion **712** terminates at the folded portion **710.** In use, the sheath can be positioned such that the seam **722** is posterior to the point of the sheath that is 180 degrees from the seam **722** (*e.g*., facing downward in the view of **FIG. 41**). The seam **722** can also be seen in **FIG. 41****,** which shows that the seam **722** need not extend the entire length of the sheath. In some aspects, the proximal end portion of the sheath includes two layers without a folded portion (*e.g*., similar to **FIG. 38**), while the distal end portion of the sheath includes two layers with a folded portion (*e.g*., similar to **FIG. 39**). In some aspects, the seam **722** can end at a transition point between portions of the sheath having a folded inner liner and portions of the sheath not having a folded inner liner.

In some aspects, the folded portion **710** can include a weakened portion, such as a longitudinal perforation, score line, and/or slit **716** along at least a portion of the length of the inner liner **704.** The slit **716** can allow for two adjacent ends **718, 720** of the folded portion **710** to move relative to one another as the sheath **700** expands to the expanded configuration shown in **FIG. 43.** As a device having an outer diameter device larger than the initial resting inner diameter of the sheath **700** is inserted through the sheath **700,** the device can cause local expansion of the sheath **700** and cause the sheath **700** to expand at the partial score or split line location **716.** The weakened portion **716** can extend longitudinally along any portion of the expandable sheath **700.**

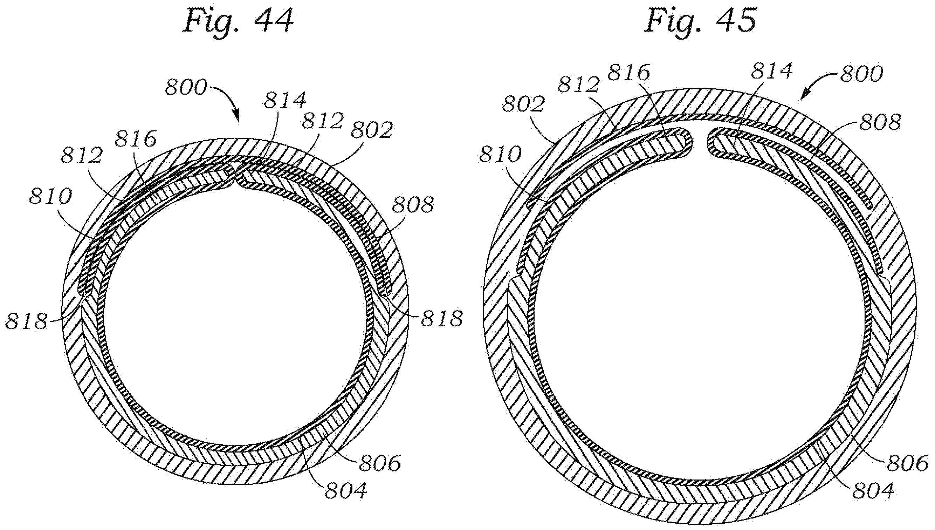

**FIGS. 44** and **45** show another aspect of an expandable sheath 800 having an initial diameter in a resting configuration (**FIG. 44**) and a larger expanded diameter in an expanded configuration (**FIG. 45**). The sheath **800** can include an elastic outer cover **802,** an inner liner **804,** and an outer layer **806.** Inner liner **804** can include first and second folded portions **808, 810.** The folded portions **808, 810** can be arranged such that they fold away from one another in opposite directions around the circumference of the sheath **800.** For example, folded portion **808** can be folded to the right in the view of **FIG. 44** and folded portion **810** can be folded to the left such that they do not overlap one another but share a common segment **812,** which is part of both folded portions **808, 810.** In contrast to previous aspects, the outer layer 806 does not include an overlapping portion in this aspect but rather has first and second underlying portions **814, 816,** which underlie the first and second folded portions **808, 810,** respectively. The inner liner **804** can extend through a gap between the ends of the adjacent underlying portions **814, 816** (*e.g*., between a first end and a second end of discontinuous outer layer **806**).

Each folded portion **808, 810** can include a weakened portion **818,** such as a slit, score line, and/or perforation. Weakened portion **818** can allow the expandable sheath **800** to expand easily without a high radial force. As the sheath **800** expands, segment **812** along the top of the folded portions **808, 810** of inner liner **804** can be configured to split apart from the rest of the folded portions **808, 810** and the first and second underlying portions **814, 816** can move away from one another so as to create an enlarged lumen within the inner liner **804.** Weakened portions **818** can allow for the segment **812** to easily split apart from the inner liner **804** as the sheath **800** expands.

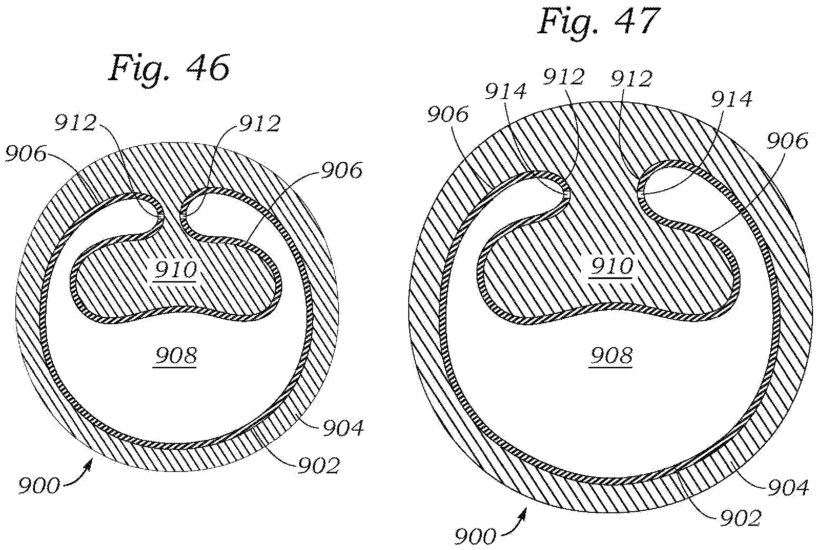

**FIGS. 46-47** show another aspect of an expandable sheath **900.** Sheath **900** can be provided with an inner liner **902** and an elastic cover **904** surrounding the inner liner **902.** While not shown, sheath **900** can additionally include an intermediate layer positioned between the inner liner **902** and the elastic cover **904.** If present, the intermediate layer can closely follow the contour of the inner liner **902.**

Inner liner **902** can be shaped to include one or more folded portions **906** arranged to form a generally horseshoe-shaped lumen **908** that extends longitudinally through sheath **900** along the inner surface of the inner liner **902.** The folded portions 906 can be arranged to form an area **910** positioned with the lumen **908** and radially inward from the elastic cover **904.** In some aspects, the area **910** can include one or more voids (*e.g*., smaller lumens or openings extending through portion **910**). In some aspects, the area **910** can be filled with material (*e.g.*, HDPE) reflowed from an intermediate layer while the sheath is being made. In some aspects, the area 910 can be filled with material reflowed from the elastic cover **904** during the sheath manufacturing process.

The inner liner **902** can include one or more weakened portions **912,** such as score lines, perforations, or slits. The weakened portions **912** can be configured to split apart, separate, or widen as the sheath expands from its initial resting configuration (**FIG**. **46**) to an expanded configuration (**FIG**. **47**) in the presence of a radial force. As the sheath **900** expands, material from the area **910** can cover any gaps **914** formed at the weakened portions **912,** thereby keeping the lumen **908** substantially sealed.

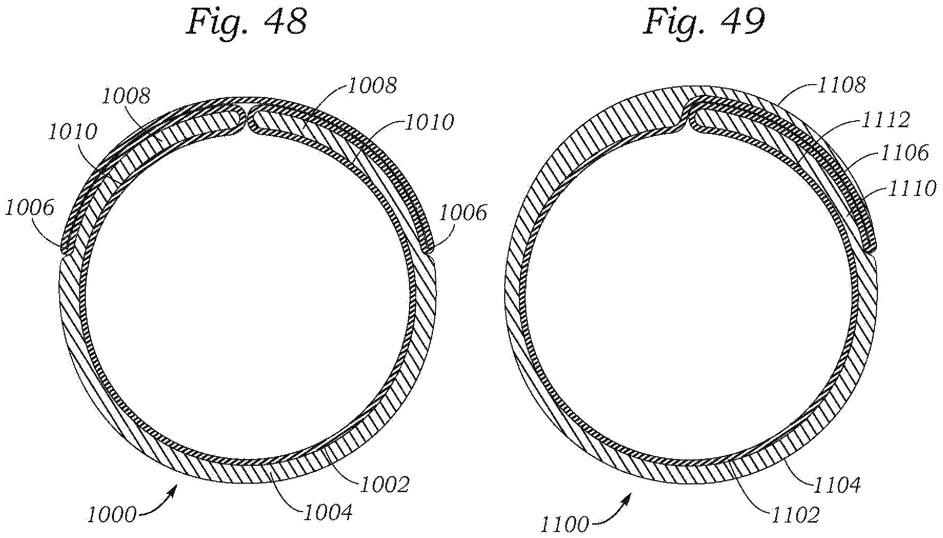

**FIG. 48** shows another aspect of an expandable sheath **1000** having an inner liner **1002** and a discontinuous outer layer **1004.** Sheath **1000** is similar to the sheath **800** of **FIG. 44****,** except that sheath **1000** is shown without an elastic outer cover, and further, the inner liner **1002** is continuous, without weakened portions at the folds **1006.** As shown in **FIG. 48****,** the inner liner **1002** can be configured to have one or more folds **1006** (*e.g*., two folds positioned on the outer surface of the outer layer **1004**), with portions **1008** of the outer layer **1004** extending between the folds **1006** and the outer surface **1010** of the inner liner **1002** underlying the folds **1006.**

**FIG. 49** shows another aspect of an expandable sheath **1100** having an inner liner **1102** and an outer layer **1104.** The sheath **1100** is similar to the sheath **100** shown in **FIG. 39** in that the inner liner **1102** can be continuous with a folded portion **1106,** and the outer layer **1104** can be discontinuous with an overlapping portion **1108** overlapping at least a part of the folded portion **1106** and an underlying portion **1110** underlying at least a part of the folded portion **1106.** The underlying portion **1110** can thus be positioned between an outer surface **1112** of the lumen-forming portion of the inner liner **1102** and the folded portion **1106.**

The inner liners **1002, 1102** of the sheaths **1000, 1100,** respectively, of **FIGS. 48-49** can be optimized to perform slightly differently than the inner liners of sheaths described above. For example, different materials can be used for the inner liner to increase the durability and softness of the seam (although such materials can also be used with the other aspects of expandable sheaths described above). For example, materials such as woven fabrics or braid filaments can be used. Such fabrics, filaments, or yarns can comprise, for example, PTFE, PET, PEEK, and/or nylon yarns or filaments. These materials can advantageously provide a soft and flexible layer that can be easily formed into the desired shapes or folded portions. Additionally, such materials can withstand high temperatures, as well as can possess high tensile strength and tear resistance. Nonetheless, these materials can also be elastic, experience minimal kinking, and provide soft distal edges for less traumatic insertion into a patient's vessels.

Various methods can be used to produce the sheaths discussed above and below throughout the present disclosure. For example, a method of making the sheath shown in **FIGS. 2A-2D** can comprise providing a mandrel and applying an inner liner on the mandrel, such as by spray coating or dip coating the mandrel. An intermediate layer, such as a mesh structure, can then be mounted on the inner liner. An outer layer can be applied over the intermediate layer, such as by a second spray coating or dip coating step. Methods can comprise etching or surface treating at least a portion of the inner liner. Also, methods can comprise providing one or more notches and/or cuts in the inner liner and/or the outer layer. Cuts and/or notches can be provided by, for example, laser cutting or etching one or more layers.

In some aspects of methods of making a sheath, such as the sheaths illustrated in **FIGS. 2A-2D****,** layers can be pre-formed and mounted on a mandrel and then fused or thermally bonded together. For example, in one method, an inner liner is applied to a mandrel. An intermediate layer can be applied to the outer surface of the inner liner. An outer layer can be applied to the outer surface of the intermediate layer. Heat shrink tubing can be applied and the assembly heated, such that the inner liner, the intermediate layer, and/or the outer layer are thermally bonded and compressed together under the heat shrink tubing.

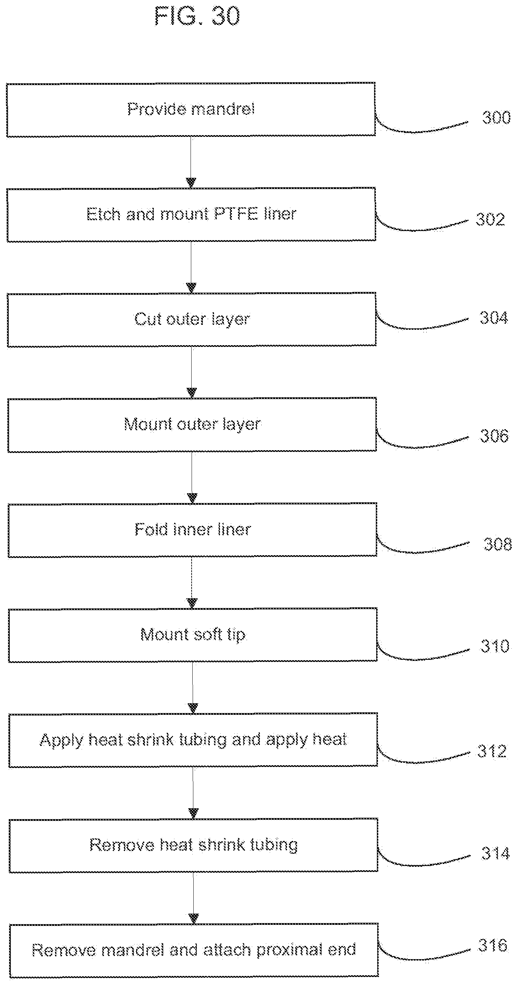

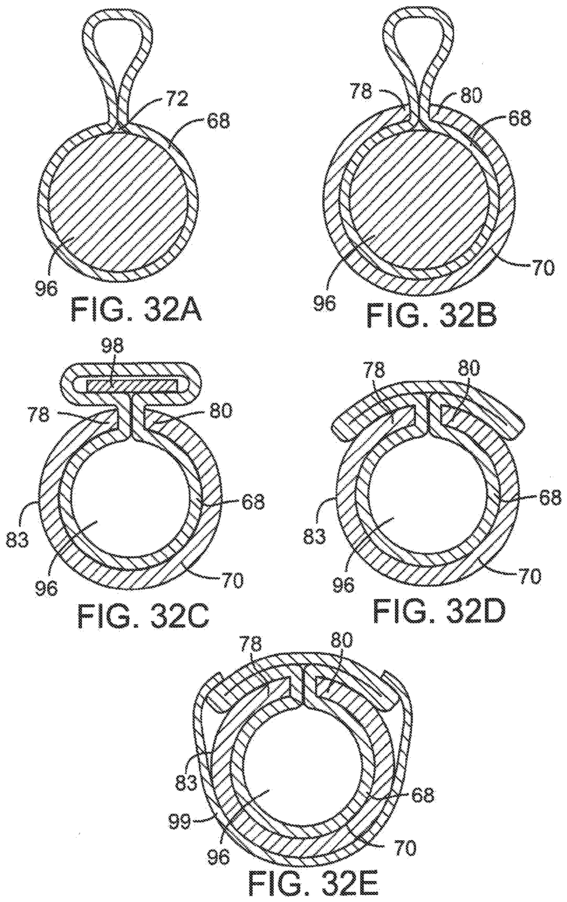

**FIG. 30** illustrates a block diagram of one method of producing a sheath for use with a delivery apparatus in minimally invasive surgery. One or more mandrels can be provided (step **300**). The mandrel can be provided with an exterior coating, such as a Teflon^{®} coating, and the mandrel's diameter can be predetermined based on the desired size of the resulting sheath. A liner that will become the inner polymeric layer of the sheath, such as a PTFE or high density polyethylene liner, can be mounted on the mandrel (step **302**). The liner can be etched and/or surface treated prior to being mounted on the mandrel, according to conventional etching and surface treatment methods. **FIG. 32A** illustrates a section view of a sheath at steps **300** and **302 of** **FIG. 30****.** A coated mandrel **96** is inserted within the lumen **72** of the inner polymeric layer **68.** The circumference of the inner polymeric layer **68** is larger than the circumference of the mandrel **96,** such that an excess portion of the inner polymeric layer **68** can be gathered above the mandrel **96.**

**A** layer of material that will become the outer polymeric tubular layer, such as a layer comprising polyurethane or polyolefin, can be cut or notched through all, substantially all, or a part of the thickness of the layer (step **304**). Such a cut or notch can extend longitudinally along the length of the layer and can extend along substantially the entire length of the outer polymeric tubular layer. In alternative aspects, the cut or notch can be provided along only a portion of the outer polymeric tubular layer. For example, the outer polymeric tubular layer can be cut starting at the distal end of the outer polymeric tubular layer, with the cut ending before the proximal end of the outer polymeric tubular layer. In one aspect, the cut can end at a transition, where the outer diameter of the outer polymeric tubular layer increases or decreases. In one specific aspect, the cut or notch can extend longitudinally along about 75% of the length of the sheath.

**The** cut or notched outer polymeric tubular layer can be applied, positioned, adhered, mounted, thermally fused or bonded, dip coated, and/or otherwise coupled to the etched inner liner (step **306**). **FIG. 32B** shows a section view of the sheath at step **306** of **FIG. 30****,** with outer polymeric tubular layer **70** applied to the inner polymeric layer **68** such that a portion of the inner polymeric layer **68** extends between the cut formed between first and second portions **78, 80** of the outer polymeric tubular layer **70.**

In alternative aspects, the outer polymeric tubular layer can be notched or cut after being mounted on the inner liner/mandrel assembly. The outer polymeric tubular layer can optionally be provided with a hydrophilic coating and/or provided with additional layers, such as being dip coated with polyurethane. Some portion of the inner liner can protrude through the cut in the outer polymeric tubular layer after an outer polymeric tubular layer is mounted onto the inner liner/mandrel arrangement. Using, for example, a split tool, the protruding portion of the inner liner can be folded down onto the outer surface of the outer polymeric tubular layer (step **308**)**.** In some aspects, the protruding portion of the inner liner is folded down along the entire length of the resulting sheath, while in other aspects, the protruding portion of the inner liner is only present along a portion of the length of the sheath or is only folded down along a portion of the length of the resulting sheath. **FIG. 32C** shows a section view of the sheath at step **308** of **FIG. 30****.** A split tool **98** is used to fold the excess portion of inner polymeric layer **68** over a portion of the outer surface **83** of the outer polymeric tubular layer **70.** **FIG. 32D** shows a section view of the sheath after completion of step **308 of** **FIG. 30****.** Split tool **98** has been removed, and folding of the excess portion of the inner polymeric layer **68** has been completed. **FIG. 32E** shows a section view of an outer covering, such as outer polymeric covering **99,** that can be applied such that it overlaps a portion of the folded portion of inner polymeric layer **68**. The outer polymeric covering **99** contacts at least a portion of the outer surface **83** of the outer polymeric tubular layer **70.**

**A** soft, atraumatic tip can be provided at the distal end of the resulting sheath (step **310**). Additional outer layers can also be applied if desired. Then, a layer of heat shrink tubing, such as fluorinated ethylene propylene (FEP) heat shrink tubing, can be positioned over the entire assembly (step **312**). An appropriate amount of heat is applied, thus shrinking the heat shrink tubing and compressing the layers of the sheath together, such that components of the sheath can be thermally bonded or fused together where desired. Once the components of the sheath have been bonded together, the heat shrink tubing can be removed (step **314**). Finally, the proximal end of the sheath can be adhered or otherwise attached to a housing of a catheter assembly, and the sheath can be removed from the mandrel (step **316**).

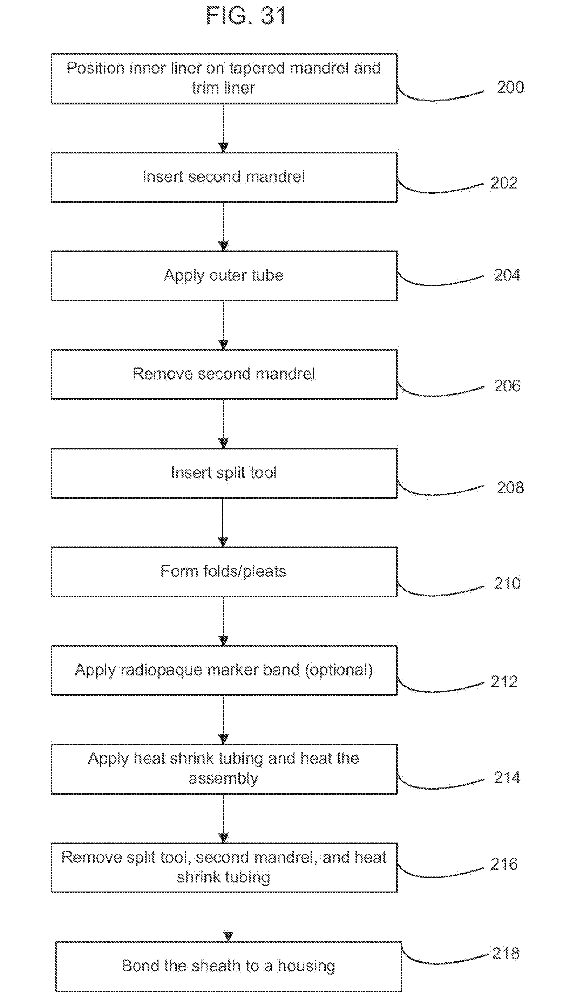

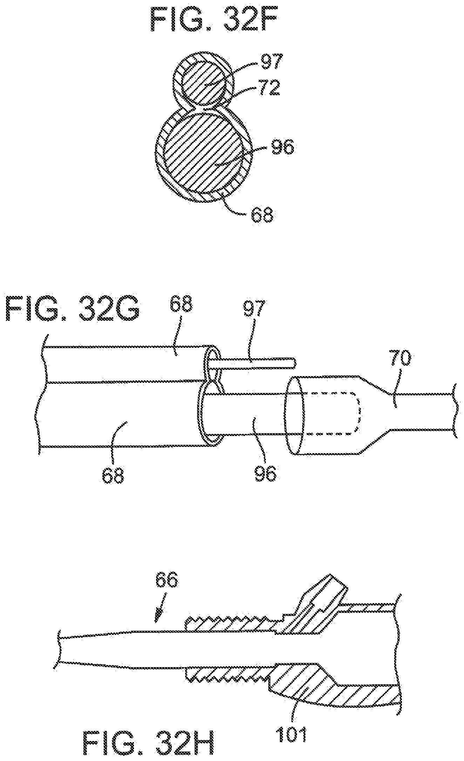

**FIG. 31** illustrates a block diagram of an alternative aspect of a method of making a sheath. An inner liner, such as an etched PTFE tubing, can be applied to a tapered mandrel, such as a 16 Fr tapered mandrel, and trimmed to an appropriate length (step **200**). A second mandrel, such as a 0.070 inches diameter mandrel, can be inserted in the lumen of the inner liner such that the mandrels are arranged side by side in the inner liner (step **202**). **FIG. 32F** shows a section view of a sheath at steps **200** and **202** of **FIG. 31****.** An inner liner or inner polymeric layer **68** is applied on a first tapered mandrel **96**. A second mandrel **97** is inserted into the lumen **72** of the inner polymeric layer **68** created by the excess portion of the inner polymeric layer **68,** as described.

A notched or cut outer polymeric tubular layer, such as high density polyethylene tubing that has been notched or cut longitudinally, can be slid onto the tapered mandrel and a portion of the inner liner, starting at the distal end of the tapered mandrel (step **204**). The second mandrel can then be removed (step **206**). **FIG. 32G** illustrates a perspective view of the sheath at steps **204** and **206** of **FIG. 31****.** A polymeric outer tubular layer **70** having a longitudinal cut is applied over the tapered mandrel 96 and inner polymeric layer **68.** The outer tubular layer conforms to the portion of the inner polymeric layer around the tapered mandrel **96,** and the portion of the inner polymeric layer **68** around the second mandrel **97** extends through the longitudinal cut in the outer polymeric tubular layer **70.**

**A** split tool can be inserted into the portion of the lumen of the inner liner that was previously occupied by the second mandrel (step **208**). The split tool can then be used to form folds and/or pleats in the excess portion of the inner liner, which now extends through the longitudinal cut in the outer polymeric tubular layer (step **210**). A radiopaque marker band can optionally be applied at the distal end of the sheath (step **212**). Heat shrink tubing, such as FEP heat shrink tubing, can be applied over the entire sheath, and heat can be applied to compress the components of the sheath and bond or fuse them together (step **214**). The split tool, heat shrink tubing, and second mandrel can then be removed (step **216**). The sheath can then be utilized with a delivery apparatus, such as by bonding the proximal end of the sheath to a polycarbonate housing of a delivery apparatus or catheter assembly (step **218**).

**FIG. 32H** illustrates an elevation view of the sheath at step **218** of **FIG. 31****.** The sheath **66,** made according to described methods and processes, can be attached or bonded to a housing **101,** such as by bonding the proximal end of the sheath **66** to the polycarbonate housing **101.**

In another example, disclosed expandable sheaths can be made using a reflowed mandrel process. A mandrel can be provided, with the size of the mandrel defining the inner diameter of the sheath lumen in its resting configuration. A tube of material, such as a PTFE tube that will become the sheath's inner liner, can be provided with an inner diameter greater than that of the mandrel (*e.g*., a 9 mm PTFE tube can be mounted on a 6 mm mandrel). The PTFE tube can be mounted on the mandrel and prepared into the final folded configuration by folding the excess material of the PTFE tube over to one or both sides. An HDPE tube that will serve as the outer layer can then be placed over the PTFE liner. The two-layer assembly can then be thermally fused together. For example, a reflow process can be performed where the assembly is heated to a temperature high enough such that the inner and/or outer layers are at least partially melted and are then fused together as the heat is removed and the assembly cools.

**An** elastic cover can be placed over at least part of the fused layers (e.g., over a proximal section of the sheath) and held in place using a thermal process. In some aspects, the same thermal process can bond the layers of the sheath and the elastic cover. In other aspects, a first thermal process can be used to fuse the layers of the sheath, and a second thermal process can be used to secure the elastic cover to the sheath. In some aspects, the elastic cover can be heat shrink tubing that is applied over the expandable sheath and heated to a temperature high enough to cause the tubing to shrink around the sheath. In some aspects, a distal soft tip can then be attached to the shaft of the expandable sheath.

In some aspects, the outer layer can be co-extruded with an adhesive layer, such as a layer formed from Tecoflex^{™}, such that the Tecoflex^{™} is positioned on an inner surface of the outer layer - in this manner, the Tecoflex^{™} will be positioned between the inner and outer layers in the completed sheath. In these aspects, an HOPE tube can be provided with a coating of Tecoflex^{™} on the inner surface. The HOPE tube can be slit along the length of the tube to open and flatten it and then cut using a template in some aspects. For example, for specific applications, portions of the outer layer can be cut and removed using a template. The cut HDPE can then be placed on the inner liner on the mandrel. In some aspects, only a portion of the outer layer will have the adhesive Tecoflex^{™}. In these aspects, the sections without Tecoflex^{™} will only be partially fused to the inner liner. In some aspects, the entire inner surface of the outer layer will have the Tecoflex^{™}, and the inner surface of the outer layer can be positioned so that it contacts the inner liner on the mandrel. To position the inner and outer layers, as shown in the sheath of **FIG. 39****,** the folded portion of the inner liner can be lifted up, and an edge of the outer layer can be tucked beneath the fold.

Sheaths of the present disclosure can be used with various methods of introducing a prosthetic device into a patient's vasculature. One such method comprises positioning an expandable sheath in a patient's vessel, passing a device through the introducer sheath, which causes a portion of the sheath surrounding the device to expand and accommodate the profile of the device, and automatically retracting the expanded portion of the sheath to its original size after the device has passed through the expanded portion. In some methods, the expandable sheath can be sutured to the patient's skin at the insertion site so that once the sheath is inserted the proper distance within the patient's vasculature, it does not move once the implantable device starts to travel through the sheath.

Disclosed aspects of an expandable sheath can be used with other delivery and minimally invasive surgical components, such as an introducer and loader. In one aspect, the expandable sheath can be flushed to purge any air within the sheath, using, for example, flush port **103** (**FIG. 35**). An introducer can be inserted into the expandable sheath, and the introducer/sheath combination can be fully inserted into vasculature over a guiding device, such as a 0.35" guidewire. Preferably, the seam formed by the intersection of the folded portion of the inner liner and the overlapping portion of the outer layer can be positioned such it is oriented downward (posterior). Once the sheath and introducer are fully inserted into a patient's vasculature, in some aspects, the expandable sheath can be sutured in place at the insertion site. In this manner, the expandable sheath can be substantially prevented from moving once positioned within the patient.

The introducer can then be removed, and a medical device, such as a transcatheter heart valve, can be inserted into the sheath, in some instances, using a loader. Such methods can additionally comprise placing the tissue heart valve in a crimped state on the distal end portion of an elongated delivery apparatus and inserting the elongated delivery device with the crimped valve into and through the expandable sheath. Next, the delivery apparatus can be advanced through the patient's vasculature to the treatment site, where the valve can be implanted.

Typically, the medical device has a greater outer diameter than the diameter of the sheath in its original configuration. The medical device can be advanced through the expandable sheath towards the implantation site, and the expandable sheath can locally expand to accommodate the medical device as the device passes through. The radial force exerted by the medical device can be sufficient to locally expand the sheath to an expanded diameter (*e.g.,* the expanded configuration) just in the area where the medical device is currently located. Once the medical device passes a particular location of the sheath, the sheath can at least partially contract to the smaller diameter of its original configuration. The expandable sheath can thus be expanded without the use of inflatable balloons or other dilators. Once the medical device is implanted, the sheath and any sutures holding in place can be removed. In some aspects, it is preferable to remove the sheath without rotating it.

In view of the many possible aspects to which the principles of the disclosed disclosure can be applied, it should be recognized that the illustrated aspects are only preferred examples of the disclosure and should not be taken as limiting the scope of the disclosure. Rather, the scope of the disclosure is defined by the following claims. We, therefore, claim as our disclosure all that comes within the scope of these claims.

## Claims

1. A sheath (100) for delivering a medical device, wherein the sheath (100) has a proximal and a distal end (104) and comprises:
an expandable inner liner (108) having an inner surface and an outer surface, wherein the inner surface of the expandable inner liner (108) defines a lumen (116) and wherein the inner liner (108) comprises at least one folded portion (118) having an inner portion and outer portion;
an outer layer (110) having an inner surface and an outer surface and extending at least partially around the inner liner (108) such that a first portion (122) of the outer surface of the outer layer (110) is positioned adjacent to the inner portion of the at least one folded portion (118) of the inner liner (108), while a first portion (120) of the inner surface (130) of the outer layer (110) is positioned adjacent to the outer portion of the at least one folded portion (118) of the inner liner (108); and
wherein the inner liner (108) comprises at least one first portion (6102) and at least one second portion (6104), wherein an outer surface of the at least one first portion (6102) comprises a composition and/or morphology that is substantially different from an outer surface of the at least one second portion (6104); and wherein
an outwardly directed radial force from a prosthetic device moving through the inner lumen (116) unfolds the at least one folded portion (118) to allow expansion of the sheath (100).

2. The sheath (100) of claim 1, wherein the at least one first portion (6102) of the inner liner (108) is etched;
preferably wherein the at least one second portion (6104) is etched and subsequently surface-modified such that a composition of the outer surface of the at least one second portion (6104) is substantially different from a composition of the outer surface of the at least one first portion (6102).

3. The sheath (100) of claim 2, wherein the inner liner (108) comprises a fluorocarbon-based polymer;
preferably wherein the outer surface of the at least one first portion (6102) comprises a fluorine-to-carbon ratio that is lower than a fluorine-to-carbon ratio present on the outer surface of the at least one second portion (6104) of the inner liner (108).

4. The sheath (100) of any one of the preceding claims, wherein the at least one second portion (6104) extends longitudinally along a length of the inner liner (108) and/or the at least one second portion (6104) extends circumferentially around a circumference of the inner liner (108).

5. The sheath (100) of any one of the preceding claims, wherein the inner liner (108) comprises two or more first portions (6102) and/or the inner liner (108) comprises two or more second portions (6104);
preferably wherein each of the two or more second portions 6104), if present, comprises the same or different composition and/or morphology; and/or wherein each of the two or more second portions (6104) has a length that is the same or different.

6. The sheath (100) of any one of the preceding claims, wherein a length of the at least one first portion (6102) is greater than a length of the at least one second portion (6104);
preferably wherein a wall thickness of the at least one second portion (6104) is up to about 2% larger than a wall thickness of the at least one first portion (6102); or alternatively
wherein a wall thickness of the at least one second portion (6104) is up to about 2% smaller than a wall of the at least one first portion (6102).

7. The sheath (100) of any one of the preceding claims, wherein the at least one second portion (6104) of the inner liner (108) extends along the inner portion and/or the outer portion of the at least one folded portion (118) of the inner liner (108); and/or
wherein the at least one second portion (6104) of the inner liner (108) extends along portions that contact the outer surface of the outer layer (110); and/or
wherein the outer layer (110) comprises high-density polyethylene polymer (HDPE), nylon, or polypropylene; and/or
wherein the at least one second portion (6104) of the inner liner (108) exhibits substantially less stickiness to the outer layer (110) as compared to the stickiness of the at least one first portion (6102) of the inner liner (108) to the outer layer (110); and/or
wherein the outer surface of the at least one second portion (6104) exhibits a contact angle from greater than about 65° to about 140° and/or wherein the outer surface of the at least one first portion (6102) exhibits a contact angle from about 30° to about 90°; and/or
wherein the inner liner (108) comprises the at least one second portion (6104) at the proximal end (106) of the sheath (100).

8. The sheath (100) of any one of the preceding claims, wherein the at least one second portion (6104) of the inner liner (108) is separated from the proximal end (106) of the sheath (100) by the at least one first portion (6102) of the inner liner (108) such that the length of the at least one first portion (6102) is substantially similar or shorter than the length of the at least one second portion (6104);
preferably wherein the at least one second portion (6104) of the inner liner (108) is followed by an additional first portion (6102) of the inner liner (108);
more preferably wherein the length of the at least one second portion (6104) of the inner liner (108) is shorter than a length of the additional first portion (6102) of the inner liner (108).

9. The sheath (100) of any one of the preceding claims, wherein the sheath (100) further comprises an outer jacket (140) comprising an inner surface and outer surface and a proximal end and a distal end and extending at least partially around the outer layer (110) such that the inner surface of the outer jacket (140) overlies the outer surface of the outer layer (110) and wherein the outer jacket (140) has a first predetermined length;
preferably wherein the at least one second portion (6104) extends from the proximal end (106) of the sheath (100), and wherein the length of the at least one second portion (6104) is substantially similar to the first predetermined length of the outer jacket (140); or alternatively
wherein the at least one second portion (6104) extends from the proximal end (106) of the sheath (100), and wherein the length of the at least one second portion (6104) is larger than the first predetermined length of the outer jacket (140); or alternatively
wherein the at least one second portion (6104) of the inner liner (108) is separated from the proximal end (106) of the sheath (100) by the at least one first portion (6102), and wherein a total length of the at least one first portion (6102) of the inner liner (108) and the at least one second portion (6104) of the inner liner (108) is substantially similar to the first predetermined length of the outer jacket (140).

10. The sheath (100) of claim 9, wherein the distal end of the outer jacket (140) is substantially sealed with the outer layer (110) of the sheath (100); preferably wherein the outer jacket (140) extends from the proximal end (106) of the sheath (100) to the distal end (104) of the sheath (100);
more preferably wherein the at least one second portion (6104) of the inner liner (108) extends along the inner portion and/or the outer portion of the at least one folded portion (118) of the inner liner (108); and/or
wherein the at least one second portion (6104) of the inner liner (108) extends along portions of the inner liner (108) that contact the outer surface of the outer layer (110).

11. The sheath (100) of any one of the preceding claims, wherein the inner liner (108) comprises two or more folded portions (118);
preferably wherein the at least one second portion (6104) of the inner liner (108) extends along the inner portion and/or the outer portion of each of the two or more folded portions (118).

12. A method of manufacturing a sheath (100) for delivering a medical device, the method comprising:
providing an inner liner (108) having an inner surface and an outer surface, wherein the inner surface defines a lumen (116) therethrough, wherein the inner liner (108) comprises at least one first portion (6102) and at least one second portion (6104), wherein an outer surface of the at least one first portion (6102) comprises a composition and/or morphology that is substantially different from an outer surface of the at least one second portion (6104); and wherein the inner liner (108) comprises at least one folded portion (118) having an inner portion and outer portion;
providing an outer layer (110) having an inner surface and an outer surface such that it extends at least partially around the inner liner (108) such that at least a first portion of the outer surface of the outer layer (110) is positioned adjacent to the inner portion of the at least one folded portion (118) of the inner liner (108), while a first portion of the inner surface of the outer layer (110) is positioned adjacent to the outer portion of the at least one folded portion (118) of the inner liner (108); and
forming an expandable sheath (100) that is configured to expand when an outwardly directed radial force from a prosthetic device moving through the inner lumen (116) unfolds the at least one folded portion (118).

13. The method of claim 12, wherein the at least one first portion (6102) and at least one second portion (6104) are formed by a selective etching.

14. The method of claim 13, wherein the at least one first portion (6102) is formed by masking a portion of the inner liner (108) to form a masked portion and then etching remaining unmasked portions of the inner liner (108) with an etchant;
preferably further comprising a step of forming the at least one second portion (6104) by unmasking the masked portion after the etching.

15. The method of claim 14, wherein the etchant comprises a liquid, a fluid, a gas, a plasma, or a combination thereof;
preferably wherein the at least one first portion (6102) is formed by etching at least a portion of the inner liner (108) to form an etched inner liner (108);
more preferably further comprising a step of ablating at least a portion of the etched inner liner (108), thereby forming the at least one second portion (6104) of the inner liner (108);
more preferably wherein the step of ablating comprises forming a gradient having a predetermined pattern (6106).

## Patentansprüche

1. Hülle (100) zum Zuführen einer medizinischen Vorrichtung, wobei die Hülle (100) ein proximales und ein distales Ende (104) aufweist und umfasst:
eine expandierbare Innenauskleidung (108) mit einer inneren Oberfläche und einer äußeren Oberfläche, wobei die innere Oberfläche der expandierbaren Innenauskleidung (108) ein Lumen (116) definiert und wobei die Innenauskleidung (108) mindestens einen gefalteten Abschnitt (118) mit einem inneren Abschnitt und einem äußeren Abschnitt umfasst;
eine äußere Schicht (110), die eine innere Oberfläche und eine äußere Oberfläche aufweist und sich zumindest teilweise um die Innenauskleidung (108) herum erstreckt, so dass ein erster Abschnitt (122) der äußeren Oberfläche der äußeren Schicht (110) benachbart zu dem inneren Abschnitt des mindestens einen gefalteten Abschnitts (118) der Innenauskleidung (108) positioniert ist, während ein erster Abschnitt (120) der inneren Oberfläche (130) der äußeren Schicht (110) benachbart zu dem äußeren Abschnitt des mindestens einen gefalteten Abschnitts (118) der Innenauskleidung (108) positioniert ist; und
wobei die Innenauskleidung (108) zumindest einen ersten Abschnitt (6102) und zumindest einen zweiten Abschnitt (6104) umfasst, wobei eine äußere Oberfläche des zumindest einen ersten Abschnitts (6102) eine Zusammensetzung und/oder Morphologie umfasst, die wesentlich verschieden von einer äußeren Oberfläche des zumindest einen zweiten Abschnitts (6104) ist; und wobei
eine nach außen gerichtete Radialkraft von einer prothetischen Vorrichtung, die sich durch das innere Lumen (116) bewegt, den zumindest einen gefalteten Abschnitt (118) entfaltet, um eine Expansion der Hülle (100) zu ermöglichen.

2. Hülle (100) gemäß Anspruch 1, wobei der zumindest eine erste Abschnitt (6102) der Innenauskleidung (108) geätzt ist;
wobei, vorzugsweise, der zumindest eine zweite Abschnitt (6104) geätzt ist und anschließend oberflächenmodifiziert ist, so dass die Zusammensetzung der äußeren Oberfläche des zumindest einen zweiten Abschnitts (6104) wesentlich verschieden ist von der Zusammensetzung der äußeren Oberfläche des zumindest einen ersten Abschnitts (6102).

3. Hülle (100) gemäß Anspruch 2, wobei die Innenauskleidung (108) ein Polymer auf Fluorkohlenstoffbasis umfasst;
wobei, vorzugsweise, die äußere Oberfläche des zumindest einen ersten Abschnitts (6102) ein Fluor-Kohlenstoff-Verhältnis aufweist, das niedriger ist als das Fluor-Kohlenstoff-Verhältnis, das auf der äußeren Oberfläche des zumindest einen zweiten Abschnitts (6104) der Innenauskleidung (108) vorhanden ist.

4. Hülle (100) gemäß einem der vorangehenden Ansprüche, wobei der zumindest eine zweite Abschnitt (6104) sich longitudinal entlang einer Länge der Innenauskleidung (108) erstreckt und/oder der zumindest eine zweite Abschnitt (6104) sich in Umfangsrichtung um den Umfang der Innenauskleidung (108) herum erstreckt.

5. Hülle (100) gemäß einem der vorangehenden Ansprüche, wobei die Innenauskleidung (108) zwei oder mehrere erste Abschnitte (6102) umfasst und/oder die Innenauskleidung (108) zwei oder mehrere zweite Abschnitte (6104) umfasst;
wobei, vorzugsweise, jede der zwei oder mehreren zweiten Abschnitte (6104), wenn vorhanden, dieselbe oder verschiedene Zusammensetzung und/oder Morphologie umfasst;
und/oder wobei jede der zwei oder mehreren zweiten Abschnitte (6104) eine gleiche oder unterschiedliche Länge aufweist.

6. Hülle (100) gemäß einem der vorangehenden Ansprüche, wobei eine Länge des zumindest einen ersten Abschnitts (6102) größer ist, als eine Länge des zumindest einen zweiten Abschnitts (6104);
wobei, vorzugsweise, eine Wandstärke des zumindest einen zweiten Abschnitts (6104) bis zu 2% größer ist als die Wandstärke des zumindest einen ersten Abschnitts (6102); oder alternativ
wobei eine Wandstärke des zumindest einen zweiten Abschnitts (6104) bis zu 2% kleiner ist als die Wandstärke des zumindest einen ersten Abschnitts (6102).

7. Hülle (100) gemäß einem der vorangehenden Ansprüche, wobei der zumindest eine zweite Abschnitt (6104) der Innenauskleidung (108) sich entlang des inneren Abschnitts und/oder des äußeren Abschnitts des zumindest einen gefalteten Abschnitts (118) der Innenauskleidung (108) erstreckt; und/oder
wobei der zumindest eine zweite Abschnitt (6104) der Innenauskleidung (108) sich entlang von Abschnitten erstreckt, die die äußere Oberfläche der äußeren Schicht (110) berühren; und/oder
wobei die äußere Schicht (110) ein Polyethylen Polymer von hoher Dichte (PE-HD), Nylon oder Polypropylen umfasst; und/oder
wobei der zumindest eine zweite Abschnitt (6104) der Innenauskleidung (108) eine wesentlich geringere Klebrigkeit zu der äußeren Schicht (110) aufweist, verglichen mit der Klebrigkeit des zumindest einen ersten Abschnitts (6102) der Innenauskleidung (108) zur äußeren Schicht (110); und/oder
wobei die äußere Oberfläche des zumindest einen zweiten Abschnitts (6104) einen Kontaktwinkel größer als ca. 65° bis ca. 140° aufweist und/oder wobei die äußere Oberfläche des zumindest einen ersten Abschnitts (6102) einen Kontaktwinkel von ca. 30° bis ca. 90° aufweist; und/oder
wobei die Innenauskleidung (108) den zumindest einen zweiten Abschnitt (6104) an dem proximalen Ende (106) der Hülle (100) umfasst.

8. Hülle (100) gemäß einem der vorangehenden Ansprüche, wobei der zumindest eine zweite Abschnitt (6104) der Innenauskleidung (108) von dem proximalen Ende (106) der Hülle (100) durch den zumindest einen ersten Abschnitt (6102) der Innenauskleidung (108) getrennt ist, so dass die Länge des zumindest einen ersten Abschnitts (6102) im Wesentlichen ähnlich oder kürzer als die Länge des zumindest einen zweiten Abschnitts (6104) ist;
wobei, vorzugsweise, der zumindest eine zweite Abschnitt (6104) der Innenauskleidung (108) gefolgt ist von einem zusätzlichen ersten Abschnitt (6102) der Innenauskleidung;
wobei, besonders bevorzugt, die Länge des zumindest einen zweiten Abschnitts (6104) der Innenauskleidung (108) kürzer als die Länge des zusätzlichen ersten Abschnitts (6102) der Innenauskleidung (108) ist.

9. Hülle (100) gemäß einem der vorangehenden Ansprüche, wobei die Hülle (100) ferner einen äußeren Mantel (140) umfasst, der eine innere Oberfläche und eine äußere Oberfläche sowie ein proximales Ende und ein distales Ende umfasst und sich zumindest teilweise um die äußere Schicht (110) herum erstreckt, so dass die innere Oberfläche des äußeren Mantels (140) die äußere Oberfläche der äußeren Schicht (110) überdeckt, und wobei der äußere Mantel (140) eine erste vorbestimme Länge aufweist;
wobei sich, vorzugsweise, der zumindest eine zweite Abschnitt (6104) von dem proximalen Ende (106) der Hülle (100) erstreckt, und wobei die Länge des zumindest einen zweiten Abschnitts (6104) im Wesentlichen ähnlich zu der ersten vorbestimmten Länge des äußeren Mantels (140) ist; oder
wobei sich alternativ der zumindest eine zweite Abschnitt (6104) von dem proximalen Ende (106) der Hülle (100) erstreckt, und wobei die Länge des zumindest einen zweiten Abschnitts (6104) größer als die erste vorbestimmte Länge des äußeren Mantels (140) ist; oder
wobei alternativ der zumindest eine zweite Abschnitt (6104) der Innenauskleidung (108) von dem proximalen Ende (106) der Hülle (100) durch den zumindest einen ersten Abschnitt (6102) getrennt ist, und wobei eine Gesamtlänge des zumindest einen ersten Abschnitts (6102) der Innenauskleidung (108) und des zumindest einen zweiten Abschnitts (6104) der Innenauskleidung (108) im Wesentlichen ähnlich zu der ersten vorbestimmten Länge des äußeren Mantels (140) ist.

10. Hülle (100) gemäß Anspruch 9, wobei das distale Ende des äußeren Mantels (140) im Wesentlichen durch die äußere Schicht (110) der Hülle (100) versiegelt ist;
wobei sich, vorzugsweise, der äußere Mantel (140) von dem proximalen Ende (106) der Hülle (106) zu dem distalen Ende (104) der Hülle (100) erstreckt;
wobei sich, noch bevorzugter, der zumindest eine zweite Abschnitt (6104) der Innenauskleidung (108) entlang des inneren Abschnitts und/oder des äußeren Abschnitts des zumindest einen gefalteten Abschnitts (118) der Innenauskleidung (108) erstreckt; und/oder
wobei sich der zumindest eine zweite Abschnitt (6104) der Innenauskleidung (108) entlang von Abschnitten der Innenauskleidung (108), die mit der äußeren Oberfläche der äußeren Schicht (110) im Kontakt stehen, erstreckt.

11. Hülle (100) gemäß einem der vorangehenden Ansprüche,
wobei die Innenauskleidung (108) zwei oder mehrere gefaltete Abschnitte (118) umfasst,
wobei sich, vorzugsweise, der zumindest eine zweite Abschnitt (6104) der Innenauskleidung (108) entlang des inneren Abschnitts und/oder des äußeren Abschnitts jedes der zwei oder mehreren gefalteten Abschnitte (118) erstreckt.

12. Verfahren zur Herstellung einer Hülle (100) zum Zuführen einer medizinischen Vorrichtung, wobei das Verfahren umfasst,
Bereitstellen einer Innenauskleidung (108) die eine innere Oberfläche und eine äußere Oberfläche aufweist, wobei die innere Oberfläche ein Lumen (116) durch diese hindurch definiert, wobei die Innenauskleidung (108) zumindest einen ersten Abschnitt (6102) und zumindest einen zweiten Abschnitt (6104) umfasst, wobei eine äußere Oberfläche des zumindest einen ersten Abschnitts (6102) eine Zusammensetzung und/oder Morphologie umfasst, die im Wesentlichen verschieden von der äußeren Oberfläche des zumindest einen zweiten Abschnitts (6104) ist; und wobei die Innenauskleidung (108) zumindest einen gefalteten Abschnitt (118) mit einem inneren und einem äußeren Abschnitt aufweist;
Bereitstellen einer äußeren Schicht (110) mit einer inneren Oberfläche und einer äußeren Oberfläche, so dass sich diese zumindest teilweise um die Innenauskleidung (108) herum erstreckt, so dass zumindest ein erster Abschnitt der äußeren Oberfläche der äußeren Schicht (110) benachbart zu dem inneren Abschnitt des zumindest einen gefalteten Abschnitts (118) der Innenauskleidung (108) positioniert ist, während ein erster Abschnitt der inneren Oberfläche der äußeren Schicht (110) benachbart zu dem äußeren Abschnitt des zumindest einen gefalteten Abschnitts (118) der Innenauskleidung (108) positioniert ist; und
Bilden einer expandierbaren Hülle (100), die dazu ausgelegt ist, sich auszudehnen, wenn eine nach außen gerichtete Radialkraft von einer prothetischen Vorrichtung, die sich durch das innere Lumen (116) bewegt, den zumindest einen gefalteten Abschnitt 118) entfaltet.

13. Verfahren gemäß Anspruch 12, wobei der zumindest eine erste Abschnitt (6102) und der zumindest eine zweite Abschnitt (6104) durch selektives Ätzen gebildet werden.

14. Verfahren gemäß Anspruch 13, wobei der zumindest eine erste Abschnitt (6102) durch Maskieren eines Abschnitts der Innenauskleidung (108), um einen maskierten Abschnitt zu bilden, und anschließendem Ätzen der verbleibenden unmaskierten Bereiche der Innenauskleidung (108) mit einem Ätzmittel gebildet wird;
ferner vorzugsweise umfassend einen Schritt des Bildens des zumindest einen zweiten Abschnitts (6104) durch Demaskieren des maskierten Abschnitts nach dem Ätzen.

15. Verfahren gemäß Anspruch 14, wobei das Ätzmittel eine Flüssigkeit, ein Fluid, ein Gas, ein Plasma oder eine Kombination davon umfasst;
wobei, vorzugsweise, der zumindest eine erste Abschnitt (6102) durch Ätzen zumindest eines Abschnitts der Innenauskleidung (108) gebildet wird, um eine geätzte Innenauskleidung (108) zu bilden;
ferner noch bevorzugter umfassend einen Schritt des Abtragens der geätzten Innenauskleidung (108), wodurch der zumindest eine zweite Abschnitt (6104) der Innenauskleidung (108) gebildet wird;
wobei noch bevorzugter der Schritt des Abtragens das Bilden eines Gradienten mit einem vorbestimmten Muster (6106) umfasst.

## Revendications

1. Gaine (100) destinée à poser un dispositif médical, la gaine (100) présentant une extrémité proximale et une extrémité distale (104) et comprenant :
une doublure interne extensible (108) présentant une surface interne et une surface externe, la surface interne de la doublure interne extensible (108) définissant une lumière (116) et la doublure interne (108) comprenant au moins une partie pliée (118) présentant une partie interne et une partie externe ;
une couche externe (110) présentant une surface interne et une surface externe et s'étendant au moins partiellement autour de la doublure interne (108) de telle sorte qu'une première partie (122) de la surface externe de la couche externe (110) est positionnée de manière adjacente à la partie interne de ladite au moins une partie pliée (118) de la doublure interne (108), tandis qu'une première partie (120) de la surface interne (130) de la couche externe (110) est positionnée de manière adjacente à la partie externe de ladite au moins une partie pliée (118) de la doublure interne (108) ; et
la doublure interne (108) comprenant au moins une première partie (6102) et au moins une seconde partie (6104), une surface externe de ladite au moins une première partie (6102) comprenant une composition et/ou une morphologie qui est sensiblement différente de celle d'une surface externe de ladite au moins une seconde partie (6104) ; et
une force radiale dirigée vers l'extérieur provenant d'un dispositif prothétique se déplaçant à travers la lumière interne (116) dépliant ladite au moins une partie pliée (118) afin de permettre l'extension de la gaine (100).

2. Gaine (100) selon la revendication 1, ladite au moins une première partie (6102) de la doublure interne (108) étant gravée ;
de préférence, ladite au moins une seconde partie (6104) étant gravée et ensuite modifiée en surface de telle sorte qu'une composition de la surface externe de ladite au moins une seconde partie (6104) est sensiblement différente d'une composition de la surface externe de ladite au moins une première partie (6102).

3. Gaine (100) selon la revendication 2, la doublure interne (108) comprenant un polymère à base de fluorocarbone ;
de préférence, la surface externe de ladite au moins une première partie (6102) présentant un rapport fluor à carbone qui est inférieur à un rapport fluor à carbone présent sur la surface externe de ladite au moins une seconde partie (6104) de la doublure interne (108).

4. Gaine (100) selon l'une quelconque des revendications précédentes, ladite au moins une seconde partie (6104) s'étendant longitudinalement le long d'une longueur de la doublure interne (108) et/ou ladite au moins une seconde partie (6104) s'étendant de manière circonférentielle autour d'une circonférence de la doublure interne (108).

5. Gaine (100) selon l'une quelconque des revendications précédentes, la doublure interne (108) comprenant deux premières parties (6102) ou plus et/ou la doublure interne (108) comprenant deux secondes parties (6104) ou plus ;
de préférence, chacune des deux secondes parties (6104) ou plus, si elles sont présentes, comprenant une composition et/ou morphologie identique ou différente ;
et/ou chacune des deux secondes parties (6104) ou plus présentant une longueur qui est identique ou différente.

6. Gaine (100) selon l'une quelconque des revendications précédentes, une longueur de ladite au moins une première partie (6102) étant supérieure à une longueur de ladite au moins une seconde partie (6104) ;
de préférence, une épaisseur de paroi de ladite au moins une seconde partie (6104) étant jusqu'à environ 2 % plus grande qu'une épaisseur de paroi de ladite au moins une première partie (6102) ; ou en variante
une épaisseur de paroi de ladite au moins une seconde partie (6104) étant jusqu'à environ 2 % plus petite qu'une épaisseur de paroi de ladite au moins une première partie (6102).

7. Gaine (100) selon l'une quelconque des revendications précédentes, ladite au moins une seconde partie (6104) de la doublure interne (108) s'étendant le long de la partie interne et/ou de la partie externe de ladite au moins une partie pliée (118) de la doublure interne (108) ; et/ou
ladite au moins une seconde partie (6104) de la doublure interne (108) s'étendant le long de parties qui sont en contact avec la surface externe de la couche externe (110) ; et/ou
la couche externe (110) comprenant un polymère de polyéthylène haute densité (HDPE), du nylon ou du polypropylène ; et/ou
ladite au moins une seconde partie (6104) de la doublure interne (108) présentant sensiblement moins d'adhésivité à la couche externe (110) par rapport à l'adhésivité de ladite au moins une première partie (6102) de la doublure interne (108) à la couche externe (110) ; et/ou
la surface externe de ladite au moins une seconde partie (6104) présentant un angle de contact de plus d'environ 65° à environ 140° et/ou la surface externe de ladite au moins une première partie (6102) présentant un angle de contact d'environ 30° à environ 90° ; et/ou
la doublure interne (108) comprenant ladite au moins une seconde partie (6104) à l'extrémité proximale (106) de la gaine (100).

8. Gaine (100) selon l'une quelconque des revendications précédentes, ladite au moins une seconde partie (6104) de la doublure interne (108) étant séparée de l'extrémité proximale (106) de la gaine (100) par ladite au moins une première partie (6102) de la doublure interne (108) de telle sorte que la longueur de ladite au moins une première partie (6102) est sensiblement similaire à la longueur de ladite au moins une seconde partie (6104) ou plus courte que celle-ci ;
de préférence, ladite au moins une seconde partie (6104) de la doublure interne (108) étant suivie d'une première partie (6102) supplémentaire de la doublure interne (108) ;
plus préférablement, la longueur de ladite au moins une seconde partie (6104) de la doublure interne (108) étant plus courte qu'une longueur de la première partie (6102) supplémentaire de la doublure interne (108).

9. Gaine (100) selon l'une quelconque des revendications précédentes, la gaine (100) comprenant en outre une enveloppe externe (140) comprenant une surface interne et une surface externe et une extrémité proximale et une extrémité distale et s'étendant au moins partiellement autour de la couche externe (110) de telle sorte que la surface interne de l'enveloppe externe (140) recouvre la surface externe de la couche externe (110) et l'enveloppe externe (140) présentant une première longueur prédéterminée ;
de préférence, ladite au moins une seconde partie (6104) s'étendant à partir de l'extrémité proximale (106) de la gaine (100) et la longueur de ladite au moins une seconde partie (6104) étant sensiblement similaire à la première longueur prédéterminée de l'enveloppe externe (140) ; ou en variante
ladite au moins une seconde partie (6104) s'étendant à partir de l'extrémité proximale (106) de la gaine (100) et la longueur de ladite au moins une seconde partie (6104) étant plus grande que la première longueur prédéterminée de l'enveloppe externe (140) ; ou en variante
ladite au moins une seconde partie (6104) de la doublure interne (108) étant séparée de l'extrémité proximale (106) de la gaine (100) par ladite au moins une première partie (6102) et une longueur totale de ladite au moins une première partie (6102) de la doublure interne (108) et de ladite au moins une seconde partie (6104) de la doublure interne (108) étant sensiblement similaire à la première longueur prédéterminée de l'enveloppe externe (140).

10. Gaine (100) selon la revendication 9, l'extrémité distale de l'enveloppe externe (140) étant sensiblement fermée de manière étanche par la couche externe (110) de la gaine (100) ;
de préférence, l'enveloppe externe (140) s'étendant à partir de l'extrémité proximale (106) de la gaine (100) vers l'extrémité distale (104) de la gaine (100) ;
plus préférablement, ladite au moins une seconde partie (6104) de la doublure interne (108) s'étendant le long de la partie interne et/ou de la partie externe de ladite au moins une partie pliée (118) de la doublure interne (108) ; et/ou
ladite au moins une seconde partie (6104) de la doublure interne (108) s'étendant le long de parties de la doublure interne (108) qui sont en contact avec la surface externe de la couche externe (110).

11. Gaine (100) selon l'une quelconque des revendications précédentes, la doublure interne (108) comprenant deux parties pliées (118) ou plus ;
de préférence, ladite au moins une seconde partie (6104) de la doublure interne (108) s'étendant le long de la partie interne et/ou de la partie externe de chacune des deux parties pliées (118) ou plus.

12. Procédé de fabrication d'une gaine (100) pour la pose d'un dispositif médical, le procédé comprenant :
la fourniture d'une doublure interne (108) présentant une surface interne et une surface externe, la surface interne définissant une lumière (116) à travers celle-ci, la doublure interne (108) comprenant au moins une première partie (6102) et au moins une seconde partie (6104), une surface externe de ladite au moins une première partie (6102) comprenant une composition et/ou une morphologie qui est sensiblement différente de celle d'une surface externe de ladite au moins une seconde partie (6104) ; et la doublure interne (108) comprenant au moins une partie pliée (118) présentant une partie interne et une partie externe ;
la fourniture d'une couche externe (110) présentant une surface interne et une surface externe de telle sorte qu'elle s'étend au moins partiellement autour de la doublure interne (108) de telle sorte qu'au moins une première partie de la surface externe de la couche externe (110) est positionnée de manière adjacente à la partie interne de ladite au moins une partie pliée (118) de la doublure interne (108), tandis qu'une première partie de la surface interne de la couche externe (110) est positionnée de manière adjacente à la partie externe de ladite au moins une partie pliée (118) de la doublure interne (108) ; et
la formation d'une gaine extensible (100) qui est conçue pour s'étendre lorsqu'une force radiale dirigée vers l'extérieur provenant d'un dispositif prothétique se déplaçant à travers la lumière interne (116) déplie ladite au moins une partie pliée (118).

13. Procédé selon la revendication 12, ladite au moins une première partie (6102) et au moins une seconde partie (6104) étant formées par une gravure sélective.

14. Procédé selon la revendication 13, ladite au moins une première partie (6102) étant formée par masquage d'une partie de la doublure interne (108) afin de former une partie masquée, puis par gravure des parties non masquées restantes de la doublure interne (108) par un agent de gravure ;
de préférence, comprenant en outre une étape de formation de ladite au moins une seconde partie (6104) par démasquage de la partie masquée après la gravure.

15. Procédé selon la revendication 14, l'agent de gravure comprenant un liquide, un fluide, un gaz, un plasma ou une combinaison de ceux-ci ;
de préférence, ladite au moins une première partie (6102) étant formée par gravure d'au moins une partie de la doublure interne (108) afin de former une doublure interne gravée (108) ;
plus préférablement, comprenant en outre une étape d'ablation d'au moins une partie de la doublure interne gravée (108), formant ainsi ladite au moins une seconde partie (6104) de la doublure interne (108) ;
plus préférablement, l'étape d'ablation comprenant la formation d'un gradient présentant un motif prédéterminé (6106).
